# FASCICULE DE BREVET EUROPEEN

(11) **EP 1 423 418 B1**
(45) Date de publication et mention de la délivrance du brevet: **24.05.2006**
(21) Numéro de dépôt: 02777408.2
(22) Date de dépôt: 06.09.2002
(51) Int. Cl.: C07K 14/16, C07K 16/10, C12N 15/48, A61K 48/00, A61K 39/21, A61K 39/395, A61P 31/18, G01N 33/53

(54) **GENE ENV MUTE, GLYCOPROTEINE ENV MUTEE ET UTILISATIONS**
MUTIERTES ENV-GEN, MUTIERTES ENV-GLYKOPROTEIN UND VERWENDUNGEN
MUTATED ENV GENE, MUTATED ENV GLYCOPROTEIN AND THE USE THEREOF

(30) Priorité: 06.09.2001 FR 0111699
(43) Date de publication de la demande: 02.06.2004
(73) Titulaire: BIOMERIEUX, 69280 Marcy-L'Etoile (FR)
(72) Inventeur: BEDIN, Frédéric, F-69002 Lyon (FR); REYNARD, Frédéric, F-69007 Lyon (FR); VERRIER, Bernard, F-69440 Mornant (FR); ATAMAN-ÖNAL, Yasemin, F-69003 Lyon (FR)
(74) Mandataire: Guerre, Dominique
(86) Numéro de dépôt international: PCT/FR2002/003039
(87) Numéro de publication internationale: WO 2003/020755

(56) Documents cités:
- WO-A-93/17705
- WO-A-98/41536
- WO-A-99/24464
- BRAND DENYS ET AL: "Antigenic properties of recombinant envelope glycoproteins derived from T-cell-line-adapted isolates or primary human immunodeficiency virus isolates and their relationship to immunogenicity." VIROLOGY, vol. 271, no. 2, 5 juin 2000 (2000-06-05), pages 350-362, XP002227026 ISSN: 0042-6822
- LEE W-R ET AL: "NONRANDOM DISTRIBUTION OF GP120 N-LINKED GLYCOSYLATION SITED IMPORTANT FOR INFECTIVITY OF HUMAN IMMUNODEFICIENCY VIRUS TYPE 1" PROCEEDINGS OF THE NATIONAL ACADEMY OF SCIENCES OF USA, NATIONAL ACADEMY OF SCIENCE. WASHINGTON, US, vol. 89, no. 6, 1 mars 1992 (1992-03-01), pages 2213-2217, XP000673663 ISSN: 0027-8424 cité dans la demande
- KWONG P D ET AL: "Structures of HIV-1 gp120 envelope glycoproteins from laboratory-adapted and primary isolates." STRUCTURE WITH FOLDING & DESIGN. ENGLAND 15 DEC 2000, vol. 8, no. 12, 15 décembre 2000 (2000-12-15), pages 1329-1339, XP002227027 ISSN: 0969-2126 cité dans la demande

## Description

Le syndrome d'immunodéficience acquise (S.I.D.A) est une affection virale qui présente une importance majeure en Amérique, en Europe, en Afrique et en Asie. Les individus infectés présentent une immunodépression sévère et la maladie peut encore être fatale bien que des progrès aient été faits grâce à le trithérapie. La transmission de la maladie s'effectue le plus souvent par contact sexuel et par utilisation de stupéfiants par voie intraveineuse. La maladie est transmissible de la mère à l'enfant. Un des agents causals de cette maladie est le rétrovirus VIH-1. Le génome du VIH-1 a été caractérisé de façon complète par Wain-Hobson et al., 1985 (22) ; Ratner et al., 1985 (23) ; Muesing et al., 1985 (24) ; Sanchez-Pescador et al., 1985 (25). Les trois parties les plus importantes du génome du VIH-1 sont les gènes *gag*, *pol* et *env.* La séquence du gène *env* code pour la protéine Env qui est synthétisée sous la forme d'un précurseur glycosylé, la gp160, qui est clivé par une endoprotéase cellulaire en deux sous unités, la protéine de surface gp120 et la protéine transmembranaire gp41. Ces deux sous unités restent liées de manière non covalente et la glycoprotéine native ancrée à la membrane cytoplasmique ou à l'enveloppe virale est très probablement un trimère ou éventuellement un tétramère du complexe gp120/gp41, stabilisé par des interactions au niveau des domaines extracellulaires des sous-unités gp41 (McInerney T.L et al., 1998 (5)). De par sa complexité structurale, la protéine Env est très difficile à purifier dans sa conformation native.

La mise au point d'un vaccin contre le VIH-1 a focalisé des efforts internationaux soutenus depuis la découverte du virus en 1983. Aujourd'hui, après presque 20 années de recherches actives, non seulement il n'existe pas de vaccin crédible, mais de nombreuses équipes cherchent encore à identifier des approches vaccinales raisonnablement utilisables. Les stratégies classiques, à savoir l'utilisation de souches atténuées ou de virus inactivés ont été exclues ; les risques liés à l'emploi d'un rétrovirus réplicatif étant trop importants pour envisager une utilisation chez l'homme. Comme il n'existe aucun cas documenté de guérison naturelle de l'infection chez l'homme, les facteurs immunitaires de la protection n'ont pas pu être identifiés avec assurance. Pour un certain nombre d'infections virales, comme par exemple le virus de la rougeole ou le virus de la grippe, le principal facteur de protection est l'induction d'une réponse humorale neutralisante, c'est-à-dire d'anticorps dont la fixation sur la particule virale bloque l'entrée dans la cellule cible. Pour cette raison, les efforts pour le développement d'un vaccin contre le VIH-1 se sont concentrés initialement sur cette approche visant à induire des anticorps neutralisants. Dans de telles stratégies vaccinales, la glycoprotéine d'enveloppe virale (Env) constitue un immunogène de choix, parce qu'elle est la principale, voire l'unique, cible de la réponse neutralisante. Pourtant, les essais de vaccination réalisés avec les protéines d'enveloppe ont donné lieu jusqu'ici à des résultats très décevants. Avec de tels immunogènes, il est en effet possible d'induire des titres neutralisants élevés vis à vis des souches de laboratoire dans les modèles animaux du chimpanzé et du macaque, ainsi que chez des volontaires sains. Cependant, ces anticorps neutralisants efficaces contre les souches adaptées à la culture sur les lignées lymphocytaires T transformées (souches de laboratoires ou TCLA) ne protègent pas de l'infection par les virus primaires qui sont impliqués dans la transmission du VIH-1 (Moore J.P. and D.D. Ho, 1995 (1).

La distinction entre les souches adaptées au laboratoire et les isolats primaires obtenus par culture de courte durée sur les cellules T primaires est apparue en 1995 et s'est précisée avec la découverte des corécepteurs du VIH-1 dont les deux principaux sont les récepteurs à chimiokines CCR5 et CXCR4. La grande majorité des virus primaires utilisent le corécepteur CCR5 lors de leur entrée dans la cellule cible, en plus du récepteur CD4 commun à tous les virus. L'adaptation aux lignées T lymphoïdes se traduit par la perte de la capacité à utiliser le CCR5 et l'acquisition de la capacité à utiliser le CXCR4, si le virus ne le possède pas déjà (Trkola A. et al, 1996 (2)). L'autre différence fondamentale entre les deux types de virus est d'ordre immunologique : les isolats primaires sont, d'une manière générale, beaucoup plus résistants que les souches de laboratoire à la neutralisation par les anticorps et les formes solubles du CD4. En effet, des épitopes comme le site de fixation au récepteur CD4 et la boucle V3 sont peu accessibles sur les protéines Env des isolats primaires, alors qu'ils sont exposés à la surface des virus adaptés à la culture. Par conséquent, les anticorps spécifiques de ces déterminants antigéniques neutralisent efficacement les souches adaptées mais pas les isolats primaires (1). De plus, la résistance des isolats primaires à la neutralisation est indépendante de l'usage différentiel de corécepteurs entre les souches adaptées et primaires. Les rares isolats primaires qui n'utilisent que le CXCR4 comme corécepteur sont aussi difficiles à neutraliser que ceux utilisant le CCR5. Par ailleurs, dans les modèles animaux, il est effectivement possible d'obtenir une protection contre les souches adaptées de laboratoire, mais celle-ci reste limitée à la souche autologue et à un petit nombre de variants qui sont proches. La réponse neutralisante demeure inefficace contre les souches hétérologues, même s'il s'agit de variants adaptés à la culture. Cette protection limitée est due probablement à l'induction d'anticorps dirigés contre la boucle V3 qui est une région hypervariable de la protéine d'enveloppe. En utilisant des mélanges d'immunogènes issus d'isolats différents, il a été possible d'élargir la spécificité de la réponse immunitaire ; cependant une protection à large spectre, couvrant plusieurs sous types, n'a jamais pu être induite (Girard, M. et al., 2000 (3)).

Face à cette situation, plusieurs équipes se sont focalisées sur l'induction d'une immunité cellulaire spécifique du VIH-1. En effet, il est possible d'obtenir chez les vaccinés humains, comme dans les modèles animaux, des réponses cellulaires à base de lymphocytes T CD8⁺ cytotoxiques (CTL) capables de tuer les cellules infectées ou d'y bloquer la réplication du VIH-1 par sécrétion de facteurs suppresseurs. Cependant, on ne parvient pas à induire de réponses CTL chez plus de 30 à 40% des vaccinés et les réponses obtenues restent souvent dirigées contre un nombre d'épitopes trop restreints.

Une troisième voie, encore peu explorée, est l'induction d'une immunité muqueuse. Des résultats peu nombreux indiquent qu'une transsudation d'immunoglobulines IgG neutralisantes et une sécrétion d'IgA pourraient jouer un rôle dans l'exclusion du virus à travers les épithéliums.

Les particularités du VIH-1, à savoir la réplication dans les cellules du système immunitaire et l'échec initial de la réponse immunitaire antivirale chez les sujets infectés, soulignent la nécessité de développer de nouvelles approches qui doivent viser à induire des anticorps qui se fixent sur les épitopes de neutralisation conservés entre les différents sous types et présents sur les glycoprotéines d'enveloppe des virus primaires.

Pour cela, les inventeurs se sont fixés plusieurs impératifs pour l'obtention d'un vaccin préventif efficace. Il faut utiliser de manière impérative des glycoprotéines d'enveloppe issues d'isolats primaires et non des souches de laboratoire TCLA. Il faut s'assurer de présenter cette protéine au système immunitaire dans sa conformation native oligomérique. Il faut identifier de nouveaux épitopes de neutralisation à large spectre en raison de l'extraordinaire capacité d'adaptation des virus primaires à muter pour résister à la neutralisation par un anticorps. En effet, dans le modèle de la souris SCID humanisée, une tentative thérapeutique consistant en l'administration d'un mélange des trois anticorps IgG1b12, 2G12 et 2F5 a sélectionné, en l'espace d'une semaine, une sous population virale résistant à la neutralisation par chacun des trois anticorps (Poignard P. et al., 1999 (5)).

La protéine d'enveloppe d'HIV-1 est fortement glycosylée (50% de la masse totale). Les sucres présents à sa surface assurent entre autres une protection vis-à-vis de la réponse neutralisante (Reitter J.N. et al., 1998 (6)). De nombreuses études ont montré le rôle que jouaient ces résidus dans l'infectivité ou la protection. Cependant, ces études, loin d'être systématiques, ont toujours pris en compte les sites de glycosylation soit indépendamment les uns des autres soit sur des éléments structuraux particuliers comme les boucles variables V1, V2, V3 ou les extrémités N-terminale et C-terminale de la protéine (6-8) .

En 1998, un modèle de structure de gp120 a été proposé par Kwong après cristallisation d'une molécule délétée de la majorité de ses boucles variables et complexée avec le CD4s et le Fab (fragment liant l'antigène) de l'anticorps 17b (Kwong P.D et al., 1998 (9)). Curieusement, peu de travaux ont été fait sur la disposition des sites potentiels de glycosylation à la surface de la molécule oligomérique. On peut citer les travaux de Zhu X. et al., 2000 (10) qui propose un modèle pour la forme totalement glycosylée de la gp120 et ceux de Whyatt R. et al., 1998 (11) concomitant aux travaux de cristallisation.

Ainsi, selon le document WO-A-99/24464, les auteurs ont identifié, à partir de la structure cristallisée de la protéine Env du VIH-1 délétée des boucles V1/V2, les différents sites de glycosylation susceptibles d'interagir au niveau d'un même épitope de neutralisation et ont obtenu des mutants correspondants. Ceux-ci n'ont néanmoins été obtenus qu'à partir de souches de laboratoire, dont on a dit précédemment qu'elles ne sont plus appropriées à la recherche d'un vaccin efficace.

Et, à l'exception de Kwong, (Kwong P.D et al., 2000 (12)) tous les résultats ont essentiellement été obtenus sur des souches TCLA. Or, les virus adaptés en culture ont des différences épitopiques très marquées par rapport aux virus primaires et les études de Zhu et Whyatt ne reflètent pas la réalité exacte de la structure antigénique de la gp120 native du VIH-1.

Les inventeurs ont tout d'abord émis et vérifié l'hypothèse qu'au niveau du trimère d'hétéro-dimères présent à la surface des cellules infectées ou du virus, les sites potentiels de glycosylation les plus conservés sont regroupés de façon à protéger des éléments conformationnels importants pour le virus.

Pour tester cette hypothèse ils ont accompli un travail considérable. Ils ont positionné tous les sites potentiels de glycosylation présents sur les séquences d'enveloppes de 13 isolats primaires de primo-infection HIV-1 (133, 146, 153, 159, 160, 374, 384, Qz, 120, 309, 355, 373, 426) et à titre de comparaison sur 5 souches TCLA (MN, HXB2, OYI, RF SF2). Les douze séquences primaires de primo-infection (à l'exception de QZ) ont été isolées et caractérisées dans le laboratoire à partir de PBMC (cellules mononucléées sanguines du sang périphérique) de patients en phase de primo-infection hospitalisés à l'hopital de la Croix-rousse à Lyon (Ataman-Önal Y. et al., 1999 (13)). Le choix des primo-infections est justifié par le fait que le virus présent à ce stade de la maladie est proche du virus qui a initié l'infection. Les sites potentiels de glycosylation qui étaient conservés dans les 13 séquences de référence ont ensuite été repérés et les séquences correspondantes sélectionnées. Ces sites correspondent au motif protéique N.X.T/S dans lequel N signifie asparagine, X représente n'importe quel acide aminé à l'exception de la proline, T signifie thréonine et S signifie sérine. Les résultats de cette étude sont illustrés par le tableau 1.

**Tableau 1 : alignement des sites potentiel de glycosylation sur 13 séquences d'isolats primaire (IP) de patients en primo-infection et 5 séquences de souche TCLA (délétées des boucles V1/V2)**

| régions | Aa | **133** | **146** | **153** | **159** | **160** | **374** | **384** | **Qz** | **120** | **309** | **355** | **373** | **426** | **MN** | **HXB2** | **OYI** | **RF** | **SF2** |
|---|---|---|---|---|---|---|---|---|---|---|---|---|---|---|---|---|---|---|---|
| **C1** | **155** | + | | | | | | | | | | | | | | | | | |
| **C2** | **190** | + | + | + | + | + | + | + | + | + | + | + | + | + | + | + | | + | + |
| I | **223** | + | | | + | + | | + | | | | + | | | | + | | | + |
| I | **227** | | + | + | + | | + | | + | + | | | + | + | | + | + | + | |
| I | **234** | + | + | + | + | + | + | + | + | + | + | + | + | + | + | + | + | + | + |
| I | **255** | + | + | + | + | + | + | + | + | + | + | + | + | + | + | + | + | + | + |
| I | **269** | + | + | + | + | + | + | + | + | + | + | + | + | + | + | + | + | + | + |
| v | **282** | | + | | | + | + | + | + | + | + | + | + | + | + | + | + | + | + |
| **V3** | **288** | | + | + | + | + | + | | + | + | + | + | + | + | + | + | + | + | + |
| 1 | **294** | + | + | + | + | + | + | + | + | + | + | + | + | + | | + | + | + | + |
| v | **324** | | + | | + | + | + | + | + | + | + | | + | + | + | + | + | + | + |
| **C3** | **326-331** | + | + | + | + | + | + | + | + | + | | | + | | + | + | | + | + |
| 1 | **347 ou 348** | + | + | + | + | + | + | + | + | + | + | + | + | + | + | + | + | + | + |
| v | **353 ou 354** | + | | + | + | + | + | + | + | | + | | + | | + | | | | + |
| **V4** | **377** | + | | | + | + | | | + | + | | + | + | | + | + | + | + | + |
| I | **383** | | + | + | + | + | + | + | + | + | + | | | + | + | + | + | + | + |
| I | **387** | | | | | | | | | | | + | | | | | + | | |
| I | **389** | + | + | + | | + | + | + | | + | | + | + | | + | + | | + | + |
| I | **393** | | | + | + | + | + | + | + | + | + | + | | | + | | + | + | |
| I | **399** | + | | + | + | + | + | + | + | + | + | | + | | + | + | | + | + |
| v | **405** | | | + | + | | | | | | | | | | | | | | |
| **C4** | **428** | | | | | | | | | | | + | | | | | | | |
| I | **432** | | | | | | | | + | | | | | + | | | | | |
| I | **436** | + | + | | | + | + | + | + | + | + | | + | | + | | | + | + |
| I | **442** | | | + | + | | | | | | | + | | | | + | | | |
| v | **447** | + | + | | | | | | | | | | | + | | | + | | + |
| **V5** | **451** | + | + | | | + | + | + | | + | +1 | | | + | | | | | |
| I | **453** | | | | | | + | | + | | | | | | + | | | + | + |
| I | **457** | | | + | + | | | | | | | | | | | + | | | |
| v | **460** | | | | + | | | | | | | | | | | | | | |
| | Sous Type | B | B | B | B | B | B | B | B | B | B | G | B | E | B | B | B | B | B |
| | Transmission | HM | HM | HM | HM | HM | HM | HM ? | HM ? | HT | HT | HT | HT | HT | HM | HM ? | ? | ? | HM |
| | IP/TCLA | IP | IP | IP | IP | IP | IP | IP | IP | IP | IP | IP | IP | IP | TC | TC | TC | TC | TC |
| | C.R. | R5 | R5 | R5 | R5 | R5 | R5 | R5 | R5 | R5 | R5 | R5 | R5 | R5 | X4 | X4 | X4 | X4R5 | X4R5 |

| | | | | | | | | | | | | | | | | | | | |
|---|---|---|---|---|---|---|---|---|---|---|---|---|---|---|---|---|---|---|---|
| + : sites de glycosylation conservés | | | | | | | | | | | | | | | | | | | |
| Transmission : HM : homosexuelle ; HT : hétérosexuelle | | | | | | | | | | | | | | | | | | | |
| Isolat primaire : IP ; souche TCLA : TC | | | | | | | | | | | | | | | | | | | |
| Utilisation des co-récepteurs : CCR5 : R5 ; CXCR4 : X4 ; double tropisme : X4R5 | | | | | | | | | | | | | | | | | | | |
| NB : la numérotation des acides aminés est donnée par rapport à la séquence complète. | | | | | | | | | | | | | | | | | | | |

Les inventeurs ont ensuite sélectionné les sites de glycosylation les plus fortement conservés et ont positionné ces sites conservés sur la structure 3D de la gp120 (#PDB database : 1G9M) à l'aide du logiciel PDB-View (Glaxo-Welcome). Les sites enfouis dans la molécule ou présents dans le domaine interne de la gp120, domaine qui interagit avec la gp41 et donc est peu ou pas exposé à la surface de l'oligomère, ont été éliminés de l'étude. Chaque séquence a été analysée indépendamment. Cette analyse a montré que les sites conservés avaient une disposition comparable à la surface de la molécule, quelle que soit la séquence des isolats primaires.

Les inventeurs ont ensuite défini des «groupes» ou «clusters de glycosylation» qui correspondent à des regroupements des sites conservés à la surface de la gp120 d'un isolat primaire à un autre. Ces groupes étant définis, une liste de mutants de déglycosylation qui tient compte de ces résultats a été établie.

Un des groupes de glycosylation correspond à une région située à proximité du motif GPGS de la boucle V3 (groupe g14).

Un autre groupe (g112/g122) recoupe partiellement les sites de glycosylation qui définissent l'épitope neutralisant 2G12 (Trkolla A. et al., 1996 (14)).

Un troisième groupe est juxtaposé à une cavité où sont logés des acides aminés intervenant dans la liaison aux co-récepteurs (groupe 1112).

Un quatrième groupe est situé au niveau de 4 feuillets bêta anti-parallèles localisés à la jonction des deux principaux domaines de la gp120 (*Bridging-sheet*).

Un cinquième groupe est présent sur la face « silencieuse » de la gp120 (groupe 113).

Les contrôles consistent soit en le gène d'enveloppe totalement déglycosylé pour la V1 (6) soit en une délétion totale des boucles V1V2.

En parallèle, et afin de valider la notion de « cluster » de glycosylation, deux mutants supplémentaires (g123 et g1123) ont été définis qui correspondent à des déglycosylations, non pas au sein d'un même cluster, mais dans chaque différent « cluster » (une mutation par « cluster »).

Au moins une mutation a été effectuée sur les séquences sélectionnées obtenues à partir du patient 133 (PHI133, numéro d'accession AF041126). Chaque mutation consiste en le remplacement de l'asparagine du site potentiel de glycosylation (NXS/T) par une glutamine et ceci avec l'aide d'un kit de mutagenèse dirigé selon les instructions du fabriquant (Quickchange Site-directed Mutagenesis Kit, Stratagène). Chaque mutant a été cloné dans le vecteur d'expression pCI (Promega) et séquencé entièrement afin de vérifier l'intégrité de la séquence après mutagenèse. La purification des plasmides mutants a été effectuée grâce au kit Nucleobond PC500 (Macherey-Nagel).

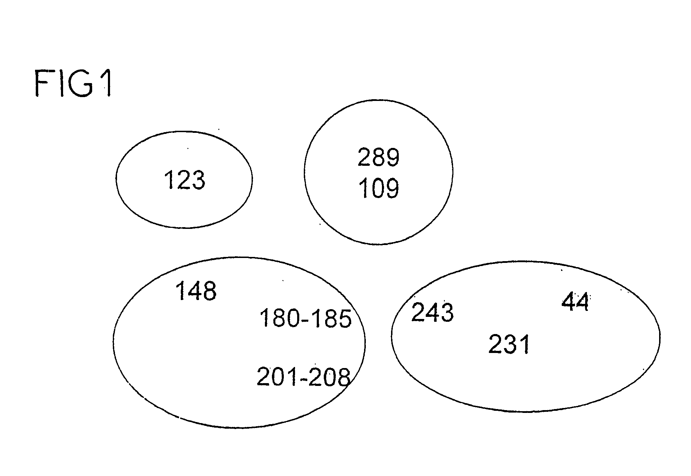

La figure 1 schématise la définition des clusters et la proximité de chaque site sur la molécule de gp120 cristallisée.

Le tableau 2 récapitule la nature et le positionnement de chaque mutation pour un mutant déterminé. Dans le tableau 2, les séquences en acides aminés de référence sont la séquence de la protéine Env de l'isolat HIV-1 133 non mutée délétée et la séquence de la protéine Env de l'isolat HIV-1 133 non mutée complète. Les correspondances en acides aminés sont données dans le tableau 2. La séquence en acides aminés de la protéine Env de l'isolat HIV-1 133 non mutée complète est identifiée en SEQ ID NO : 11 et la séquence nucléotidique correspondante du gène *env* de l'isolat HIV-1 133 non mutée (complète) est identifiée en SEQ ID NO : 1. Les séquences SEQ ID Nos 2 à 10 et SEQ ID Nos 12 à 20 qui sont listées ci-dessous correspondent respectivement aux séquences des gènes env mutés complets et tronqués de l'isolat HIV-1 133 et aux séquences des protéines Env mutées complètes et tronquées de l'isolat HIV-1 133. La séquence de la gp160 est donnée en référence à SEQ ID NO : 11. La séquence de la gp120 commence à l'acide aminé 1 et se termine à l'acide aminé 498 par référence à la SEQ ID NO : 11. La séquence de la gp140 (qui correspond à la gp120 plus les domaines extracellulaires de la gp41) commence à l'acide aminé 1 et se termine à l'acide aminé 669 par référence à la séquence SEQ ID NO : 11.

**Tableau 2 : caractéristiques des différents mutants de la séquence d'enveloppe 133.**

| **cluster/nom du mutant** | **mutations N->Q de l'acide aminé n°** _{**(a)**}**/autres** | **mutations N->Q de l'acide aminé n°** _{**(b)**}**/autres** | **régions de gp120** |
|---|---|---|---|
| **g1** | 243 | 389 | V4 |
| **g11** | 243/231 | 389/377 | V4/V4 |
| **g21** | 44 | 190 | C2 (V1V2 stem) |
| **g2** | 180-185 | 326-331 | C3 |
| **g12** | 180-185/148 | 326-331/294 | C3/V3 |
| **g112** | 180-185/148/201-208 | 326-331/294/347-354 | C3/V3/C3 |
| **g1112** | 180-185/148/201-208/243 | 326-331/294/347-354/389 | C3/V3/C3/V4 |
| **g22** | 180-185/201-208 | 326-331/347-354 | C3/C3 |
| **g122** | 180-185/201-208/231 | 32G-331/347-354/377 | C3/C3N4 |
| **g3** | 289 | 434 | C4 |
| **g13** | 289/109 | 434/255 | C4/C2 |
| **g113** | 289/109/123 | 434/255/269 | C4/C2/C2 |
| **g23** | 289/123 | 434/269 | C4/C2 |
| **g4** | 123 | 269 | C2 |
| **g14** | 123/148 | 269/294 | C2/V3 |
| **g123** | 289/123/243 | 434/269/389 | C4/C2/V4 |
| **(non cluster)** | | | |
| **g1123** | 289/123/243/180-185 | 434/269/389/326-331 | C4/C2N4/C3 |
| **(non cluster)** | | | |
| **V1 deglyc.** | V1 déglycosylée | | V1 |
| **DV1V2** | V1 V2 délétion | | V1 V2 |
| **133 native** | pas de mutation-native (contrôle +) | | - |
| **133 anti** | pas de mutation-antisens (contrôle - ) | | - |

| | | | |
|---|---|---|---|
| (a) : d'après la séquence 133 délétée [9] (b) : d'après la séquence 133 complète. | | | |

Il est bien entendu qu'en fonction de l'isolat primaire la position des sites de glycosylation conservés peut varier de 1 à 8 acides aminés et l'invention englobe ces variations de positionnement.

Le tableau 3 ci-dessous présente certains exemples de variation des sites de glycosylation et établit une récapitulation de tous les sites potentiels de glycosylation dans les gènes env des isolats primaires 133, 146, 153, 159, 160, 374 et 384. Les positions en acides aminés sont indiquées pour les séquences délétées selon Kwong et al. 1998. Chaque ligne du tableau correspond à un site potentiel, la correspondance entre les différentes positions a été déterminée par un alignement des séquences délétées. Les sites de glycosylation conservés dans toutes les séquences sont soulignés.

**Tableau 3 :**

| **Sequence** | **133** | **146** | **153** | **159** | **160** | **374** | **384** | **mutants** |
|---|---|---|---|---|---|---|---|---|
| no aa | 9 | - | - | - | - | - | - | |
| | 44 | 44 | 44 | 44 | 44 | 44 | 44 | |
| | 77 | - | - | 77 | 77 | - | 77 | |
| | - | 81 | 81 | 81 | - | 82 | - | |
| | 88 | 88 | 88 | 88 | 88 | 88 | 88 | |
| | 109 | 109 | 109 | 109 | 109 | 109 | 109 | |
| | 123 | 123 | 123 | 123 | 123 | 123 | 123 | g14 |
| | - | 136 | - | - | 136 | 136 | 136 | |
| | - | 142 | 142 | 142 | 142 | - | 142 | |
| | 148 | 148 | 148 | 148 | 148 | 148 | 148 | g12/g112/g14 |
| | 180 | 178 | - | 178 | 178 | 178 | 178 | |
| | 185 | 185 | 185 | 185 | 185 | 183 | 185 | g12/g112 |
| | 201 | 202 | 202 | 201 | 201 | 201 | 201 | g112 |
| | 208 | - | 208 | 207 | 207 | 207 | 207 | |
| | 231 | - | - | 231 | 231 | - | - | |
| | - | 238 | 238 | 237 | 237 | 238 | 237 | |
| | - | - | 242 | - | 241 | - | 241 | |
| | - | - | 243 | - | - | 243 | - | |
| | 243 | 244 | 247 | 247 | 248 | 247 | 247 | |
| | 253 | - | 253 | 253 | 254 | 255 | 253 | |
| | - | - | 258 | 259 | - | - | - | |
| | 289 | 288 | 295 | 296 | 290 | 290 | 289 | |
| | 301 | 301 | - | - | - | 304 | - | |
| | 306 | 304 | 311 | 311 | 306 | 307 | 305 | |
| | - | - | - | 314 | - | - | - | |

Les inventeurs ont ensuite étudié les caractéristiques antigéniques et fonctionnelles de chacun de ces mutants et sélectionné les mutants qui présentent les meilleures caractéristiques dans une visée vaccinale préventive. Les mutants g12, g112 et g14 ont été sélectionnés. Les résultats sont présentés dans les exemples et figures qui suivent.

La séquence SEQ ID NO: 2 représente la séquence nucléotidique du mutant g12 (gène env).

La séquence SEQ ID NO: 3 représente la séquence nucléotidique du mutant g12 tronquée qui code pour la gp120.

La séquence SEQ ID NO: 4 représente la séquence nucléotidique du mutant g12. tronquée qui code pour la gp140.

La séquence SEQ ID NO: 5 représente la séquence nucléotidique du mutant g112 (gène env).

La séquence SEQ ID NO: 6 représente la séquence nucléotidique du mutant g112 tronquée qui code pour la gp120.

La séquence SEQ ID NO: 7 représente la séquence nucléotidique du mutant g112 tronquée qui code pour la gp140.

La séquence SEQ ID NO: 8 représente la séquence nucléotidique du mutant g14 (gène *env).*

La séquence SEQ ID NO: 9 représente la séquence nucléotidique du mutant g14 tronquée qui code pour la gp120.

La séquence SEQ ID NO: 10 représente la séquence nucléotidique du mutant g14 tronquée qui code pour la gp140.

La séquence SEQ ID NO: 12 représente la séquence en acides aminés du mutant g12 (protéine Env).

La séquence SEQ ID NO: 13 représente la séquence en acides aminés de la gp120 du mutant g12.

La séquence SEQ ID NO: 14 représente la séquence en acides aminés de la gp140 du mutant g12.

La séquence SEQ ID NO: 15 représente la séquence en acides aminés du mutant g112 (protéine Env).

La séquence SEQ ID NO: 16 représente la séquence en acides aminés de la gp120 du mutant g112.

La séquence SEQ ID NO: 17 représente la séquence en acides aminés de la gp140 du mutant g112

La séquence SEQ ID NO: 18 représente la séquence en acides aminés du mutant g14 (protéine Env).

La séquence SEQ ID NO: 19 représente la séquence en acides aminés de la gp120 du mutant g14.

La séquence SEQ ID NO: 20 représente la séquence en acides aminés de la gp140 du mutant g14.

Les séquences gp120 et gp140 sont obtenues soit par troncature du gène, soit par insertion d'un codon stop et/ou un décalage de trame de lecture sans délétion du gène. Les formes gp120 et gp140 sont solubles, donc plus faciles à purifier et à administrer que la protéine Env complète.

Ainsi, la présente invention a pour objet un gène env muté codant pour une glycoprotéine d'enveloppe mutée du virus VIH-1, ledit gène présentant par rapport au gène env d'un isolat primaire de primo-infection, dit de référence, au moins deux mutations au niveau des sites de glycosylation conservés d'un isolat primaire à un autre, chaque mutation consistant en le remplacement d'un codon AAC ou AAT qui code pour une asparagine par un codon CAG ou CAA qui code pour une glutamine, les deux mutations au moins étant choisies parmi les suivantes :
(a) au moins deux mutations dans la partie du gène env codant pour la région C3 de la protéine env,
(b) au moins une mutation dans la partie codant pour la région C3 et au moins une mutation dans la partie codant pour la région V3,
(c) au moins une mutation dans la partie codant pour la région C2 et au moins une mutation dans la partie codant pour la région V3 choisie parmi les sites de glycosylation au niveau des codons 862-864 et 970-972,
(d) au moins une mutation dans la partie codant pour la région C2 choisie parmi les sites de glycosylation au niveau des codons 667-669, 679-681, 700-702, 763-765, 844-846, et au moins une mutation dans la partie codant pour la région V3,
la position d'au moins l'un quelconque desdits codons pouvant varier de trois à vingt-quatre nucléotides,
ou le gène env muté consiste en l'une quelconque des séquences SEQ ID NO: 8, SEQ ID NO: 9 ou SEQ ID NO: 10 ou leurs séquences complémentaires,

Un des intérêts des multiples mutants (au moins deux mutations) selon l'invention réside dans la diminution de la probabilité d'avoir des mutations compensatoires qui conduiraient à retrouver un phénotype sauvage.

Les mutations préférentielles selon l'invention sont les suivantes.

Selon les mutations (a), au moins une mutation est effectuée au niveau des codons 976-978, 991-993 de la partie codant pour la région C3 et au moins une mutation est effectuée au niveau des codons 1039-1041 ou 1060-1062 de la partie codant pour la région C3, la position d'au moins l'un quelconque desdits codons pouvant varier de trois à vingt-quatre nucléotides. De préférence, le gène consiste en une séquence choisie parmi les séquences identifiées en SEQ ID NO: 21, SEQ ID NO: 22 et SEQ ID NO:23 ou leurs séquences complémentaires.

Selon les mutations (b), au moins une mutation est effectuée au niveau des codons 976-978, 991-993, 1039-1041 ou 1060-1062 de la partie codant pour la région C3 et au moins une mutation est effectuée au niveau du codon 880-882 de la partie codant pour la région V3, la position d'au moins l'un quelconque desdits codons pouvant varier de trois à vingt-quatre nucléotides ; selon cette variante, le gène comprend au moins trois mutations, à savoir au moins deux mutations dans la partie codant pour la région C3 et au moins une mutation dans la partie codant pour la région V3 :
de préférence, ces mutations sont effectuées au niveau des codons 976-978, 991-993 et 880-882, la position d'au moins l'un quelconque desdits codons pouvant varier de trois à vingt-quatre nucléotides ; avantageusement, la séquence du gène ainsi muté consiste en une séquence choisie parmi les séquences identifiées en SEQ ID NO: 2, SEQ ID NO: 3 et SEQ ID NO:4 ou leurs séquences complémentaires, ou
au moins une mutation est effectuée au niveau du codon 976-978 ou 991-993, au moins une mutation est effectuée au niveau du codon 880-882, et au moins une mutation est effectuée au niveau du codon 1039-1041 ou 1060-1062 ; préférentiellement, les mutations sont effectuées au niveau des codons 976-978, 991-993, 880-882, 1039-1041 et 1060-1062 ; avantageusement, la séquence du gène ainsi muté consiste en une séquence choisie parmi les séquences identifiées en SEQ ID NO.: 5, SEQ ID NO: 6 et SEQ ID NO: 7 ou leurs séquences complémentaires,
étant entendu que la position d'au moins l'un quelconque desdits codons précité peut varier de trois à vingt-quatre nucléotides.

Selon les mutations (c), au moins une mutation est effectuée au niveau du codon 805-807 de la partie codant pour la région C2, la position d'au moins l'un quelconque desdits codons pouvant varier de trois à vingt-quatre nucléotides.

Selon les mutations (d), au moins une mutation est effectuée au niveau du codon 880-882 de la partie codant pour la région V3, la position d'au moins l'un quelconque desdits codons pouvant varier de trois à vingt-quatre nucléotides.

La séquence du gène muté peut aussi consister en une séquence choisie parmi les séquences identifiées en SEQ ID NO: 8, SEQ ID NO: 9 et SEQ ID NO: 10 ou leurs séquences complémentaires.

L'invention concerne également une glycoprotéine Env mutée du virus VIH-1, laquelle glycoprotéine présente par rapport à une protéine Env native d'un isolat primaire de primo-infection, dit de référence, au moins deux mutations au niveau des sites de glycosylation conservés de ladite protéine de référence, d'un isolat primaire à un autre, chaque mutation consistant en le remplacement d'une asparagine par une glutamine, les deux mutations au moins étant choisies parm les suivantes :
(a') au moins deux mutations dans la région C3 de la protéine Env,
(b') au moins une mutation dans la région C3 et au moins une mutation dans la région V3,
(c') au moins une mutation dans la région C2 et au moins une mutation dans la région V3 choisie parmi les sites de glycosylation au niveau de l'acide aminé 288 ou 324,
(d') au moins une mutation dans la région C2 choisie parmi les sites de glycosylation au niveau de l'un quelconque des acides aminés 223, 227, 234, 255, 282, et au moins une mutation dans la région V3,
la position desdits sites de glycosylation conservés d'un isolat primaire de primo-infection à un autre pouvant varier d'un à huit acides aminés,
ou la glycoprotéine Env mutée consiste en l'une quelconque des séquences SEQ ID NO: 18, SEQ ID NO: 19 ou SEQ ID NO: 20.

La position des acides aminés des sites de glycosylation mutés est donnée par référence à la séquence de la protéine Env native.

Les mutations préférentielles selon l'invention sont les suivantes.

Selon les mutations (a'), au moins une mutation est effectuée au site de glycosylation au niveau de l'acide aminé 326 ou 331 et au moins une mutation est effectuée au site de glycosylation au niveau de l'acide aminé 347 ou 354, la position desdits sites de glycosylation conservés d'un isolat primaire de primo-infection à un autre pouvant varier d'un à huit acides aminés. De préférence, la glycoprotéine consiste en une séquence choisie parmi les séquences identifiées en SEQ ID NO: 21, SEQ ID NO: 22 et SEQ ID NO: 23.

Selon les mutations (b'), au moins une mutation est effectuée au niveau de l'acide aminé 326 ou 331 et au moins une mutation est effectuée au niveau de l'acide aminé 294, la position d'au moins l'un quelconque desdits sites de glycosylation conservés d'un isolat primaire de primo-infection à un autre, pouvant varier d'un à huit acides aminés ; selon une variante, la glycoprotéine comporte au moins trois mutations, de préférence au moins deux mutations dans la région C3 et au moins une mutation dans la région V3 :

De préférence, ces mutations sont effectuées au niveau des acides aminés 326, 331 et 294 ; avantageusement, une glycoprotéine Env mutée de l'invention consiste en une séquence choisie parmi les séquences identifiées en SEQ ID NO: 12, SEQ ID NO: 13 et SEQ ID NO: 14 ; ou
au moins une mutation est effectuée au niveau de l'acide aminé 326 ou 331, au moins une mutation est effectuée au niveau de l'acide aminé 347 ou 354 et au moins une mutation est effectuée au niveau de l'acide aminé 294, ; avantageusement, les mutations sont effectuées au niveau des acides aminés 326, 331, 294, 347 et 354 ; mieux encore, une glycoprotéique Env mutée de l'invention consiste en une séquence choisie parmi les séquences identifiées en SEQ ID NO: 15, SEQ ID NO: 16 et SEQ ID NO: 17,
étant entendu que la position desdits sites de glycosylation conservés d'un isolat primaire de primo-infection à un autre pouvant varier d'un à huit acides aminés.

Selon les mutations (c'), au moins une mutation est effectuée au niveau de l'acide aminé 269 de la région C2.

Selon les mutations (d'), au moins une mutation est effectuée au niveau de l'acide aminé 294 de la région V3.

Une glycoprotéine Env mutée de l'invention peut consister en une séquence choisie parmi les séquences identifiées en SEQ ID NO: 18, SEQ ID NO: 19 et SEQ ID NO: 20.

L'invention a aussi pour objet une composition pharmaceutique comprenant :
(i) au moins un gène muté codant pour une glycoprotéine d'enveloppe mutée gp120 du virus VIH-1, ledit gène muté étant choisi parmi l'un quelconque des gènes décrits ci-dessus et étant placé sous le contrôle de séquences de régulation permettant son expression dans une cellule hôte,
   un véhicule pharmaceutiquement acceptable, et
   de manière facultative un agent additionnel qui facilite la pénétration dudit gène muté dans ladite cellule et/ou qui permet de cibler ladite cellule, ou
(ii) au moins le gène muté identifié en (i) cloné dans un vecteur viral recombinant.

Parmi les séquences de régulation, on peut citer le promoteur intermédiaire/précoce du CMV humain (« intermediate/early CMV human promotor ») et le gène rev du VIH-1.

Si souhaité, on peut cibler lesdites cellules hôtes ou un compartiment cellulaire déterminé desdites cellules hôtes et pour ce faire il est possible de coupler au principe actif, à savoir l'ADN muté de l'invention, au moins un agent de ciblage. Par agent de ciblage, on entend une molécule chimique ou biologique qui permet de cibler in vivo ladite cellule hôte. Il peut, entre autres, s'agir d'un ligand spécifique, d'un anti-ligand naturellement présent au niveau de la cellule hôte ou du compartiment cellulaire à cibler, en particulier présent à la surface de ladite cellule hôte, et on peut citer à titre d'exemple les anticorps comme ligands et les antigènes comme anti-ligands. Ainsi, si un anticorps est couplé au principe actif et est spécifique d'un antigène de surface de la cellule hôte, il agira comme agent de ciblage de ladite cellule hôte par l'intermédiaire de la formation spécifique d'un complexe anticorps/antigène.

Par agent qui facilite la pénétration dudit gène muté dans une cellule hôte, on entend, entre autres, des agents tels que la bupivacaïne et la cardiotoxine.

Par véhicule pharmaceutiquement acceptable, on entend, entre autres, les liposomes, les virosomes, les nanoparticules, les microparticules (exemple : les billes d'or), les iscoms.

Pour le vecteur viral recombinant, on peut citer à titre d'exemple le MVA (Modified Vaccinia Ankara), les alphavirus, le SFV (Semliki Forest Virus), les adénovirus, le AAV (Adeno-Associated Virus).

Les compositions pharmaceutiques définies ci-dessus sont des compositions vaccinales à ADN particulièrement avantageuses, en particulier par rapport aux compositions vaccinales « classiques » à base de protéine recombinante. En effet, l'utilisation à visée vaccinale de protéines recombinantes est un système lourd et onéreux, notamment parce qu'il exige de très importantes étapes de purification des antigènes recombinants. De plus, une des difficultés rencontrées est d'obtenir une persistance du vaccin suffisamment longue pour maintenir une bonne mémoire immunitaire. Au contraire, la méthode de vaccination par l'ADN, dont les avantages sont inhérents aux propriétés intrinsèques de l'ADN, est simple et peu coûteuse et est effectuée simplement par injection intramusculaire ou intradermique. De plus, il convient de noter que :
- les vaccins à ADN sont non infectieux/non réplicatifs,
- que du fait que l'immunisation par l'ADN est une forme de transfection in vivo, l'antigène viral est exprimé dans les cellules mammifères sous sa conformation native,
- comme dans le cas d'une infection virale, une large réponse immune, à la fois humorale et cellulaire est induite, et que
- de plus, les vaccins à ADN peuvent facilement être combinés en raison de leur homogénéité physico-chimique.

Mais, l'invention n'est pas limitée à une composition vaccinale à ADN telle que définie ci-dessus et concerne également une composition pharmaceutique comprenant :
- au moins une glycoprotéine d'enveloppe mutée gp120 du virus VIH-1, ladite glycoprotéine mutée étant choisie parmi l'une quelconque des glycoprotéines décrites ci-dessus,
- un véhicule et/ou excipient et/ou adjuvant et/ou diluant pharmaceutiquement acceptable.

Une telle composition vaccinale préparée est injectable, c'est-à-dire en solution liquide ou en suspension. En option, la préparation peut aussi être émulsifiée. La molécule antigénique, c'est à dire la glycoprotéine mutée, peut être mélangée avec des excipients qui sont pharmaceutiquement acceptables et compatibles avec l'ingrédient ou principe actif. Des exemples d'excipients favorables sont l'eau, une solution saline, le dextrose, le glycérol, l'éthanol ou des équivalents et leurs combinaisons. Si désiré, le vaccin peut contenir des quantités mineures de substances auxiliaires comme des agents mouillants ou émulsifiants, des agents qui tamponnent le pH ou des adjuvants comme l'hydroxyde d'aluminium, le dipeptide muramyl ou leurs variations, les polymères cationiques, les nanoparticules de polymères cationiques ou leurs variants (polyacide lactique, polyacide lactique - coglycoside).

Le vaccin est administré conventionnellement par injection, par exemple intramusculaire.

Par « véhicule pharmaceutiquement acceptable », on entend les supports et véhicules administrables à l'être humain ou à un animal, tels que décrits par exemple dans Remington's Pharmaceutical Sciences 16^{th} ed., Mack Publishing Co. Le véhicule pharmaceutiquement acceptable est de préférence isotonique, hypotonique ou présente une faible hypertonicité et a une force ionique relativement basse. Les définitions des excipients et adjuvants pharmaceutiquement acceptables sont également données dans Remington's Pharmaceutical Sciences précité.

La présente demande divulgue également un procédé pour induire une réponse humorale et/ou cellulaire améliorée contre le virus VIH-1 chez un animal mammifère selon lequel :
- on administre à un animal mammifère par injection intramusculaire ou intradermique une quantité, suffisante pour être efficace, d'une composition vaccinale à ADN, comprenant au moins le gène muté qui code pour une protéine mutée de l'invention telle que définie précédemment, ou
- on administre à un animal mammifère par injection intramusculaire ou intradermique une quantité, suffisante pour être efficace, d'une composition vaccinale comprenant au moins la protéine mutée de l'invention telle que définie ci-dessus.

Dans le cas de vaccins ADN, la concentration en acide nucléique dans la composition utilisée pour une administration in vivo est d'environ 100 µg/ml à 10 mg/ml, de préférence 1 mg/ml.

La quantité de protéine administrée est fonction de l'addition ou non d'un adjuvant, mais sera généralement comprise entre 10 et 50 µg/ml de protéine.

Le vaccin est administré à une dose déterminée en une ou plusieurs injection(s) par voie intramusculaire ou intradermique, suivie(s) de rappel(s), si nécessaire. L'effet immunisant du vaccin est suivi par un dosage d'anticorps anti-VIH-1 chez l'individu ou l'animal vacciné.

L'administration d'acide(s) nucléique(s) ou de protéine(s) d'intérêt ou de leur(s) fragment(s), seuls ou en combinaison, est utilisée pour la prophylaxie et/ou la thérapie. Quand un ou des acide(s) nucléiques ou leurs fragments ou une ou des protéine(s) ou leurs fragments sont administrés, leur caractéristique est de ne pas présenter la virulence du VIH-1 mais d'avoir la propriété d'induire une réponse immunitaire, humorale et/ou cellulaire, chez l'individu ou l'animal auquel ils ou elles sont administrés. Quand il s'agit de protéine(s), celles ci peuvent être obtenues par des techniques synthétiques ou de recombinaison génétique ou par modification de protéine(s) naturelles par des traitements chimiques ou physiques.

La ou les protéine (s) ou leurs fragments, candidate (s) pour la formulation d'un vaccin sont identifiées et analysées dans un test fonctionnel pour s'assurer qu'elles ont perdu leur toxicité et pour vérifier leur immunogénicité (i) en réalisant un test in vitro de prolifération de lymphocytes T CD4+ spécifiques de l'antigène administré (T cell assay) ou un test in vitro de cytotoxicité des lymphocytes CD8+ spécifiques de l'antigène administré et, (ii) en mesurant, entre autres, le taux d'anticorps circulants dirigés contre la protéine naturelle et leur capacité à neutraliser des isolats primiares. Ces formes modifiées sont utilisées pour immuniser des hommes par des procédures standardisées avec des adjuvants appropriés.

L'invention concerne encore :
- un procédé pour obtenir des anticorps selon lequel on immunise un animal mammifère non-humain, tel que souris ou rat ou singe, avec au moins un gène muté codant pour une glycoprotéine d'enveloppe mutée du virus VIH-1, ledit gène muté étant choisi parmi l'un quelconque des gènes décrits précédemment et étant placé sous le contrôle de séquences de régulation permettant son expression *in vivo,* et
- un procédé pour obtenir des anticorps selon lequel on immunise un animal mammifère non-humain, tel que souris ou rat ou singe, avec au moins une glycoprotéine d'enveloppe mutée du virus VIH-1, ladite glycoprotéine mutée étant choisie parmi l'une quelconque des glycoprotéines décrites précédemment.

Par anticorps, on entend les anticorps polyclonaux, les anticorps monoclonaux, les anticorps transmembraires et les anticorps humanisés ou des fragments desdits anticorps. La production d'anticorps polyclonaux et monoclonaux fait partie des connaissances générales de l'homme du métier. On peut citer à titre de référence Köhler G. et Milstein C. (1975) : Continuous culture of fused cells secreting antibody of predefined specificity, Nature 256 :495-497 et Galfre G. et al. (1977) Nature, 266 : 522-550 pour la production d'anticorps monoclonaux et Roda A., Bolelli G.F. : Production of high-titer antibody to bile acids, Journal of Steroid Biochemistry, Vol. 13, pp. 449-454 (1980) pour la production d'anticorps polyclonaux. Pour la production d'anticorps monoclonaux, l'inununogène peut être couplé à de l'hémocyanine de Lymphet Keyhole (peptide KLH) comme support pour l'immunisation ou à de l'albumine sérique (peptide SA). Les animaux sont soumis à une injection d'immunogène en utilisant de l'adjuvant complet de Freund. Les sérums et les surnageants de culture d'hybridome issus des animaux immunisés sont analysés pour leur spécificité et leur sélectivité en utilisant des techniques classiques, telles que par exemple des tests ELISA ou de Western Blot. Les hybridomes produisant les anticorps les plus spécifiques et les plus sensibles sont sélectionnés. Des anticorps monoclonaux peuvent également être produits in vitro par culture cellulaire des hybridomes produits ou par récupération de liquide d'ascite, après injection intrapéritonéale des hybridomes chez la souris. Quel que soit le mode de production, en surnageant ou en ascite, les anticorps sont ensuite purifiés. Les méthodes de purification utilisées sont essentiellement la filtration sur gel échangeur d'ions et la chromatographie d'exclusion ou la chromatographie d'affinité (protéine A ou G). Un nombre suffisant d'anticorps sont criblés dans des tests fonctionnels pour identifier les anticorps les plus performants. La production *in vitro* d'anticorps, de fragments d'anticorps ou de dérivés d'anticorps, tels que des anticorps chimères produits par génie génétique est bien connue de l'homme du métier.

Par anticorps transmembranaire on entend un anticorps dont au moins la région fonctionnelle capable de reconnaître et de se fixer à son antigène spécifique est exprimée à la surface des cellules cibles pour permettre lesdites reconnaissance et fixation. Plus particulièrement, les anticorps consistent en des polypeptides de fusion comprenant les amino acides définissant ladite région fonctionnelle et une séquence d'amino acides (polypeptide transmembranaire) permettant l'ancrage au sein de la double couche lipidique membranaire de la cellule cible ou à la surface externe de cette bi-couche. Les séquences nucléiques codant pour de nombreux polypeptides transmembranaires sont décrites dans la littérature.

Les formes « humanisées » d'anticorps non humains, par exemple murins, sont des anticorps chimères qui comprennent une séquence minimale dérivée d'une immunoglobuline non humaine. Pour la plupart, les anticorps humanisés sont des immunoglobulines humaines (anticorps récepteur) dans lesquelles des résidus d'une région hypervariable du récepteur sont remplacés par des résidus d'une région hypervariable d'une espèce donneur (anticorps donneur) non humaine, telle que souris, rat, lapin ou primate non humain, ayant la spécificité, l'affinité et la capacité souhaitées. Dans certains cas, les résidus (FR) de la région Fv de l'immunoglobuline humaine sont remplacés par des résidus correspondants non humains. De plus, les anticorps humanisés peuvent comprendre des résidus qui ne sont pas trouvés dans l'anticorps receveur ou dans l'anticorps donneur. Ces modifications sont effectuées pour améliorer les performances de l'anticorps. En général, l'anticorps humanisé comprendra au moins et de préférence deux domaines variables, dans lesquels tout ou à peu près tout des boucles hypervariables correspondent à une immunoglobuline non humaine et tout ou à peu près tout des régions FR seront celles d'une immunoglobuline humaine. Les anticorps humanisés facultativement pourront aussi comprendre au moins une partie d'une région constante (Fc) d'une immunoglobuline, telle qu'une immunoglobuline humaine (Jones et al., Nature 321 : 522-525 (1986) ; Reichmann et al., Nature 332 : 323-329 (1988) ; et Presta et al., Curr. Op. Struct. Biol. 2 : 593-596 (1992).

Par fragment d'anticorps on entend les fragments F(ab)2, Fab, Fab', sFv (Blazar et al., 1997, Journal of Immunology 159 : 5821-5833 et Bird et al., 1988, Science 242 : 423-426) d'un anticorps natif et par dérivé on entend, entre autres, un dérivé chimérique d'un anticorps natif (voir par exemple Arakawa et al., 1996, J. Biochem 120 : 657-662 et Chaudray et al., 1989, Nature 339 : 394-397).

Ces anticorps peuvent être incorporés dans une composition pharmaceutique, en particulier quand il s'agit d'anticorps neutralisants, pour répondre à une infection virale par le VIH-1.

Enfin, l'invention concerne un procédé d'évaluation d'un agent thérapeutique, selon lequel on administre à un animal non-humain au moins un gène muté codant pour une glycoprotéine d'enveloppe mutée du virus VIH-1 tel que décrit précédemment et on réalise :
- un dosage d'anticorps spécifiques de la gp120 mutée, et/ou
- un dosage des anticorps neutralisants spécifiques de la glycoprotéine mutée ou native qui vont induire une diminution de l'infectivité du virus, et/ou
- un dosage de la réponse immune cellulaire induite contre la gp120 mutée par exemple par un test d'activation *in vitro* de cellules lymphocytes T « helper » spécifique(s) de la glycoprotéine mutée.

Pour une meilleure compréhension de la partie expérimentale qui va suivre, il convient de revenir en quelques lignes sur le mécanisme d'infection du VIH-1. Au cours de l'infection, la gp120 se fixe au récepteur CD4. Suite à cette interaction, la gp120 subit un réarrangement structural libérant des épitopes dits « CD4 induits » qui permettent la fixation du co-récepteur. La fixation du CD4 puis celle du co-récepteur sont des événements pré-requis pour l'ancrage du peptide de fusion de la gp41 dans la membrane cellulaire et donc l'infection de la cellule.

### Figures.

La figure 1 schématise la définition des clusters et la proximité de chaque site sur la molécule de gp120 cristallisée.
La figure 2 représente la détection de gp160 (□) et de gp120 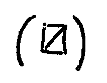 dans chaque lysat cellulaire pour chacun des mutants réalisés et pour les contrôles.
La figure 3A représente l'antigénicité testé en ELISA dans les lysats cellulaires vis à vis d'un pool de sérums HIV positif et la figure 3B représente l'antigénicité testé en ELISA dans les surnageants de culture vis à vis du pool de sérums HIV positif.
La figure 4A représente la quantité de CD4s détectée dans les lysats cellulaires et la figure 4B représente la quantité de CD4s détectée dans les surnageants de culture.
La figure 5A représente la reconnaissance de l'épitope F105 testée en ELISA dans les surnageants de culture et la figure 5B représente la reconnaissance de l'épitope F105 testée en ELISA dans les lysats cellulaires.
La figure 6A représente la reconnaissance de l'épitope CG10 en présence de CD4 testée en ELISA dans les lysats cellulaires et la figure 6B représente la reconnaissance de l'épitope CG10 seule (■) et en présence de CD4 (□) testée en ELISA dans les surnageants de culture.
La figure 7A représente la reconnaissance de l'épitope 17b seul (□) et en présence de CD4 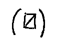 testée en ELISA dans les lysats cellulaires et la figure 7B représente la reconnaissance de l'épitope 17b seul (□) et en présence de CD4 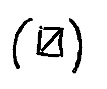 testée en ELISA dans les surnageants de culture.
La figure 8 illustre la neutralisation du virus FR (virus primaire X4) sur des cellules Hela P4PCCR5 par le sérum de souris immunisées par différentes constructions (Exemple 4) .
La figure 9 illustre l'infection par des pseudo-particules portant les glycoprotéines mutées identifiées dans le tableau 2, de cellules GHOST-CCR5 et GHOST-CXCR4.
La figure 10 illustre la neutralisation des pseudo-particules portant les glycoprotéines mutées par un sérum humain CRI/LY neutralisant.

### Exemples.

### Exemple 1 : Expression et clivage.

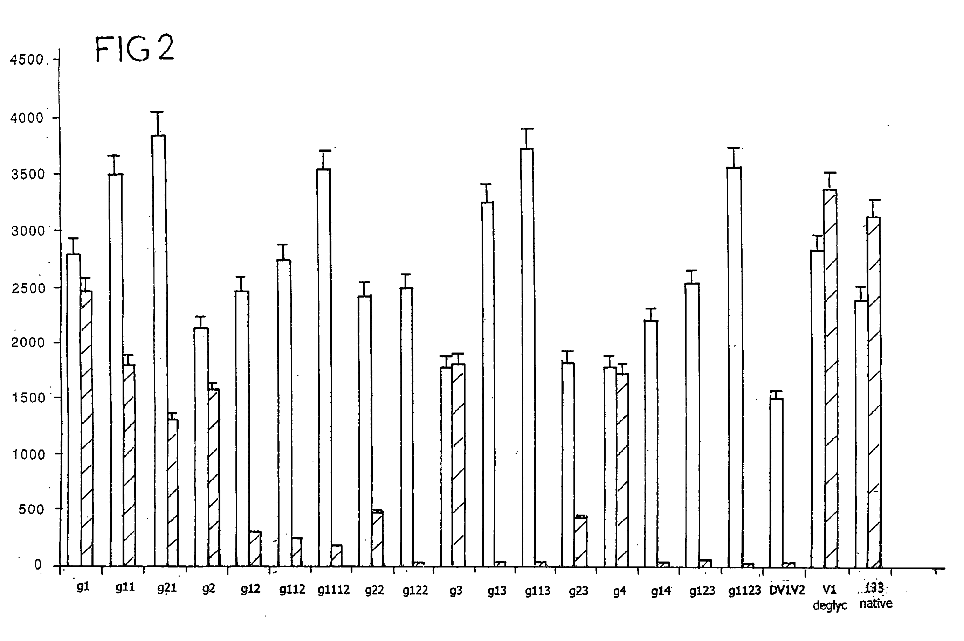

Des cellules 293T (cellules épithéliales de rein) sont co-transfectées par chacune des constructions PCI-env et le plasmide d'expression PCI-rev à l'aide du kit Lipofectamine-Plus (Gibco-BRL) selon les instructions du fabriquant. Rev est une protéine de régulation de HIV-1 qui joue un rôle important dans l'expression de la protéine d'enveloppe. Quarante-huit heures après transfection, le surnageant de culture et les cellules sont récupérées et testés en Western-blot avec un anticorps polyclonal de mouton anti-gp120 (Biogenesis) dilué au 1/5000. Pour cela, les échantillons sont déposés sur un gel dénaturant d'acrylamide 8%. Après séparation, les échantillons sont transférés sur une membrane de PVDF (ImmobilonP, Amersham) selon les instructions du fabricant. Pour les lysats, la normalisation des échantillons a été faite avec un anticorps monoclonal anti-Actine dilué au 1/5000 (A4700, Sigma) et anti-Rev 2D4D10 dilué au 1/5000 (bioMérieux). Pour les surnageants la normalisation a été faite d'après le taux d'albumine sérique du milieu de culture. La révélation des blots est effectuée à l'aide du kit ECF (Amersham) et les bandes révélées sont quantifiées sur Typhoon 8600 grâce au logiciel ImageQuant. Toutes les constructions expriment une protéine qui réagit avec le polyclonal anti-gp120. La migration des protéines mutantes est affectée par la perte d'une ou de plusieurs glycosylation. La baisse de gp120 détectée dans le lysat cellulaire est associée à l'augmentation du nombre de mutations (figure 2). Parfois, la bande spécifique est totalement absente (mutants g1112, g122, g13, g113, g14, g123, g1123 et DV1V2). Elle est également absente du surnageant de culture correspondant : on peut penser que le taux de clivage est affecté. Moulard (15) a décrit ce phénomène qu'il associe à une mauvaise translocation dans le RE et/ou un défaut de repliement empêchant l'accessibilité du site de coupure. A *contrario,* Pour le mutant g14, une quantité importante de gp120 est détectée dans le surnageant (environ 8x plus que la protéine native, à quantité de protéines normalisées) alors que celle-ci n'est pas détectée dans le lysat cellulaire. I1 semble donc que les mutations présentes sur ce clone affectent les interactions liant entre-elles les sous-unites de l'enveloppe et non pas le taux de clivage de la gp160. Le taux de clivage pour les simples mutants est en général comparable à celui observé pour l'enveloppe native (excepté pour le mutant g21). Il est intéressant de noter qu'en comparaison le mutant correspondant à la déglycosylation totale de la boucle V1 semble peu affectée par la perte de 3 glycosylations.

### Exemple 2 : Aptitude des mutants à former des syncytia in vitro.

Quarante-huit heures après transfection, les cellules 293T qui expriment les différents mutants sont repiqués au 1/10 et mises en présence de cellules GHOST-CCR5 ou -CxCR4 à 50% de densité.

Après 16 heures de co-culture, les cellules sont fixées 15 minutes au glutaraldéhyde 0,5% puis les noyaux et les cytoplasmes sont colorés en May-Grünwald/Giemsa.

L'observation et le comptage des syncytia formés sont appréciés par observation au microscope optique inversé.

Pour les GHOST-CCR5, la capacité à induire des syncytia est réduite quand le nombre des mutations est augmenté. Elle est nulle pour les clones qui ne sont pas clivés (g113, g1123 et peut-être g1112). Ce résultat confirme les résultats précédents et donne une indication sur le maintien de la fonctionnalité des différentes enveloppes après mutation. Les constructions n'induisent pas la formation de syncytia sur des cellules indicatrices CXCR4 comme cela a pu être mis en évidence par Pollakis (16) après déglycosylation partielle de la boucle V3 de la gp160 ADA.

Les résultats sont présentés dans le tableau 4.

**Tableau 4 : Aptitude des mutants de l'enveloppe à former des syncytia in vitro.**

| constructions | G1 | g11 | g21 | g2 | g12 | g112 | G1112 | G22 | g122 | g3 | g13 | g113 | g23 | g4 | g14 |
|---|---|---|---|---|---|---|---|---|---|---|---|---|---|---|---|
| FS CCR5 | ++ | ++ | + | ++ | + | + | ? | ++ | + | ++ | + | - | ++ | ++ | + |
| FS CXCR4 | - | - | - | - | - | - | - | - | - | - | - | - | - | - | - |
| constructions | G123 | g1123 | DV1V2 | dgV1 | natif | anti | HxB2 | | | | | | | | |
| FS CCR5 | + | - | - | ++ | ++ | - | - | | | | | | | | |
| FS CXCR4 | - | - | - | - | - | - | + | | | | | | | | |

| | | | | | | | | | | | | | | | |
|---|---|---|---|---|---|---|---|---|---|---|---|---|---|---|---|
| FS CCR5 : formation syncytia CCR5 FS CCR4 : formation syncytia CCR4 | | | | | | | | | | | | | | | |

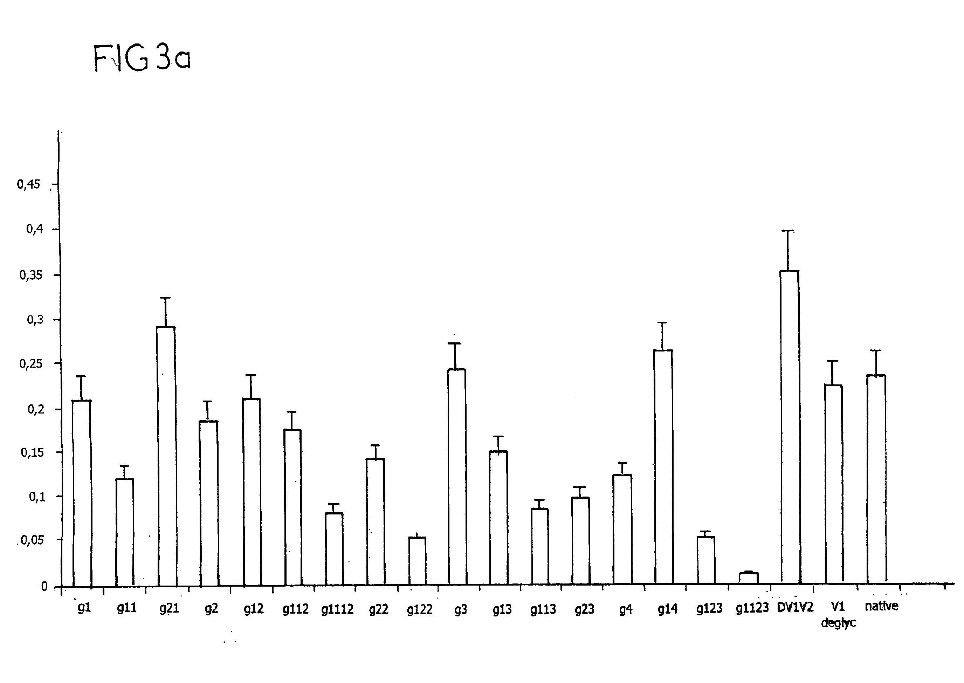

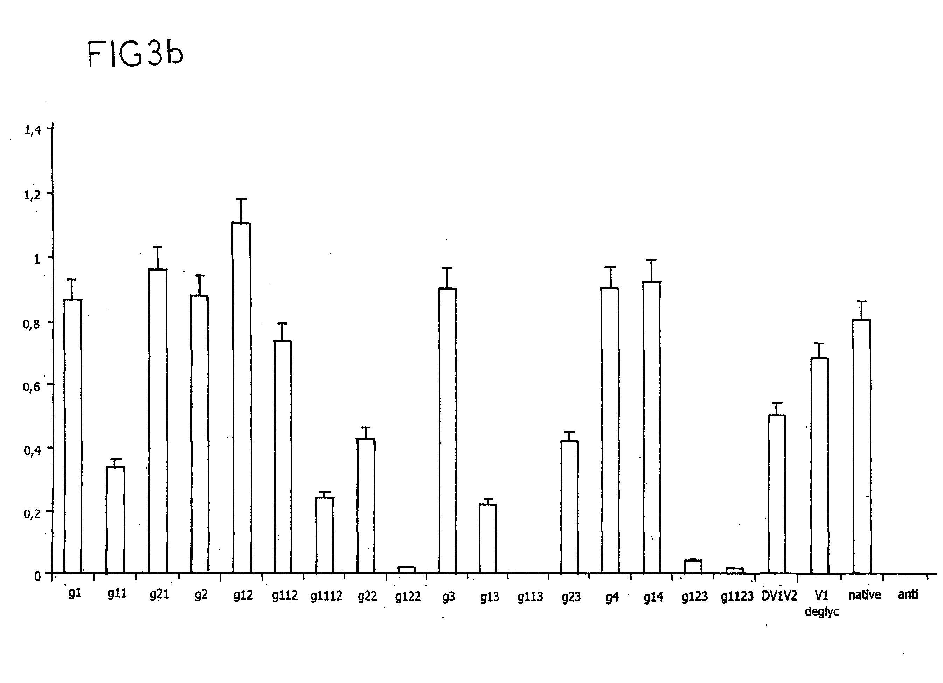

### Antigénicité vis-à-vis d'un pool de sérums HIV positif (cf. figure 3).

Les lysats et les surnageants ont été testés en ELISA de capture avec un pool de sérums HIV+ provenant de l'hôpital de la Croix-Rousse à Lyon. Ce pool de sérums est constitué de 2 échantillons sous-types B et d'un échantillon sous-type C. Tous les résultats d'ELISA correspondent à la moyenne de 2 expériences en duplicate. Le format du test est le suivant:
- Anticorps de capture : D7324 (dirigé contre le site de clivage alterne situé dans la région C5; Aalto) à 1µg/ml en PBS1x ou 12G10B11 (dirigé contre la gp41, bioMérieux) à 5µg/ml en PBS1x ; incubation la nuit à 6°C.
- Saturation par PBS1x-Tween 0,1%-lait écrémé en poudre 3% ; incubation 1heure à 22°C.
- Lysats dilués au 1/10 en PBS-NP40 1%-BSA 20µg/ml ou surnageants au pur ; incubation 2 heures à 22°C.
- Pool de sérums HIV+ dilué au 1/100 en PBS1x-Tween 0,1%-BSA 20µg/ml ; incubation 1 heure à 22°C.
- Conjugué anti-Fc IgG humain couplé à la peroxydase (Jackson) dilué au 1/5000 en PBS1x-Tween 0,1%-BSA 20ug/ml ; incubation 30 minutes à 22°C.
- Révélation par un mélange de 20mg OPD (Pierce) et de 10ml de Color2 (BioMérieux) ; incubation de 15 minutes à 22°C avant lecture à 492 nm sur un lecteur de plaques (Biorad).

Entre chaque incubation 4 lavages au PBS1x-Tween 0,1% sont effectués avec un laveur de plaques Biorad.

Les surnageants sont capturés par l'anticorps D7324 ; les lysats sont capturés soit par le D7324, soit par le 12G11B10. Ce dernier a principalement été utilisé car l'épitope D7324 est peu exposé sur les gp160 non clivées et le signal détecté dépend alors du taux de clivage des différents mutants.

Les résultats montrent que la perte d'antigénicité corrèle avec le nombre de mutations. Les résultats sur les lysats (coating 12G11B10) et sur les surnageants coïncident et confirment l'absence de gp120 dans les surnageants pour les mutants g113 et g1123.

### Exemple 3 : Reconnaissance

### Reconnaissance de l'épitope 2G12.

2G12 est un anticorps monoclonal humain obtenu par électrofusion de PBL HIV+ et de cellules CB-F7 (17). Il a un pouvoir neutralisant à large spectre sur des isolats primaires et certains virus TCLA. L'épitope reconnu par cet anticorps est discontinu et couvre principalement les régions V3 et V4 ainsi que certains sucres présents dans ces régions. La reconnaissance de 2G12 a été testée en ELISA sur les lysats cellulaires et sur les surnageants des différents mutants. Le format du test est le même que le précédent, excepté pour l'anticorps de détection qui est le 2G12 (1µg/ml). Aucun signal positif n'est détecté même pour la protéine 133-native. Ce résultat confirme des résultats obtenus en RIPA et en ELISA par l'équipe de D. Brand (Tours) sur l'enveloppe 133. Parren, en 1998, a décrit un isolat primaire résistant aux trois anticorps neutralisant 2G12, 2F5 et B12. Ses résultats indiquent que cette résistance est probablement due à un changement global de la structure oligomérique de l'enveloppe.

### Reconnaissance du site de fixation au CD4.

La glycoprotéine d'enveloppe a la capacité de lier le CD4 avec une forte affinité. Cette capacité de liaison est primordiale dans le processus d'infection.
- Fixation du CD4 soluble (CD4s). Le format ELISA suivant a été utilisé :

- Coating D7324 (surnageant) ou 12G11B10 (lysat).
- Saturation.
- Lysat dilué au 1/10 ou surnageant au pur.
- CD4s (NIAID) dilué à 1µg/ml en PBS1x-Tween 0,1%-BSA 20 µg/ml ; incubation 2 heures à 22°C.
- Polyclonal anti-CD4 biotinylé dilué à 1µg/ml en PBS1x-Tween 0,1%-BSA 20 µg/ml ; incubation 1 heure à 22°C.
- Streptavidine-peroxidase (Jackson) dilué au 1/10000 en PBS1x-Tween 0,1%-BSA 20 ug/ml ; incubation 30 minutes à 22°C.
- Révélation par un mélange de 20mg OPD (Pierce) et de 10ml de Color2 (bioMérieux).
- Lecture à 492nm.

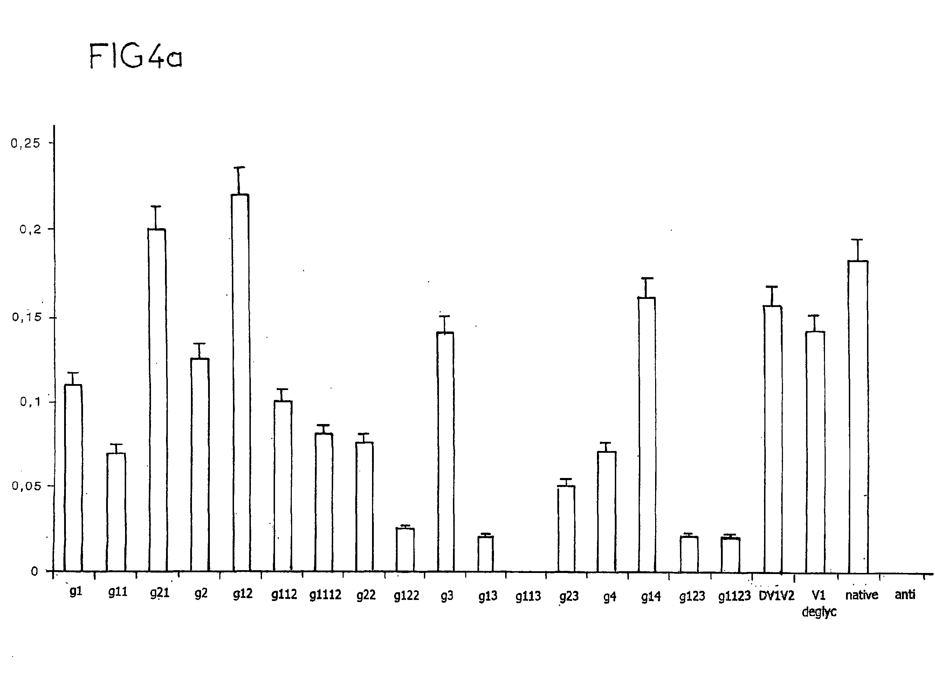

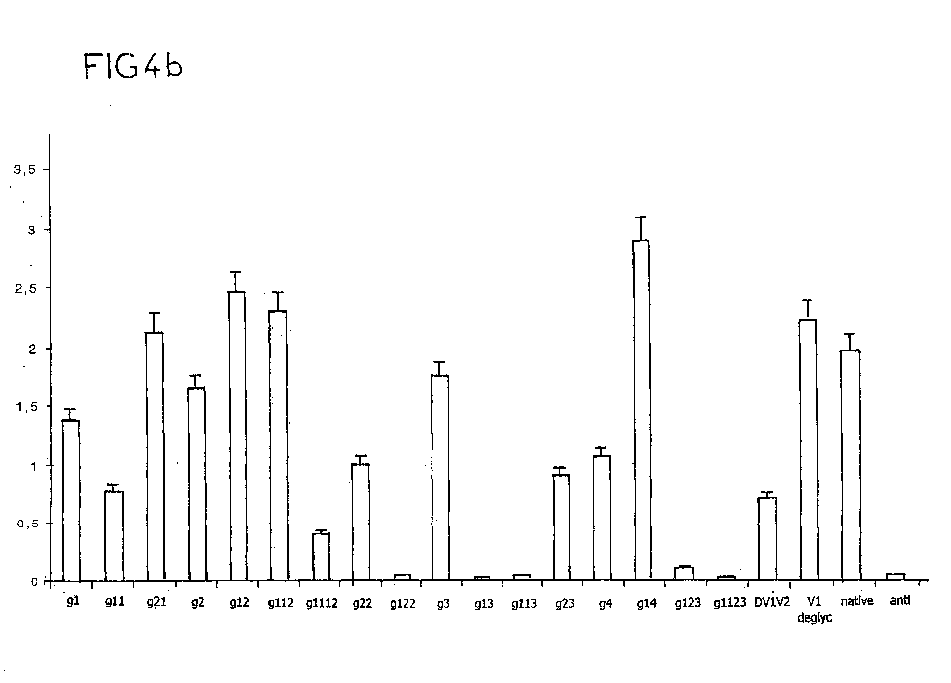

La quantité de CD4s détectée varie de la même façon sur les lysats et sur les surnageants (figure 4). L'intensité du signal est fonction du nombre de sites déglycosylés exception faite pour les mutants g12 et g14 qui correspondent à des doubles mutations. Les clones correspondant à des protéines d'enveloppe dont l'antigénicité ou le taux de clivage sont réduits ont un signal bas (g122, g13, g113, g123 et g1123).

### Reconnaissance de l'épitope IgG1b12.

Cet anticorps monoclonal humain est décrit comme ayant une importante activité neutralisante à la fois sur isolats primaires (clade A-D) et sur souches de laboratoire. L'épitope est discontinu et recouvre le site de fixation au CD4. La reconnaissance se fait préférentiellement sur les oligomères à la surface des cellules infectées ou des virus, au dépend des « débris » qui empêchent une réponse neutralisante efficace.

Aucun signal positif n'a été noté quels que soient les clones testés. Ceci est en accord avec les résultats préliminaires de D. Brand sur l'enveloppe 133. IgG1b12 est décrit comme étant très sensible aux substitutions au niveau des boucles V1 et V2. Certains acides aminés dans les régions C2 et C3 sont aussi décrits comme jouant un rôle crucial dans la reconnaissance de l'épitope b12 (18). Une des particularités de l'enveloppe 133 est d'avoir une séquence protéique atypique comparée aux autres protéines d'enveloppe du même sous-type.

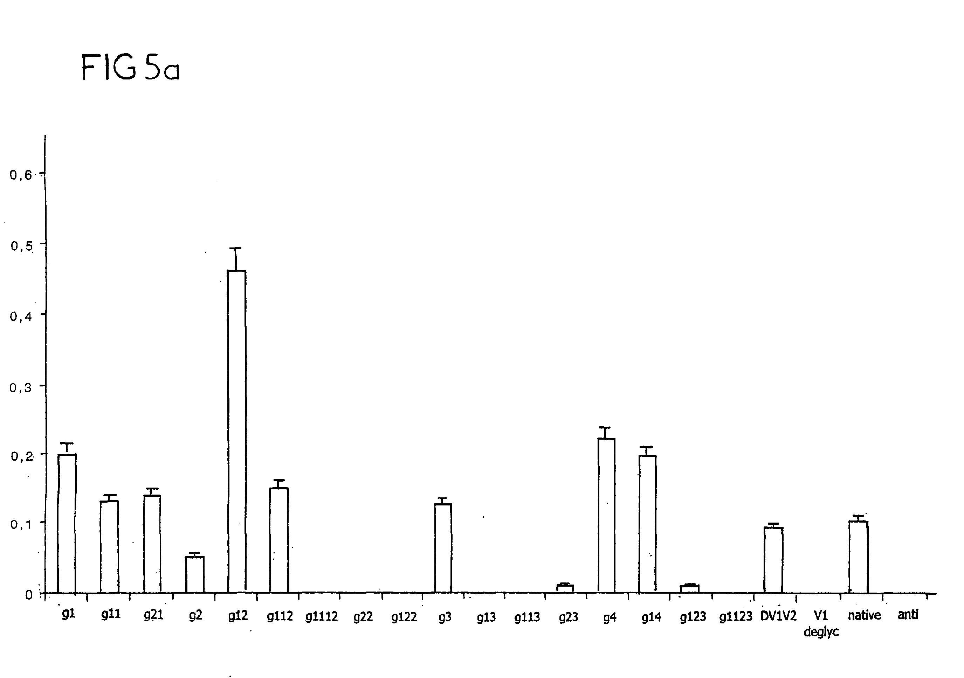

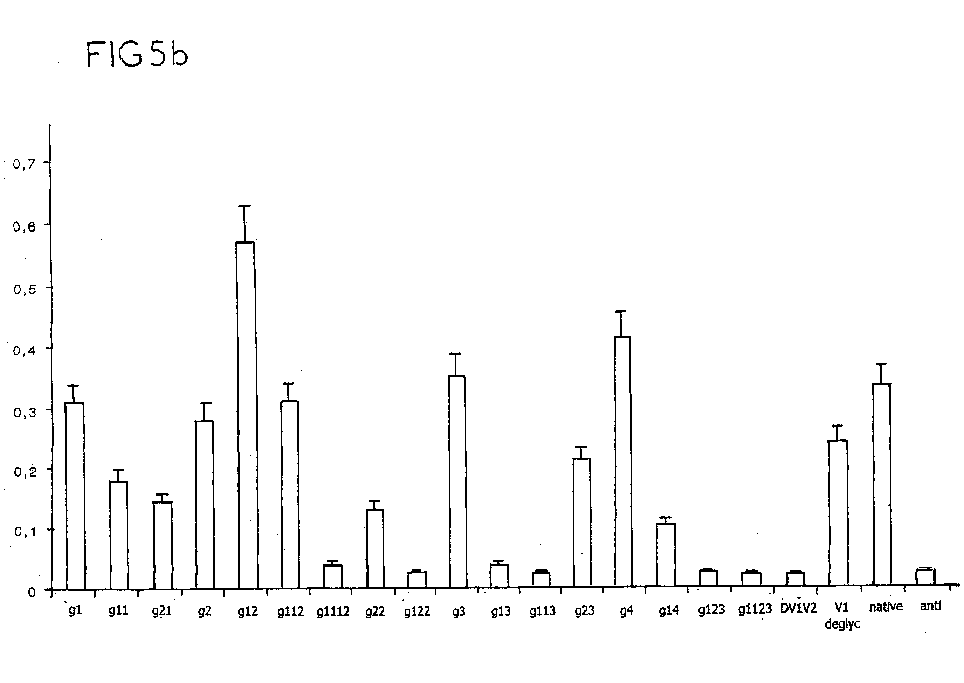

### Reconnaissance de l'épitope F105 (cf. figure 5).

L'anticorps monoclonal humain F105 se lie à proximité du site de fixation au CD4. Le CD4s peut inhiber sa fixation. L'anticorps F105 neutralise les souches TCLA. Certains auteurs ont décrit une neutralisation pour quelques isolats primaires (19) mais les résultats sont très controversés.

La fixation de cet anticorps a été testée sur les lysats et les surnageants. Le format du test est le même que celui utilisé avec le 2G12. L'anticorps F105 (NIBSC) est utilisé à une concentration de 1µg/ml. Un signal important est noté pour le clone g12 (jusqu'à 4,5x le signal de la protéine native à quantité de protéine équivalente). La mutation n°294 présente sur g12 (mais non sur g2) est située au niveau de la boucle V3 ; on sait que l'attachement de F105 sur des glycoprotéines délétées pour V3 est amélioré. D'autres mutations semblent avoir un rôle crucial dans la reconnaissance de la protéine par l'anticorps. C'est le cas de la mutation n°255, présente dans les clones g13 et g113. Elle est localisée dans un peptide de la C2 décrit comme important dans l'échappement à la neutralisation par F105 (20). Il n'y pas de signal pour les mutants g1112 et ceux de la série g22/g122.

### Reconnaissance des épitopes CD4 induits.

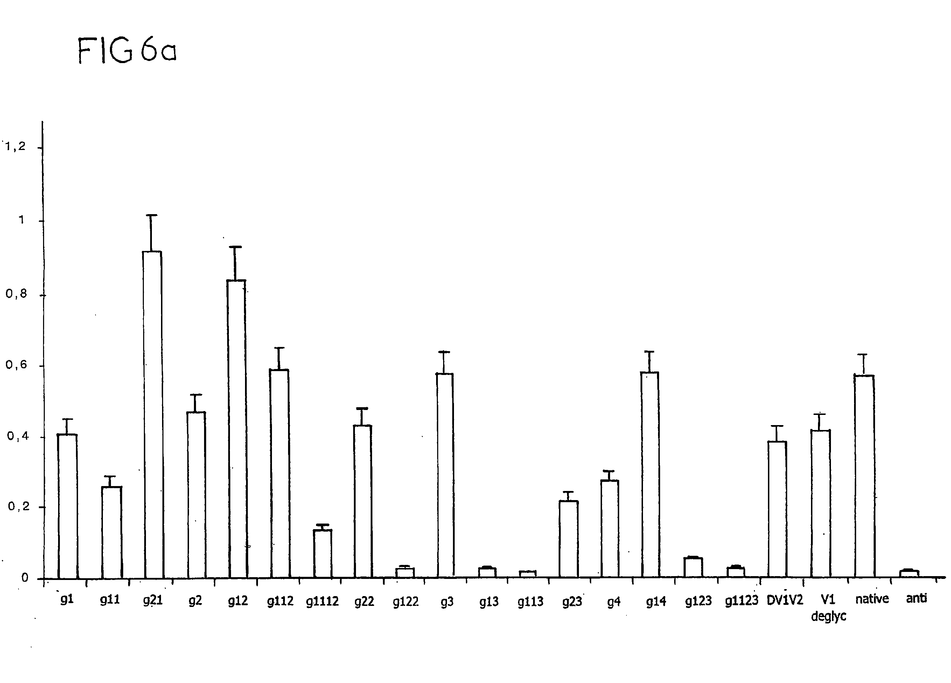

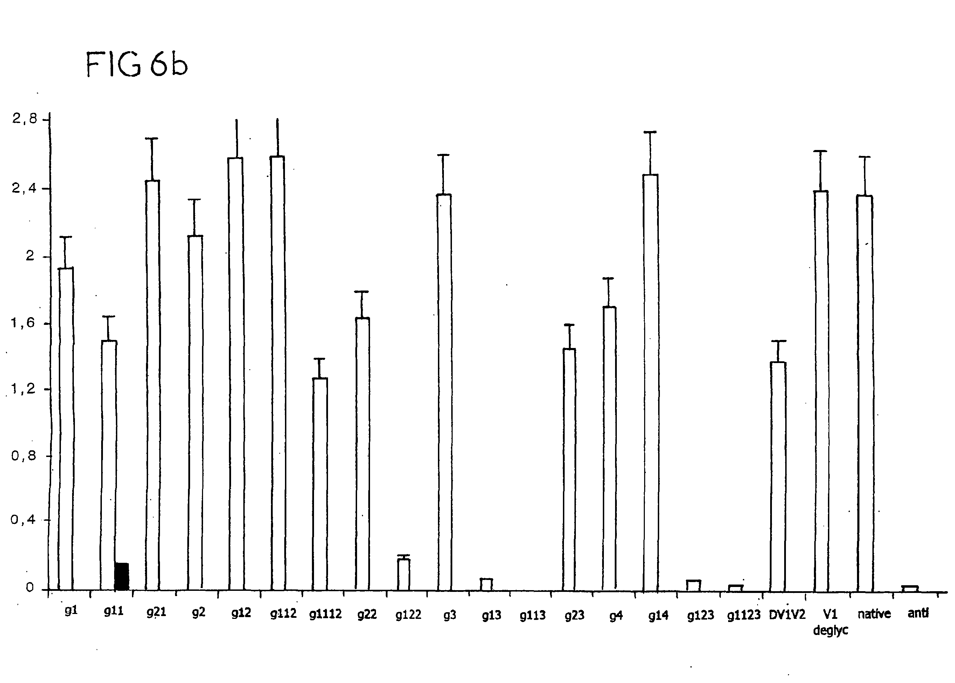

La fixation du CD4 induit des changements de conformation et permet la formation d'épitopes *de novo* dont l'émergence conditionne la fixation du co-récepteur.
- Reconnaissance de l'épitope CG10 (figure 6). CG10 est un anticorps monoclonal murin capable de neutraliser certaines souches TCLA. Il se lie à proximité du « bridging-sheet » en compétition avec le 17b. Sa fixation est strictement dépendante du CD4 (21).

L'ELISA utilisé a le format suivant:
Coating: D7324 (surnageants) ou 12G11B10 (lysats) .
Saturation.
Lysats dilués au 1/10 ou surnageants au pur.
Ajout (ou non ajout) de CD4s à 1µg/ml dilué en PBS1x-Tween 0,1%-BSA 20µg/ml ; incubation 2 heures à 22°C.
CG10 (don de Francisco Veas , UMR 5087, Montpellier) à 1µg/ml dilué en PBS1x-Tween 0,1%-BSA 20µg/ml ; incubation 1 heure à 22°C.
Conjugué anti-IgG de souris couplé à la peroxydase (Jackson) dilué au 1/5000 en PBS1x-Tween 0,1%-BSA 20µg/ml
Révélation : 20mg OPD (Pierce) et de 10ml de Color2 (bioMérieux).
Lecture à 492nm.

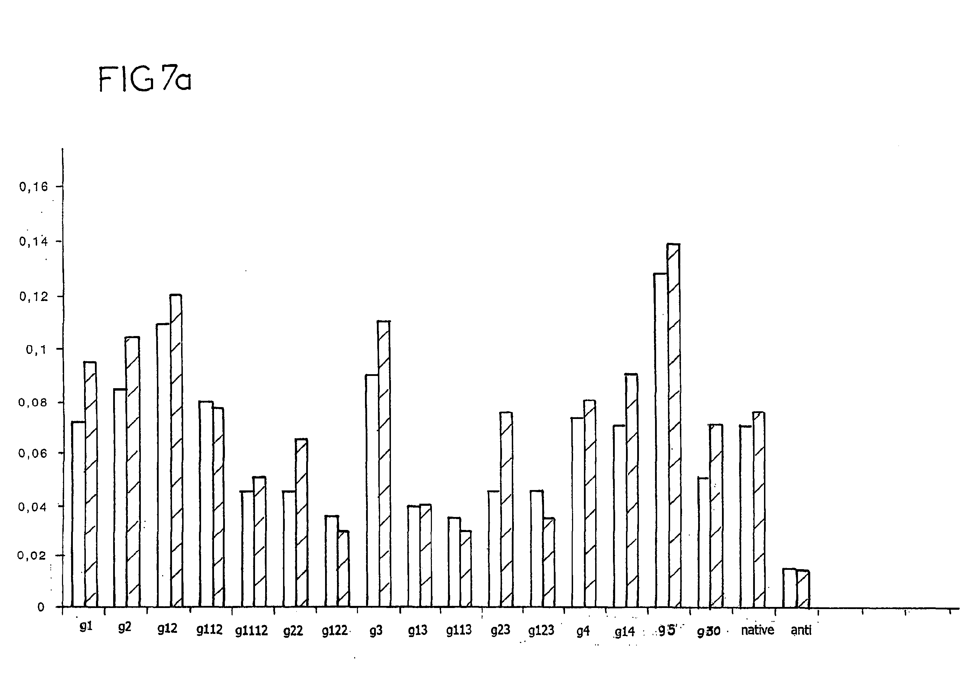

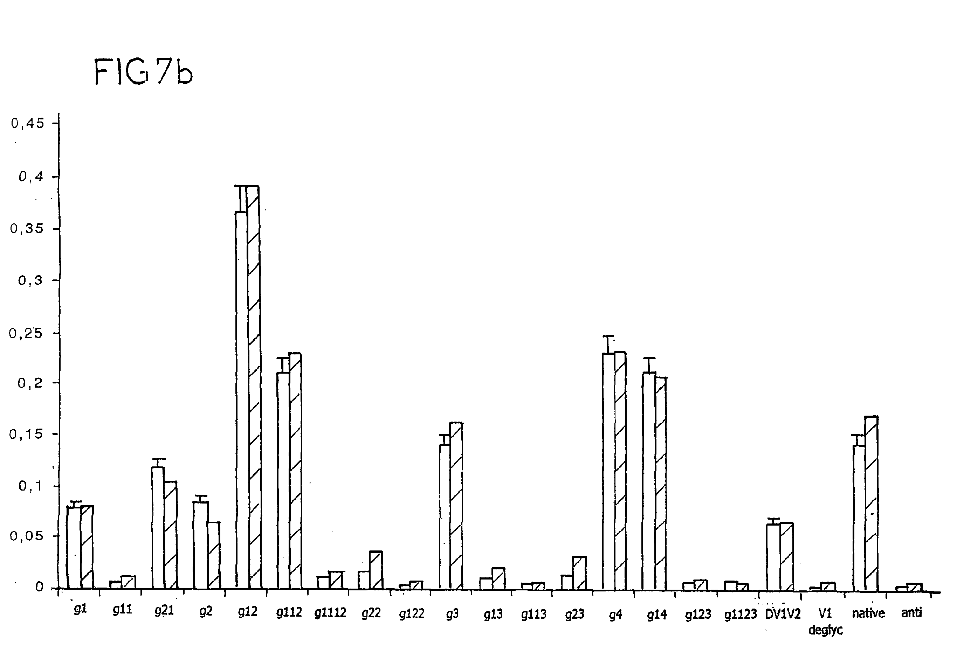

Les résultats en ELISA montrent une bonne reconnaissance de l'épitope CG10 en présence de CD4s et pour la majorité des protéines d'enveloppe à l'exception des mutants g122, g13, g113 g123 et g1123. Le signal corrèle bien avec le taux de fixation au CD4s (cf. figure 5). L'utilisation de CG10 seul conduit à une absence de signal.
- Reconnaissance de l'épitope 17b (cf. figure 7). 17b est un anticorps monoclonal humain dirigé contre un épitope CD4-induit. Il a une activité neutralisante limitée à la fois pour les isolats primaires et les souches TCLA. Si l'exposition de l'épitope ne dépend pas strictement de la fixation du CD4 elle est néanmoins améliorée par sa présence. Le format d'ELISA utilisé est le même que pour CG10. L'anticorps 17b est utilisé à la concentration de 1µg/ml. La détection se fait avec un anticorps anti-Fc IgG humain couplé à la peroxydase et dilué au 1/5000 (Jackson). Dans l'expérience, on observe que certains mutants ont une bonne reconnaissance de cet anticorps et notamment les mutants g12/g112, g3 et g4/g14. Pour g12, le signal est environ 3x supérieur à celui enregistré pour la protéine native à quantité de protéine équivalente. Les mutations présentes dans g12/g112 concernent la boucle V3 et le début de la région C3. Selon Wyatt (11) V2 et V3 couvriraient partiellement l'épitope 17b et c'est la fixation du CD4 qui permettrait un retrait de la boucle V2. On peut imaginer que la déglycosylation de la boucle V3 (NB: la boucle V3 de la séquence 133 a un seul site potentiel de glycosylation) et des sites situés à sa base permettent à la fois un démasquage partiel de la boucle V3 et une augmentation de sa flexibilité qui pourraient en conséquence conduire à une meilleure accessibilité de l'épitope 17b.

Les expériences détaillées ci-dessus montrent l'intérêt des mutants g12, g112 et g14 au regard de leurs caractéristiques antigéniques et fonctionnelles.

L'immunogénicité des mutants g12 et g112 (bonne affinité pour F105 et pour 17b) doit être bonne. Les mutants de la série g4/g14 sont intéressants car ils ont une bonne réactivité vis à vis des différents anticorps testés.

### Exemple 4 : sélection d'un panel de mutants et évaluation du pouvoir neutralisant des anticorps générés après immunisation de souris Balb/C

D'après les résultats d'ELISA, le panel de mutants suivant : g12, g112, g14 a été choisi. Le mutant g13 a aussi été évalué du fait du positionnement en cluster des sites mutés sur la face « silencieuse » du domaine externe de la gp120. Le mutant g123 (mutant non-cluster), ainsi que le gène de l'enveloppe sauvage, ont été utilisés comme références.

Chaque gène d'enveloppe a été sous-cloné dans un vecteur pCi-Rev qui dérive du vecteur pCi-Néo (Promega) par remplacement du gène de la *néomycine* par le gène rev qui se trouve alors sous la dépendance du promoteur SV40. Chaque construction a été préparée avec un Kit d'extraction de plasmide endotoxine-free (Macherey-Nagel PC2000 EF).

Les immunisations ont été effectuées de la façon suivante : pour chaque construction 5 souris Balb/C femelles ont été immunisées par biolistique (GeneGun, Biorad) à l'aide de particules d'or préalablement recouvertes de 4 µg de plasmide à injecter. Cinq injections successives ont eu lieu à J0, J14, J28, J54 et J68. A J40 et J80 des échantillons de sang sont prélevés dans l'oeil des animaux. Ces échantillons ont servi à tester la montée des anticorps dirigés contre l'enveloppe après les différentes immunisations. Pour ce faire, des ELISA ont été effectués : de la protéine d'enveloppe gp160 (ABL) diluée à 1µg/ml été adsorbée sur des plaques 96 puits (Nunc). Après saturation des sites non spécifiques, les sérums des souris immunisées aux jours J0, J40 et J80 sont utilisés à des dilutions allant de 1/100 à 1/800, puis révélés par un anticorps dirigé contre les anticorps de souris couplé à la peroxidase de raifort (Jackson). Une DO lue supérieure à 0,35 est considérée comme significative. Le tableau 5 ci-dessous récapitule les souris positives obtenues pour chaque construction.

**Tableau 5**

| **constructions** | **souris "positives" en ELISA** |
|---|---|
| pCI-Rev-g12 | S3 , S4 |
| pCI-Rev-g112 | S1, S2 |
| pCI-Rev-g13 | S5 |
| pCI-Rev-g14 | S3, S4 |
| pCI-Rev-g123 | S2, S4, S5 |
| pCI-Rev-env sauvage | S1, S2, S3, S4 |

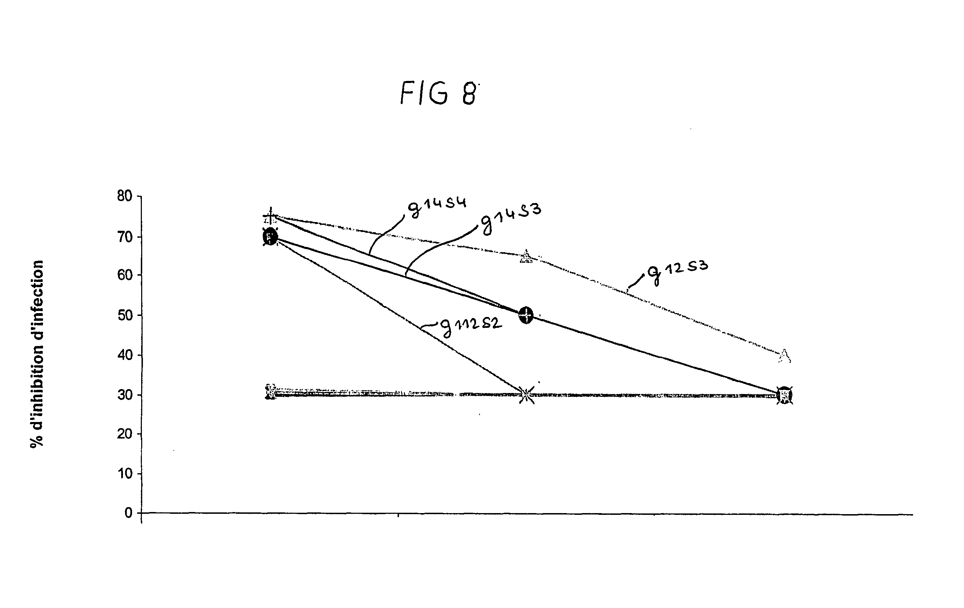

Les sérums de ces souris ont été utilisés dans deux tests de neutralisation décrits ci-après :
a- Le virus FR (virus primaire X4) a été mis en contact avec des dilutions sériées (au 1/10^{ième}, 1/20^{ième} et 1/40^{ième}) des différents sérums de souris pendant 1h à 37°C dans du milieu DMEM (Euromedex) puis rajouté dans des plaques 96 puits où ont été préalablement mises en culture des cellules Hela P4PCCR5. Ces cellules possèdent le récepteur CD4 et les co-récepteurs CCR5 et CXCR4. Elles ont le gène de la B-galactosidase, sous la dépendance d'un LTR rétroviral. Après 1h de co-culture, 200µl de milieu DMEM contenant 5% de sérum de veau foetal sont rajoutés. Les cellules sont alors incubées 48h à 37°C. A l'issue de ces 24h, l'expression de la B-galactosidase est revélée grâce à une solution d'X-gal à 400µg/ml en potassium ferricyanide 0,2M/potassium ferrocyanide 0,2M/MgCl₂ 2M. Le pourcentage de neutralisation correspond au pourcentage de cellules non infectées, pour lesquelles la synthèse de B-Galactosidase n'a pas été induite. Des essais ont été effectués en parallèle avec du virus mais sans sérum de souris (contrôle d'infection) ou avec du sérum de souris non immunisées (contrôle du bruit de fond de la neutralisation, Tneg). Les résultats obtenus sont résumés dans le tableau 6 ci-dessous et la figure 8.

**TABLEAU 6**

| | | | |
|---|---|---|---|
| TnegS1 | 30 | 30 | 30 |
| TnegS2 | 30 | 30 | 30 |
| g12-S3 | 75 | 65 | 40 |
| g12-S4 | 70 | 30 | 30 |
| g13-S5 | 30,5 | 30 | 30 |
| g14-S3 | 70 | 50 | 30 |
| g14-54 | 75 | 50 | 30 |
| g112-S1 | 30,2 | 30 | 30 |
| g112-S2 | 29,7 | 30 | 30 |
| g123-S3 | 30,5 | 30 | 30 |
| g123-S4 | 31,5 | 30 | 30 |
| g123-S5 | 31 | 30 | 30 |
| wt-S1 | 30,7 | 30 | 30 |
| wt-S2 | 30,5 | 30 | 29,7 |

On observe que pour la majorité des sérums, il n'y a pas de réponse neutralisante se différenciant du bruit de fond (environ 30% de neutralisation). Cependant, pour les constructions pCI-rev-g12 et pCI-rev-g14, les souris positives en ELISA (respectivement S3/S4 et S3/S4) donnent une réponse neutralisante significative à la dilution 1/10 (70% de neutralisation). Ce pourcentage est moindre pour une dilution au 1/20 et rejoint la valeur du bruit de fond pour une dilution au 1/40, excepté pour la souris S3 immunisée par pCI-rev-g12 dont la valeur est légèrement supérieure (40%). Les sérums des souris immunisées par les constructions non mutées (Pci-rev-env sauvage = wt) et positifs en ELISA ne sont pas neutralisants dans ce format de test.
b- Les sérums de souris ont été évalués pour leur capacité à neutraliser l'infection de cellules permissives par des pseudo-particules portant l'enveloppe 133 sauvage (= autologue non mutée). Les sérums des souris positives ainsi que 2 sérums de souris non immunisées ont été incubés pendant 1h à 37°C en dilutions sériées (de 1/8 à 1/128) avec les surnageants de culture contenant les pseudo-particules. Les pseudo-particules portant l'enveloppe 133 sauvage ont été obtenues de la façon suivante : Des cellules 293T ont été co-transfectées par la construction pCI-*env133* et par le plasmide pNL4-3*luc* (NIH AIDS Research and Reference Reagent Program) avec un rapport pNL4-3*luc*/pCI-*envl33* de 1/3. Cette transfection est réalisée grâce au kit Calcium Phosphate Transfection System (Gibco). Le plasmide pNL4-3*luc* permet d'exprimer tous les gènes viraux sauf vpr et env (saut de trame) ainsi que le gène de la Luciférase. Par complémentation, des pseudo-particules virales portant les enveloppes recombinantes 133 sauvage sont formées. Quarante-huit heures après transfection, les surnageants de culture sont récupérés et centrifugés afin d'éliminer les débris cellulaires. Ils sont ensuite complétés à 20% de sérum de veau foetal, aliquotés et stockés à -80°C. Un dosage de la p24, réalisé sur l'automate VIDAS (bioMérieux), permet d'évaluer la quantité de particules virales présente dans ces surnageants.

L'infection de cellules GHOST-CCR5 ou GHOST-CXCR4 (NIH AIDS Research and Reference Reagent Program ; ces cellules expriment le CD4) est effectuée sur la nuit, en plaques de culture de 96 puits, par contact avec un volume de surnageant contenant les pseudoparticules préalablement mises en contact avec les sérums (10ng d'équivalent p24 par puit). Du milieu de culture est ensuite rajouté. Après 3 jours supplémentaires d'incubation, les surnageants sont éliminés, les cellules sont rincées avec du PBS 1X. La lyse et la réaction enzymatique sont effectuées avec le kit LucLite Plus (Perkin Elmer) ; la lecture de la luminescence est réalisée sur un lecteur de plaques TopCount (Packard Biosciences).

Les résultats montrent que pour les sérums g12-S3, g14-S3 et g14-S4, on observe une légère neutralisation pour une dilution au 1/8 puisque, pour ces échantillons, la luminescence est environ 23% moins importante que celle enregistrée pour les sérums de souris avant immunisation (V_{g19-S3}= 85 000 contre V_{T0}= 111 000). Ces résultats semblent aller dans le même sens que ceux décrits dans l'expérience précédente. L'exception vient du sérum g12-S4 pour lequel aucune activité neutralisante n'est constatée.

### Exemple 5 : évaluation de la fonctionnalité de tous les mutants de déglycosylation : infection par des pseudo-virus et essais de neutralisation de cette infection par des sérums humains HIV-1 positifs.

L'obtention de pseudoparticules exprimant les différents mutants de déglycosylation est importante car elle permet d'étudier la protéine d'enveloppe dans son contexte naturel c'est à dire intégrée dans une particule virale.

Des cellules 293T ont été co-transfectées par chacune des constructions pCI-env mutées (mutants de déglycosylation, cf. liste sur tableau 2) et par le plasmide pNL4-31uc (NIH AIDS Research and Reference Reagent Program) suivant le protocole décrit dans l'exemple 1 ci-dessus.

Quarante-huit heures après transfection, les surnageants de culture sont récupérés et centrifugés afin d'éliminer les débris cellulaires. Ils sont ensuite complétés à 20% de sérum de veau foetal, aliquotés et stockés à -80°C. Un dosage de la p24, réalisé sur l'automate VIDAS (bioMérieux), permet d'évaluer la quantité de particules virales présentes dans ces surnageants.

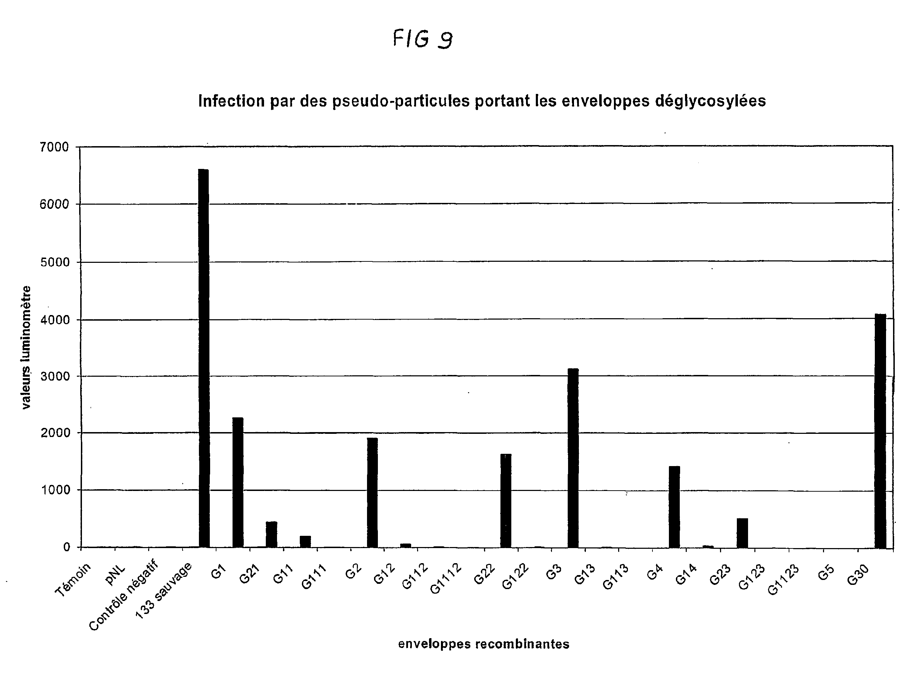

L'infection de cellules GHOST-CCR5 ou -CXCR4 (NIH AIDS Research and Reference Reagent Program) est effectuée sur la nuit, en plaques de culture de 24 puits, par contact avec un volume de surnageant correspondant à 50ng de p24 par puit. Le milieu de culture est alors renouvelé. Après 3 jours supplémentaires d'incubation, les surnageants sont éliminés, les cellules sont rincées avec du PBS 1X et lysées par 300µl de CCLR (Cell Culture Lysis Reagent de Promega) pendant 15 minutes. Un volume de 20µl de ces lysats est mis en contact avec 80µl de substrat (Luciferase Assay System de Promega) et la mesure de la luminescence est réalisée sur un luminomètre (Turner Designs TD 20/20). Les résultats sont regroupés dans la figure 9.

Cette expérience indique que plus le nombre de mutations est important, plus la capacité des enveloppes recombinantes à induire l'infection des GHOST-CCR5 diminue. Elle est nulle pour les constructions qui sont peu ou pas clivées. Aucun mutant n'induit l'infection des cellules GHOST-CXCR4. Ces résultats sont en accord avec les tests de formation de syncytia précédemment décrits (cf. tableau 4). Cependant aucune infection n'est détectée pour les mutants g112, g122, g13 et g123, alors qu'une légère activité de fusion cellule à cellule était observée. Pour le mutant g14, utilisé dans les expériences de neutralisation, un faible taux d'infection est observé. Afin de compléter cette étude, nous avons contrôlé la présence de la protéine d'enveloppe dans les virions. Pour cela, nous avons concentré et purifié les pseudo-particules par ultracentrifugation sur un coussin de saccharose 20% pendant 3h à 70000g. Les culots ont été lysés en PBS-Triton 0.5% et un dosage de la p24 a permis de normaliser les échantillons. Un Western-Blot a alors été réalisé en utilisant un anticorps polyclonal anti-gp120 (Biogenesis). Env est détectée uniquement dans les pseudo-particules qui infectent les cellules GHOST : lorsqu'on augmente le nombre de sites déglycosylés, l'exportation de la protéine d'enveloppe à la membrane plasmique ainsi que l'incorporation de l'enveloppe dans les pseudo-particules semble être affectée.

Enfin, la sensibilité de ces pseudo-particules vis-à-vis de sérums neutralisants a été évaluée : avant l'infection de cellules indicatrices GHOST-CCR5, les surnageants de culture contenant les pseudo-particules sont incubés pendant 1h à 37°C en présence de dilutions sériées de sérum humain. Il s'agit du sérum GRI/LY (don du Dr C. Moog, INSERM U544, Strasbourg) dont le titre neutralisant a été déterminé pour 5 isolats primaires de différents sous- types. En parallèle, les sérums HIV-1 neutralisants de référence #1 et #2 (NIBSC) ont été utilisés en contrôle sur les pseudo-particules portant l'enveloppe 133 native. Le protocole d'infection est similaire à celui décrit plus haut. L'infection est réalisée en plaques 96 puits par contact avec un volume équivalent à 10ng de p24. La lyse et la réaction enzymatique sont effectuées avec le kit LucLite Plus (Perkin Elmer). La lecture de la luminescence est réalisée sur un lecteur de plaques TopCount (Packard Biosciences).

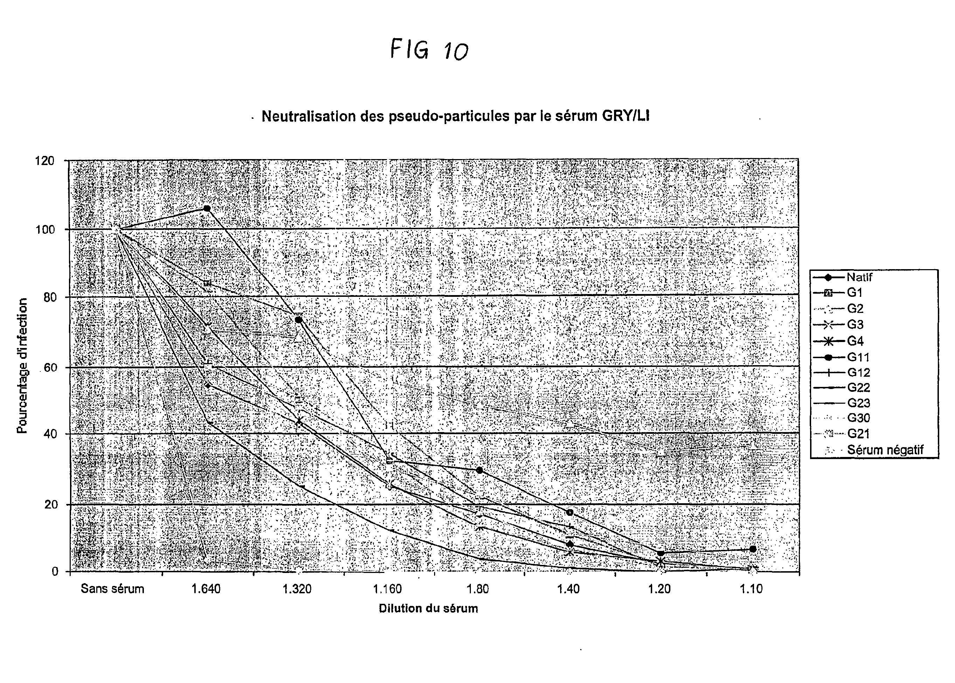

Les résultats sont illustrés dans la figure 10.

La courbe « sérum négatif » correspond à une expérience menée sur des pseudo-virions exprimant l'enveloppe sauvage mis en présence d'un sérum HIV-1 négatif (CTS Lyon-Gerland). C'est la courbe déterminant le bruit de fond. La neutralisation est effective pour une dilution de sérum supérieure à 1/640^{ème}.

Ces résultats montrent que les pseudo-particules sont neutralisées différemment suivant l'enveloppe exprimée à leur surface. Cependant, seuls les mutants g30 et g22 voient leur pourcentage d'infection significativement réduit par rapport à l'enveloppe sauvage (non mutée). Même si il n'y a pas de corrélation directe entre cette expérience et la capacité d'un tel mutant à induire des anticorps neutralisants in vivo, le mutant g22 pourrait être un candidat intéressant à tester pour sa capacité à induire des anticorps neutralisants après immunisation ADN de petits animaux.

### REFERENCES BIBLIOGRAPHIQUES

1. Moore, JP et Ho, DD, AIDS (1995), 9 suppl. A, pS117-S136
2. Trkola, A et al., Nature (1996) 384 (6605), p184-187
3. Girard, M et al., J. Virol. (1995) 69(10) p6239-6248
4. Poignard, P et al., Immunity (1999) 10(4) p431-438
5. McInerney, TL et al., J. Virol. (1998) 72(2) p1523-1533
6. Reitter, JN, Means RE et Desrosiers RC, Nat. Med (1998) 4(6) p679-684
7. Lee, WR et al., PNAS (1992) 89 p2213-2217
8. Ly A. et Stamatatos L, J. Virol. (1998) 74 p6769-6776
9. Kwong, PD, et al., Nature (1998) 393 (6686) p648-659
10. Zhu, X et al., Biochem (2000) 39 p11194-11204
11. Wyatt, R et al., Nature (1998) 393 (6686) p705-711
12. Kwong, PD et al., Structure (2000) 8 p1329-1339
13. Ataman-Önal, Y et al., AIDS Res. Hum. Retroviruses (1999) 15 (11) p1035-1039
14. Trkolla, A et al., J. Virol. (1996) 69 p6609-6617
15. Moullard, M et Decrolly, E, BBA (2000) 1469 p121-132
16. Pollakis, P et al., J. Bio. Chem. (2001) 276 p13433-13441
17. Buchacher, A et al., AIDS Res. & Hum. Ret. (1994) 10 p359-369
18. Moore, JP et al., J. Virol. (1995) 69 p101-109
19. Posner, M et al., J. AIDS (1993) 6 p7-14
20. Thali, M et al., J. Virol. (1992) 66 p5635-5641
21. Gershoni, JM et al., FASEB J. (1993) 7 p1185-1187
22. Wain-Hobson, L et al., Cell (1985) 40 p9-17
23. Ratner, L et al., Nature (1985) 313 p277-284
24. Muesing, MA et al., Nature (1985) 313 p450-458
25. Sanchez-Pescador et al., Science (1985) 227 p484-492.

## Revendications

1. Gène env muté codant pour une qlycoprotéine d'enveloppe mutée du virus VTH-1 *d'un isolat primaire*, **caractérisé en ce que** ledit gène présente par rapport au gène *env* d'un isolat primaire de primo-infection, dit de référence, au moins deux mutations au niveau des sites de glycosylation conservés d'un isolat primaire à un autre, chaque mutation consistant en le remplacement d'un codon AAC ou AAT qui code pour une asparagine par un codon CAG ou CAA qui code pour une glutamine, les deux mutations au moins étant choisies parmi les suivantes :
(a) au moins deux mutations dans la partie du gène env codant pour la région C3 de la protéine env,
(b) au moins une mutation dans la partie codant pour la région C3 et au moins une mutation dans la partie codant pour la région V3,
(c) au moins une mutation dans la partie codant pour la région C2 et au moins une mutation dans la partie codant pour la région V3 choisie parmi les sites de glycosylation au niveau des codons 862-864 et 970-972,
(d) au moins une mutation dans la partie codant pour la région C2 choisie parmi les sites de glycosylation au niveau des codons 667-669, 679-681, 700-702, 763-765, 844-846, eL au moins une mutation dans la partie codant pour la région V3,
la position d'au moins l'un quelconque desdits codons pouvant varier de trois à vingt-quatre nucléotides,
ou le gène *env* muté consiste en l'une quelconque des séquences SEQ ID NO: 8, SEQ ID NO: 9 ou SEQ ID NO: 10 ou leurs séquences complémentaires,

2. Gène selon la revendication 1, **caractérisé en ce que** selon les mutations (a), au moins une mutation est effectuée au niveau des codons 976-978, 991-993 de la partie codant pour la région C3 et au moins une mutation est effectuée au niveau des codons 1039-1041 ou 1060-1062 de la partie codant pour la région C3, la position d'au moins l'un quelconque desdits codons pouvant varier de trois à vingt-quatre nucléotides.

3. Gène selon la revendication 2, **caractérisé en ce que** sa séquence consiste en une séquence choisie parmi les séquences identifiées en SEQ ID NO: 21, SEQ ID NO: 22 et SEQ ID NO:23 ou leurs séquences complémentaires.

4. Gène selon la revendication 1, **caractérisé en ce que** selon les mutations (b), au moins une mutation est effectuée au niveau des codons 976-978, 991-993, 1039-1041 ou 1060-1062 de la partie codant pour la région C3 et au moins une mutation est effectuée au niveau du codon 880-882 de la partie codant pour la région V3, la position d'au moins l'un quelconque desdits codons pouvant varier de trois à vingt-quatre nucléotides.

5. Gène selon la revendication 4, **caractérisé en ce que** les mutations sont effectuées au niveau des codons 976-978, 991-993 et 880-882, la position d'au moins l'un quelconque desdits codons pouvant varier de trois à vingt-quatre nucléotides.

6. Gène selon la revendication 5, **caractérisé en ce que** sa séquence consiste en une séquence choisie parmi les séquences identifiées en SEQ ID NO: 2, SEQ ID NO: 3 et SEQ ID NO:4 ou leurs séquences complémentaires.

7. Gène selon la revendication 4, **caractérisé en ce que**, au moins une mutation est effectuée au niveau du codon 976-978 ou 991-993, au moins une mutation est effectuée au niveau du codon 880-882, et au moins une mutation est effectuée au niveau du codon 1039-1041 ou 1060-1062 , la position d'au moins l'un quelconque desdits codons pouvant varier de trois à vingt-quatre nucléotides.

8. Gène selon la revendication 7, **caractérisée en ce que** les mutations sont effectuées au niveau des codons 976-978, 991-993, 880-882, 1039-1041 et 1060-1062.

9. Gène selon la revendication 8, **caractérisé en ce que** sa séquence consiste en une séquence choisie parmi les séquences identifiées en SEQ ID NO : b, SEQ ID NO : 6 et SEQ ID NO : 7 ou leurs séquences complémentaires.

10. Gène selon la revendication 1, **caractérisé en ce que** selon les mutations (c), au moins une mutation est effectuée au niveau du codon 805-807 de la partie codant pour la région C2, la position d'au moins l' un quelconque desdits codons pouvant varier de trois à vingt-quatre nucléotides.

11. Gène selon la revendication 1, **caractérisé en ce que** selon les mutations (d), au moins une mutation est effectuée au niveau du codon 880-882 de la partie codant pour la région V3, la position d'au moins l'un quelconque desdits codons pouvant varier de trois à vingt-quaLre nucléotides.

12. Glycoprotéine Env mutée du virus VIii-1 *d'un isolat primaire,* **caractérisée en ce qu'**elle présente par rapport à une protéine Env native d'un isolat primaire de primo-infection, dit de référence, au moins deux mutations au niveau dcs sites de glycosylation conservés de ladite protéine de référence, d'un isolat primaire à un autre, chaque mutation consistant en le remplacement d'une asparagine par une glutamine, les deux mutations au moins étant choisies parm les suivantes :
(a') au moins deux mutations dans la région C3 de la protéine Env,
(b') au moins une mutation dans la région C3 et au moins une mutation dans la région V3,
(c') au moins une mutation dans la région C2 et au moins une mutation dans la région V3 choisie parmi les sites de glycosylation au niveau de l'acide aminé 288 ou 324,
(d') au moins une mutation dans la région C2 choisie parmi les sites de glycosylation au niveau de l'un quelconque des acides aminés 223, 227, 234, 255, 282, et au moins une mutation dans 1a région V3,
la position desdits sites de glycosylation conservés d'un isolat primaire de primo-infection à un autre pouvant varier d'un à huit acides aminés,
ou la glycoprotéine Env mutée consiste en l'une quelconque des séquences SEQ ID NO: 18, SEQ ID NO: 19 ou SEQ ID NO: 20.

13. Glycoprotéine selon la revendication 12, **caractérisée en ce que** selon les mutations (a'), au moins une mutation est effectuée au site de glycosylation au niveau de l'acide aminé 326 ou 331 et au moins une mutation est effectuée au site de glycosylation au niveau de l'acide aminé 347 ou 354,
la position desdits sites de glycosylation conservés d'un isolat primaire de primo-infection à un autre pouvant varier d'un à huit acides aminés.

14. Glycoprotéine selon la revendication 13, **caractérisée en ce que** sa séquence consiste en une séquence choisie parmi les séquences identifiées en SEQ ID NO: 21, SEQ ID NO: 22 et SEQ ID NO: 23.

15. Glycoprotéine selon la revendication 12, **caractérisée en ce que** selon les mutations (b'), au moins une mutation est effectuée au niveau de l'acide aminé 326 ou 331 et au moins une mutation est effectuée au niveau de l'acide aminé 294,
la position d'au moins l'un quelconque desdits sites de glycosylation conservés d'un isolat primaire de primo-infection à un autre, pouvant varier d'un à huit acides aminés.

16. Glycoprotéine selon la revendication 15, **caractérisée en ce que** les mutations sont effectuées au niveau des acides aminés 326, 331 et 294,
la position d'au moins l'un quelconque desdits sites de glycosylation conservés d'un isolat primaire de primo-infection à un autre pouvant varier d'un à huit acides aminés.

17. Glycoprotéine selon la revendication 16, **caractérisée en ce que** sa séquence consiste en une séquence choisie parmi les séquences identifiées en SEQ ID NO: 12, SEQ ID NO: 13 et SEQ ID NO: 14.

18. Glycoprotéine selon la revendication 12, **caractérisée en ce**, selon les mutations (b'), au moins une mutation est effectuée au niveau de l'acide aminé 326 ou 331, au moins une mutation est effectuée au niveau de l'acide aminé 347 ou 354 et au moins une mutation est effectuée au niveau de l'acide aminé 294,
la position d'au moins l'un quelconque desdits sites de glycosylation conservés d'un isolat primaire de primo-infection à un autre pouvant varier d'un à huit acides aminés.

19. Glycoprotéine selon la revendication 18, **caractérisée en ce que** les mutations sont effectuées au niveau des acides aminés 326, 331, 294, 347 et 354,
la position d'au moins l'un quelconque desdits sites de glycosylation conservés d'un isolat primaire de primo-infection à un autre pouvant varier d'un à huit acides aminés.

20. Glycoprotéine selon la revendication 19, **caractérisée en ce que** sa séquence consiste en une séquence choisie parmi les séquences identifiées en SEQ ID NO: 15, SEQ ID NO: 16 et SEQ ID NO: 17.

21. Glycoprotéine selon la revendication 12, **caractérisée en ce que**, selon les mutations (c'), au moins une mutation est effectuée au niveau de l'acide aminé 269 de la région C2, la position desdits sites de glycosylation conservés d'un isolat primaire de primo-infection à un autre pouvant varier d'un à huit acides aminés.

22. Glycoprotéine selon la revendication 12, **caractérisée en ce que**, selon les mutations (d'), au moins une mutation est effectuée au niveau de l'acide aminé 294 de la région V3, la position desdits sites de glycosylation conservés d'un isolat primaire de primo-infection à un autre pouvant varier d'un à huit acides aminés.

23. Composition pharmaceutique comprenant :
(i) au moins un gène muté codant pour une glycoprotéine d'enveloppe mutée du virus VIH-1, ledit gène muté étant choisi parmi l'un quelconque des gènes décrits dans l'une quelconque des revendications 1 à 11 et étant placé sous le contrôle de séquences de régulation permettant son expression dans une cellule hôte,
un véhicule pharmaceutiquement acceptable, et
de manière facultative un agent additionnel qui facilite la pénétration dudit gène muté dans ladite cellule et/ou qui permet de cibler ladite cellule ; ou
(ii) au moins le gène identifié en (i) cloné dans un vecteur viral recombinant.

24. Composition pharmaceutique comprenant :
- au moins une glycoprotéine d'enveloppe mutée du virus VIH-1, ladite glycoprotéine mutée étant choisie parmi l'une quelconque des glycoprotéines décrites dans l'une quelconque des revendications 12 à 22,
- un véhicule et/ou excipient et/ou adjuvant et/ou diluant pharmaceutiquement acceptable.

25. Procédé pour obtenir des anticorps selon lequel on immunise un animal mammifère non-humain, tel que souris ou rat ou singe, avec au moins un gène muté codant pour une glycoprotéine d'enveloppe mutée du virus VIH-1, ledit gène muté étant choisi parmi l'un quelconque des gènes décrits dans l'une quelconque des revendications 1 à 11 et étant placé sous le contrôle de séquences de régulation permettant son expression *in vivo.*

26. Procédé pour obtenir des anticorps selon lequel on immunise un animal mammifère non-humain, tel que souris ou rat ou singe, avec au moins une glycoprotéine d'enveloppe mutée du virus VIH-1, ladite glycoprotéine mutée étant choisie parmi l'une quelconque des glycoprotéines décrite dans l'une quelconque des revendications 12 à 22.

27. Procédé d' évaluation d'un agent thérapeutique, selon lequel on administre à un animal non-humain au moins un gène muté codant pour une glycoprotéine d'enveloppe mutée du virus VIH-1 tel que décrit dans l'une quelconque des revendications 1 à 11, et on réalise :
- un dosage d'anticorps spécifiques de la glycoprotéine mutée, et/ou
- un dosage des anticorps neutralisants spécifiques de la glycoprotéine mutée ou native, et/ou
- un dosage de 1a réponse immune cellulaire induite contre la qlycoprotéine mutée par exemple par un test d'activation in vitro de cellules lymphocytes T « helper » spécifique(s) de la glycoprotéine mutée.

## Claims

1. Mutated env gene encoding a mutated envelope glycoprotein of the HIV-1 virus of a primary isolate, **characterized in that** said gene exhibits, compared to the env gene of a "reference" primary infection primary isolate, at least two mutations at the glycosylation sites conserved from one primary isolate to another, each mutation consisting of replacement of an AAC or AAT codon which encodes an asparagine with a CAG or CAA codon which encodes a glutamine, the two mutations at least being chosen from the following:
(a) at least two mutations in the part of the env gene encoding the C3 region of the env protein,
(b) at least one mutation in the part encoding the C3 region and at least one mutation in the part encoding the V3 region,
(c) at least one mutation in the part encoding the C2 region and at least one mutation in the part encoding the V3 region chosen from the glycosylation sites at codons 862-864 and 970-972,
(d) at least one mutation in the part encoding the C2 region chosen from the glycosylation sites at codons 667-669, 679-681, 700-702, 763-765 and 844-846, and at least one mutation in the part encoding the V3 region,
it being possible for the position of at least any one of said codons to vary by three to twenty-four nucleotides,
or the mutated env gene consists of any one of the sequences SEQ ID NO: 8, SEQ ID NO: 9 or SEQ ID NO: 10 or the sequences complementary thereto.

2. Gene according to Claim 1, **characterized in that**, according to the mutations (a), at least one mutation is effected at codons 976-978, 991-993 of the part encoding the C3 region and at least one mutation is effected at codons 1039-1041 or 1060-1062 of the part encoding the C3 region, it being possible for the position of at least any one of said codons to vary by three to twenty-four nucleotides.

3. Gene according to Claim 2, **characterized in that** its sequence consists of a sequence chosen from the sequences identified in SEQ ID NO: 21, SEQ ID NO: 22 and SEQ ID N0:23, or the sequences complementary thereto.

4. Gene according to Claim 1, **characterized in that**, according to the mutations (b), at least one mutation is effected at codons 976-978, 991-993, 1039-1041 or 1060-1062 of the part encoding the C3 region and at least one mutation is effected at codon 880-882 of the part encoding the V3 region, it being possible for the position of at least any one of said codons to vary by three to twenty-four nucleotides.

5. Gene according to Claim 4, **characterized in that** the mutations are effected at codons 976-978, 991-993 and 880-882, it being possible for the position of at least any one of said codons to vary by three to twenty-four nucleotides.

6. Gene according to Claim 5, **characterized in that** its sequence consists of a sequence chosen from the sequences identified in SEQ ID NO: 2, SEQ ID NO: 3 and SEQ ID NO: 4, or the sequences complementary thereto.

7. Gene according to Claim 4, **characterized in that** at least one mutation is effected at codon 976-978 or 991-993, at least one mutation is effected at codon 880-882, and at least one mutation is effected at codon 1039-1041 or 1060-1062, it being possible for the position of at least any one of said codons to vary by three to twenty-four nucleotides.

8. Gene according to Claim 7, **characterized in that** the mutations are effected at codons 976-978, 991-993, 880-882, 1039-1041 and 1060-1062.

9. Gene according to Claim 8, **characterized in that** its sequence consists of a sequence chosen from the sequences identified in SEQ ID NO: 5, SEQ ID NO: 6 and SEQ ID NO: 7, or the sequences complementary thereto.

10. Gene according to Claim 1, **characterized in that**, according to the mutations (c), at least one mutation is effected at codon 805-807 of the part encoding the C2 region, it being possible for at least any one of said codons to vary by three to twenty-four nucleotides.

11. Gene according to Claim 1, **characterized in that** according to the mutations (d), at least one mutation is effected at codon 880-882 of the part encoding the V3 region, it being possible for the position of at least any one of said codons to vary by three to twenty-four nucleotides.

12. Mutated Env glycoprotein of the HIV-1 virus of a primary isolate, **characterized in that** it exhibits, compared to a native Env protein of a "reference" primary infection primary isolate, at least two mutations at the glycosylation sites of said reference protein which are conserved from one primary isolate to another, each mutation consisting of replacement of an asparagine with a glutamine, the two mutations at least being chosen from the following:
(a') at least two mutations in the C3 region of the Env protein,
(b') at least one mutation in the C3 region and at least one mutation in the V3 region,
(c') at least one mutation in the C2 region and at least one mutation in the V3 region chosen from the glycosylation sites at amino acid 288 or 324,
(d') at least one mutation in the C2 region chosen from the glycosylation sites at any one of amino acids 223, 227, 234, 255 and 282, and at least one mutation in the V3 region,
it being possible for the position of said glycosylation sites conserved from one primary infection primary isolate to another to vary by one to eight amino acids,
or the mutated Env glycoprotein consists of any one of the sequences SEQ ID NO: 18, SEQ ID NO: 19 or SEQ ID NO: 20.

13. Glycoprotein according to Claim 12, **characterized in that**, according to the mutations (a'), at least one mutation is effected at the glycosylation site at amino acid 326 or 331 and at least one mutation is effected at the glycosylation site at amino acid 347 or 354,
it being possible for the position of said glycosylation sites conserved from one primary infection primary isolate to another to vary by one to eight amino acids.

14. Glycoprotein according to Claim 13, **characterized in that** its sequence consists of a sequence chosen from the sequences identified in SEQ ID NO: 21, SEQ ID NO: 22 and SEQ ID NO: 23.

15. Glycoprotein according to Claim 12, **characterized in that**, according to the mutations (b'), at least one mutation is effected at amino acid 326 or 331 and at least one mutation is effected at amino acid 294,
it being possible for the position of at least any one of said glycosylation sites conserved from one primary infection primary isolate to another to vary by one to eight amino acids.

16. Glycoprotein according to Claim 15, **characterized in that** the mutations are effected at amino acids 326, 331 and 294,
it being possible for the position of at least any one of said glycosylation sites conserved from one primary infection primary isolate to another to vary by one to eight amino acids.

17. Glycoprotein according to Claim 16, **characterized in that** its sequence consists of a sequence chosen from the sequences identified in SEQ ID NO: 12, SEQ ID NO: 13 and SEQ ID NO: 14.

18. Glycoprotein according to Claim 12, **characterized in that**, according to the mutations (b'), at least one mutation is effected at amino acid 326 or 331, at least one mutation is effected at amino acid 347 or 354, and at least one mutation is effected at amino acid 294,
it being possible for the position of at least any one of said glycosylation sites conserved from one primary infection primary isolate to another to vary by one to eight amino acids.

19. Glycoprotein according to Claim 18, **characterized in that** the mutations are effected at amino acids 326, 331, 294, 347 and 354,
it being possible for the position of at least any one of said glycosylation sites conserved from one primary infection primary isolate to another to vary by one to eight amino acids.

20. Glycoprotein according to Claim 19, **characterized in that** its sequence consists of a sequence chosen from the sequences identified in SEQ ID NO: 15, SEQ ID NO: 16 and SEQ ID NO: 17.

21. Glycoprotein according to Claim 12, **characterized in that**, according to the mutations (c'), at least one mutation is effected at amino acid 269 of the C2 region, it being possible for the position of said glycosylation sites conserved from one primary infection primary isolate to another to vary by one to eight amino acids.

22. Glycoprotein according to Claim 12, **characterized in that**, according to the mutations (d'), at least one mutation is effected at amino acid 294 of the V3 region, it being possible for the position of said glycosylation sites conserved from one primary infection primary isolate to another to vary by one to eight amino acids.

23. Pharmaceutical composition comprising:
(i) at least one mutated gene encoding a mutated envelope glycoprotein of the HIV-1 virus, said mutated gene being chosen from any one of the genes described in any one of Claims 1 to 11 and being placed under the control of regulatory sequences which allow its expression in a host cell,
a pharmaceutically acceptable vehicle,
and optionally, an additional agent which facilitates the penetration of said mutated gene into said cell and/or which makes it possible to target said cell, or
(ii) at least the gene identified in (i) cloned into a recombinant viral vector.

24. Pharmaceutical composition comprising:
- at least one mutated envelope glycoprotein of the HIV-1 virus, said mutated glycoprotein being chosen from any one of the glycoproteins described in any one of Claims 12 to 22,
- a pharmaceutically acceptable vehicle and/or excipient and/or adjuvant and/or diluent.

25. Method for obtaining antibodies, according to which a non human mammalian animal, such as a mouse, rat or monkey, is immunized with at least one mutated gene encoding a mutated envelope glycoprotein of the HIV-1 virus, said mutated gene being chosen from any one of the genes described in any one of Claims 1 to 11 and being placed under the control of regulatory sequences which allow its expression *in vivo.*

26. Method for obtaining antibodies, according to which a non human mammalian animal, such as a mouse, rat or monkey, is immunized with at least one mutated envelope glycoprotein of the HIV-1 virus, said mutated glycoprotein being chosen from any one of the glycoproteins described in any one of Claims 12 to 22.

27. Method for evaluating a therapeutic agent, according to which at least one mutated gene encoding a mutated envelope glycoprotein of the HIV-1 virus as described in any one of Claims 1 to 11 is administered to a non human animal, and the following are carried out:
- measurement of the titre of antibodies specific for the mutated glycoprotein, and/or
- measurement of the titre of neutralizing antibodies specific for the mutated or native glycoprotein, and/or
- measurement of the cellular immune response induced against the mutated glycoprotein, for example using an assay for the in vitro activation of T "helper" lymphocyte cells specific for the mutated glycoprotein.

## Patentansprüche

1. Mutiertes env-Gen, das für ein mutiertes Hüll-Glycoprotein des HIV-1-Virus *eines Primärisolats* codiert, **dadurch gekennzeichnet, daß** dieses Gen im Vergleich zu dem env-Gen eines Erstinfektions-Primärisolats, Bezugsgen genannt, mindestens zwei Mutationen an den Glycosylierungsstellen, die von einem Primärisolat zu einem anderen Isolat konserviert sind, aufweist, wobei jede Mutation aus der Substitution eines AAC- oder AAT-Codons, das für ein Asparagin codiert, durch ein CAG- oder CAA-Codon, das für ein Glutamin codiert, besteht, wobei die beiden Mutationen mindestens aus der Reihe der folgenden Mutationen stammen:
(a) mindestens zwei Mutationen in demjenigen Abschnitt des env-Gens, der für die C3-Region des env-Proteins codiert,
(b) mindestens eine Mutation in demjenigen Abschnitt, der für die C3-Region codiert, sowie mindestens eine Mutation in demjenigen Abschnitt, der für die V3-Region codiert,
(c) mindestens eine Mutation in demjenigen Abschnitt, der für die C2-Region codiert, sowie mindestens eine Mutation in demjenigen Abschnitt, der für die V3-Region codiert, aus der Reihe der Glycosylierungsstellen an den Codons 862-864 und 970-972,
(d) mindestens eine Mutation in demjenigen Abschnitt, der für die C2-Region codiert, aus der Reihe der Glycosylierungsstellen an den Codons 667-669, 679-681, 700-702, 763-765 und 844-846, sowie mindestens eine Mutation in demjenigen Abschnitt, der für die V3-Region codiert, wobei die Lage des beliebigen, mindestens einen dieser Codons im Bereich von 3 bis 24 Nukleotide schwanken kann,
wobei das mutierte env-Gen aus einer beliebigen Sequenz der Sequenzen SEQ ID NO: 8, SEQ ID NO: 9 oder SEQ ID NO: 10 oder ihren komplementären Sequenzen besteht.

2. Gen nach Anspruch 1, **dadurch gekennzeichnet, daß** gemäß den Mutationen (a) mindestens eine Mutation an den Codons 976-978, 991-993 des für die C3-Region codierenden Abschnitts und mindestens eine Mutation an den Codons 1039-1041 oder 1060-1062 des für die Region C3 codierenden Abschnitts durchgeführt wird, wobei die Lage des beliebigen mindestens einen dieser Codons im Bereich von 3 bis 24 Nukleotide schwanken kann.

3. Gen nach Anspruch 2, **dadurch gekennzeichnet, daß** seine Sequenz aus einer Sequenz aus der in SEQ ID NO: 21, SEQ ID NO: 22 und SEQ ID NO:23 identifizierten Sequenzen oder ihren komplementären Sequenzen stammt.

4. Gen nach Anspruch 1, **dadurch gekennzeichnet, daß** gemäß den Mutationen (b) mindestens eine Mutation an den Codons 976-978, 991-993, 1039-1041 oder 1060-1062 des für die C3-Region codierenden Abschnitts und mindestens eine Mutation am Codon 880-882 des für die Region V3 codierenden Abschnitts durchgeführt wird, wobei die Lage des beliebigen mindestens einen dieser Codons im Bereich von 3 bis 24 Nukleotide schwanken kann.

5. Gen nach Anspruch 4, **dadurch gekennzeichnet, daß** die Mutationen an den Codons 976-978, 991-993 und 880-882 durchgeführt werden, wobei die Lage des beliebigen mindestens einen dieser Codons im Bereich von 3 bis 24 Nukleotide schwanken kann.

6. Gen nach Anspruch 5, **dadurch gekennzeichnet, daß** die Sequenz aus einer Sequenz aus der in SEQ ID NO: 2, SEQ ID NO: 3 und SEQ ID NO: 4, identifizierten Sequenzen oder ihren komplementären Sequenzen stammt.

7. Gen nach Anspruch 4, **dadurch gekennzeichnet, daß** mindestens eine Mutation am Codon 976-978 oder 991-993, mindestens eine Mutation am Codon 880-882 und mindestens eine Mutation am Codon 1039-1041 oder 1060-1062 durchgeführt wird, wobei die Lage des beliebigen mindestens einen dieser Codons im Bereich von 3 bis 24 Nukleotide schwanken kann.

8. Gen nach Anspruch 7, **dadurch gekennzeichnet, daß** die Mutationen an den Codons 976-978, 991-993, 880-882, 1039-1041 und 1060-1062 durchgeführt werden.

9. Gen nach Anspruch 8, **dadurch gekennzeichnet, daß** seine Sequenz aus einer Sequenz aus der Rehe der in SEQ ID NO: 5, SEQ ID NO: 6 and SEQ ID NO: 7, identifizierten Sequenzen oder ihren komplementären Sequenzen stammt.

10. Gen nach Anspruch 1, **dadurch gekennzeichnet, daß** gemäß den Mutationen (c) mindestens eine Mutation am Codon 805-807 des für die C2-Region codierenden Abschnitts durchgeführt wird, wobei die Lage des beliebigen mindestens einen dieser Codons im Bereich von 3 bis 24 Nukleotide schwanken kann.

11. Gen nach Anspruch 1, **dadurch gekennzeichnet, daß** gemäß den Mutationen (d) mindestens eine Mutation am Codon 880-882 des für die V3-Region codierenden Abschnitts durchgeführt wird, wobei die Lage des beliebigen mindestens einen dieser Codons im Bereich von 3 bis 24 Nukleotide schwanken kann.

12. Mutiertes Env-Glycoprotein des HIV-1-Virus eines *Primärisolats,* **dadurch gekennzeichnet, daß** es im Vergleich zu einem nativen Env-Protein eines Erstinfektions-Primärisolats, das als Referenzprotein bezeichnet wird, mindestens zwei Mutationen an den zwischen einem Primärisolat zu einem anderen konservierten Glycosylierungsstellen dieses Referenzproteins aufweist, wobei jede Mutation aus der Substitution eines Asparagin durch ein Glutamin besteht, wobei die beiden Mutationen mindestens aus den folgenden Mutationen stammen:
(a') mindestens zwei Mutationen in der C3-Region des Env-Proteins,
(b') mindestens eine Mutation in der C3-Region sowie mindestens eine Mutation in der V3-Region,
(c') mindestens eine Mutation in der C2-Region sowie mindestens eine Mutation in der V3-Region aus der Reihe der Glycosylierungsstellen an der Aminosäure 288 oder 324,
(d') mindestens eine Mutation in der C2-Region aus der Reihe der Glycosylierungsstellen an der beliebigen, einen Aminosäure aus der Reihe der Aminosäuren 223, 227, 234, 255 und 282, sowie mindestens eine Mutation in der V3-Region,
wobei die Lage dieser von einem Erstinfektions-Primärisolat zu einem anderen konservierten Glycosylierungsstellen im Bereich von einer bis acht Aminosäuren schwanken kann,
wobei das Env-Glycoprotein aus einer beliebigen Sequenz der Sequenzen SEQ ID NO: 18, SEQ ID NO: 19 oder SEQ ID NO: 20 besteht.

13. Glycoprotein nach Anspruch 12, **dadurch gekennzeichnet, daß** gemäß den Mutationen (a') mindestens eine Mutation an der Glycosylierungsstelle an der Aminosäure 326 oder 331 und mindestens eine Mutation an der Glycosylierungsstelle an der Aminosäure 347 oder 354 durchgeführt werden,
wobei die Lage dieser zwischen einem Erstinfektions-Primärisolat und einem anderen konservierten Glycosylierungsstellen im Bereich von einer bis acht Aminosäuren schwanken kann.

14. Glycoprotein nach Anspruch 13, **dadurch gekennzeichnet, daß** seine Sequenz aus einer Sequenz aus der Reihe der in SEQ ID NO: 21, SEQ ID NO: 22 und SEQ ID NO: 23 identifizierten Sequenzen stammt.

15. Glycoprotein nach Anspruch 12, **dadurch gekennzeichnet, daß** gemäß den Mutationen (b') mindestens eine Mutation an der Aminosäure 326 oder 331 und mindestens eine Mutation an der Aminosäure 294 durchgeführt wird,
wobei die Lage der beliebigen, mindestens einen dieser zwischen einem Erstinfektions-Primärisolat und einem anderen konservierten Glycosylierungsstellen im Bereich von einer bis acht Aminosäuren schwanken kann.

16. Glycoprotein nach Anspruch 15, **dadurch gekennzeichnet, daß** die Mutationen an den Aminosäuren 326, 331 und 294 durchgeführt werden,
wobei die Lage der beliebigen, mindestens einen dieser zwischen einem Erstinfektions-Primärisolat und einem anderen konservierten Glycosylierungsstellen im Bereich von einer bis acht Aminosäuren schwanken kann.

17. Glycoprotein nach Anspruch 16, **dadurch gekennzeichnet, daß** seine Sequenz aus einer Sequenz aus der Reihe der in SEQ ID NO: 12, SEQ ID NO: 13 und SEQ ID NO: 14 identifizierten Sequenzen stammt.

18. Glycoprotein nach Anspruch 12, **dadurch gekennzeichnet, daß** gemäß den Mutationen (b') mindestens eine Mutation an der Aminosäure 326 oder 331, mindestens eine Mutation an der Aminosäure 347 oder 354 und mindestens eine Mutation an der Aminosäure 294 durchgeführt wird,
wobei die Lage der beliebigen, mindestens einen dieser zwischen einem Erstinfektions-Primärisolat und einem anderen konservierten Glycosylierungsstellen im Bereich von einer bis acht Aminosäuren schwanken kann.

19. Glycoprotein nach Anspruch 18, **dadurch gekennzeichnet, daß** die Mutationen an den Aminosäuren 326, 331, 294, 347 und 354, durchgeführt werden,
wobei die Lage der beliebigen, mindestens einen dieser zwischen einem Erstinfektions-Primärisolat und einem anderen konservierten Glycosylierungsstellen im Bereich von einer bis acht Aminosäuren schwanken kann.

20. Glycoprotein nach Anspruch 19, **dadurch gekennzeichnet, daß** seine Sequenz aus einer Sequenz aus der Reihe der in SEQ ID NO: 15, SEQ ID NO: 16 und SEQ ID NO: 17 identifizierten Sequenzen stammt.

21. Glycoprotein nach Anspruch 12, **dadurch gekennzeichnet, daß** gemäß den Mutationen (c') mindestens eine Mutation an der Aminosäure 269 der C2-Region durchgeführt wird, wobei die Lage dieser zwischen einem Erstinfektions-Primärisolat und einem anderen konservierten Glycosylierungsstellen im Bereich von einer bis acht Aminosäuren schwanken kann.

22. Glycoprotein nach Anspruch 12, **dadurch gekennzeichnet, daß** gemäß den Mutationen (d') mindestens eine Mutation an der Aminosäure 294 der v3-Region durchgeführt wird, wobei die Lage dieser zwischen einem Erstinfektions-Primärisolat und einem anderen konservierten Glycosylierungsstellen im Bereich von einer bis acht Aminosäuren schwanken kann.

23. Pharmazeutische Zusammensetzung, die folgendes umfaßt:
(i) mindestens ein mutiertes Gen, das für ein mutiertes Hüll-Glycoprotein des HIV-1-Virus codiert, wobei das mutierte Gen aus der Reihe beliebige Gene, die in einem beliebigen der Ansprüche 1-11 beschrieben sind und unter der Kontrolle von Regulationssequenzen, die seine Expression in einer Wirtszelle gestattet, steht,
einen pharmazeutisch unbedenklichen Träger sowie
gegebenenfalls einen Zusatzstoff, der das Eindringen des mutierten Gens in die Zelle erleichtert und/oder der eine Zielsteuerung an dieser Zelle ermöglicht; oder
(ii) mindestens das in (i) identifizierte Gen, das in einen rekombinanten Virusvektor kloniert ist.

24. Pharmazeutische Zusammensetzung, die folgendes umfaßt:
- mindestens ein mutiertes Hüll-Glycoprotein des HIV-1-Virus, wobei dieses mutierte Glycoprotein aus der Reihe beliebige Glycoproteine, die in einem beliebigen der Ansprüche 12-22 beschrieben sind, stammt,
- einen pharmazeutisch unbedenklichen Grundstoff und/oder Träger und/oder Hilfsstoff und/oder ein pharmazeutisch unbedenkliches Verdünnungsmittel.

25. Verfahren zur Erzeugung von Antikörpern, bei dem man ein nichtmenschliches Säugetier, wie eine Maus oder Ratte oder einen Affen, mit mindestens einem mutierten Gen, das für ein mutiertes Hüll-Glycoprotein des HIV-1-Virus codiert, immunisiert, wobei das mutierte Gen aus der Reihe der beliebigen Gene, die in einem beliebigen der Ansprüche 1-11 beschrieben sind, und unter der Kontrolle von Regulationssequenzen, die seine Expression in vivo gestatten, steht.

26. Verfahren zur Erzeugung von Antikörpern, bei dem man ein nichtmenschliches Säugetier, wie eine Maus oder Ratte oder einen Affen mit mindestens einem mutierten Hüll-Glycoprotein des HIV-1-Virus immunisiert, wobei das mutierte Glycoprotein aus der Reihe der beliebigen Glycoproteine, die in einem beliebigen der Ansprüche 12-22 beschrieben sind, stammt.

27. Verfahren zur Auswertung eines Therapeutikums, bei dem man einem nichtmenschlichen Tier mindestens ein wie in einem beliebigen der Ansprüche 1-11 beschriebenes mutiertes Gen, das für ein mutiertes Hüll-Glycoprotein des HIV-1-Virus codiert, verabreicht und man
- einen Assay der spezifischen Antikörper des mutierten Glycoproteins durchführt, und/oder
- einen Assay der spezifischen neutralisierenden Antikörper des mutierten oder nativen Glycoproteins durchführt, und/oder
- einen Assay der gegen das mutierte Glycoprotein induzierten zellulären Immunantwort durchführt, zum Beispiel mittels eines In-vitro-Tests auf Aktivierung der für das mutierte Glycoprotein spezifischen "Helfer-"T-Lymphozyten.


### LISTE DE SEQUENCES

<110> bioMérieux
<120> GENE ENV du VIH-1 MUTE, GLYCOPROTEINE ENV MUTEE ET UTILISATIONS
<130> HIV1-m
<140>
   <141>
<150> FR0111699
   <151> 2001-09-06
<160> 26
<170> PatentIn Ver. 2.1
<210> 1
   <211> 2535
   <212> ADN
   <213> VIH-1 133
<400> 1
   atgagagtga aggagatcag gaggaattat cagcacttgt ggagatgggg cacgatgctc 60
   cttgggatgt tcatgatctg tagtgcagaa gaacaattgt gggtcacagt ctattatggg 120
   gtacctgtgt ggaaagaagc aactaccact ctattttgtg catcagatgc taaagcatat 180
   gatacagagg tacataatgt ctgggccaca catgcctgtg tacccacaga ccccaaccca 240
   cgagaagtag taatgggaaa tgtgacagaa gaatttaaca tatggaacaa tagcatggta 300
   gaacagatgc atgaagatat aattagttta tgggatcaaa gtctaaagcc atgtgtaaaa 360
   ttaaccccac tctgtgttac tttaaattgc actaactaca atagtactag taatgatact 420
   accaaggaaa caggaataaa aaaatgctct ttcaatatca ccacctcagg cgtaaaagat 480
   aggtttacga aggaaaatgc acttttgtat acagctgatg tagtaccaat agataatagt 540
   agtatcaact ataggttgat aggttgtaac acctcagtca ttacccaggc ctgtccaaag 600
   gtatcctttg agccaattcc catacattat tgtgccccgg ctggttttgc gattctaaag 660
   tgtaataatg aaacattcga tggaaaagga ccatgtacaa atgtcagcac agtacaatgt 720
   acacatggaa ttagaccagt agtatcaact caactgctgt taaatggcag tctagcagaa 780
   acagaggtag taattagatc tgacaatttc tcgaacaatg ctaaaaccat aatagtacaa 840
   ctgacagaac ctgtagtaat caattgtata agacccaaca acaacacaag aagaagtata 900
   catataggac cagggagcgc atttatacia acaggacaaa taataggaga tataagacaa 960
   gcacattgta acattaatgg aacacaatgg aataaaactt taagtctaat agttgataaa 1020
   ttaaagaaac aatttaataa taaaacaata atctttagga actcctcagg aggggaccca 1080
   gaaattgtaa tgcacagttt taactgtgga ggggaatttt tctactgtaa tacaacacaa 1140
   ctgtttaata gtatttggat gcttaataat acttgggatg gtactaaaga ggaaaatatt 1200
   acactcccat gcagaataaa acaaattata aacatgtggc aggaagtagg aaaagcaatg 1260
   tatgcccctc ccatcaaagg acaaattaga tgttcatcaa acattacagg gctgctatta 1320
   acaagagatg gtggtggtaa cacgagcagc gataatgaga ccttcagacc tggaggagga 1380
   gatatgaggg ataattggag aagtgaatta tataaatata aagtagtaaa aattgaacca 1440
   ttaggagtag cacccaccaa ggcaaggaga agagtggtgc agagagaaaa aagagcagtg 1500
   ggaatgctag gagccatgtt ccttgggttc ttgggagcag caggaagcac tatgggcgca 1560
   gcgtcactgg cgttgacggt acaggccaga caagtattgt ctggtatagt gcaacagcag 1620
   aacaatttgc tgagggctat tgaggcgcaa cagcacctgt tgcaactcac agtctggggc 1680
   atcaagcagc tccaggcaag agtcctggct gtggaaaggt acctagggga tcaacagctc 1740
   ctgggaattt ggggttgctc tggaaaactc atctgcacca ctgctgtgcc ttggaatgat 1800
   agttggagta ataaatctct gaaatatatt tgggataaca tgacctggat gcagtgggaa 1860
   aaagaaattg acacccacat aggcacaata taccaattaa ttgaagaatc gcagtaccaa 1920
   caagaaaaga atgaaaaaga attattggaa ctagataaat gggcaagttt gtggaattgg 1980
   tttgacataa caaactggct gtggtatata aaaatattca taatgatagt aggaggcttg 2040
   ataggtttaa gaatagtttt tactgtactt tctatagtga atagagttag gcagggatac 2100
   tcaccattat cgttggagac ccgcctccca acccagaggg gacccgacag gcccgaagga 2160
   atcgaagaag aaggtggaga gagagacaga gacacatccg gtcaattagt gaatggattc 2220
   ttagcaatta tctgggtcga cctgcggagc ctgtgcctct tcagctacca ccgcttgaga 2280
   gacttactct tgattgtaac gaggattgtg gaacttctgg gacgcagggg gtgggaagcc 2340
   ctcaagtatt tgtggaatct cctccagtat tggagtcagg aactaaagaa gagtgctgtt 2400
   agcttgttca atgccatagc catagcagta gctgagggga cagataggat aatagaagta 2460
   atacaaaggg cttatagggc cattctccac atacctagaa gaataagaca gggcttggaa 2520
   aggcttttgc tataa                                                  2535
<210> 2
   <211> 2535
   <212> ADN
   <213> VIH-1 mutant g12
<400> 2
   atgagagtga aggagatcag gaggaattat cagcacttgt ggagatgggg cacgatgctc 60
   cttgggatgt tcatgatctg tagtgcagaa gaacaattgt gggtcacagt ctattatggg 120
   gtacctgtgt ggaaagaagc aactaccact ctattttgtg catcagatgc taaagcatat 180
   gatacagagg tacataatgt ctgggccaca catgcctgtg tacccacaga ccccaaccca 240
   cgagaagtag taatgggaaa tgtgacagaa gaatttaaca tatggaacaa tagcatggta 300
   gaacagatgc atgaagatat aattagttta tgggatcaaa gtctaaagcc atgtgtaaaa 360
   ttaaccccac tctgtgttac tttaaattgc actaactaca atagtactag taatgatact 420
   accaaggaaa caggaataaa aaaatgctct ttcaatatca ccacctcagg cgtaaaagat 480
   aggtttacga aggaaaatgc acttttgtat acagctgatg tagtaccaat agataatagt 540
   agtatcaact ataggttgat aggttgtaac acctcagtca ttacccaggc ctgtccaaag 600
   gtatcctttg agccaattcc catacattat tgtgccccgg ctggttttgc gattctaaag 660
   tgtaataatg aaacattcga tggaaaagga ccatgtacaa atgtcagcac agtacaatgt 720
   acacatggaa ttagaccagt agtatcaact caactgctgt taaatggcag tctagcagaa 780
   acagaggtag taattagatc tgacaatttc tcgaacaatg ctaaaaccat aatagtacaa 840
   ctgacagaac ctgtagtaat caattgtata agacccaacc agaacacaag aagaagtata 900
   catataggac cagggagcgc attttataca acaggacaaa taataggaga tataagacaa 960
   gcacattgta acattcaggg aacacaatgg cagaaaactt taagtctaat agttgataaa 1020
   ttaaagaaac aatttaataa taaaacaata atctttagga actcctcagg aggggaccca 1080
   gaaattgtaa tgcacagttt taactgtgga ggggaatttt tctactgtaa tacaacacaa 1140
   ctgtttaata gtatttggat gcttaataat acttgggatg gtactaaaga ggaaaatatt 1200
   acactcccat gcagaataaa acaaattata aacatgtggc aggaagtagg aaaagcaatg 1260
   tatgcccctc ccatcaaagg acaaattaga tgttcatcaa acattacagg gctgctatta 1320
   acaagagatg gtggtggtaa cacgagcagc gataatgaga ccttcagacc tggaggagga 1380
   gatatgaggg ataattggag aagtgaatta tataaatata aagtagtaaa aattgaacca 1440
   ttaggagtag cacccaccaa ggcaaggaga agagtggtgc agagagaaaa aagagcagtg 1500
   ggaatgctag gagccatgtt ccttgggttc ttgggagcag caggaagcac tatgggcgca 1560
   gcgtcactgg cgttgacggt acaggccaga caagtattgt ctggtatagt gcaacagcag 1620
   aacaatttgc tgagggctat tgaggcgcaa cagcacctgt tgcaactcac agtctggggc 1680
   atcaagcagc tccaggcaag agtcctggct gtggaaaggt acctagggga tcaacagctc 1740
   ctgggaattt ggggttgctc tggaaaactc atctgcacca ctgctgtgcc ttggaatgat 1800
   agttggagta ataaatctct gaaatatatt tgggataaca tgacctggat gcagtgggaa 1860
   aaagaaattg acacccacat aggcacaata taccaattaa ttgaagaatc gcagtaccaa 1920
   caagaaaaga atgaaaaaga attattggaa ctagataaat gggcaagttt gtggaattgg 1980
   tttgacataa caaactggct gtggtatata aaaatattca taatgatagt aggaggcttg 2040
   ataggtttaa gaatagtttt tactgtactt tctatagtga atagagttag gcagggatac 2100
   tcaccattat cgttggagac ccgcctccca acccagaggg gacccgacag gcccgaagga 2160
   atcgaagaag aaggtggaga gagagacaga gacacatccg gtcaattagt gaatggattc 2220
   ttagcaatta tctgggtcga cctgcggagc ctgtgcctct tcagctacca ccgcttgaga 2280
   gacttactct tgattgtaac gaggattgtg gaacttctgg gacgcagggg gtgggaagcc 2340
   ctcaagtatt tgtggaatct cctccagtat tggagtcagg aactaaagaa gagtgctgtt 2400
   agcttgttca atgccatagc catagcagta gctgagggga cagataggat aatagaagta 2460
   atacaaaggg cttatagggc cattctccac atacctagaa gaataagaca gggcttggaa 2520
   aggcttttgc tataa                                                  2535
<210> 3
   <211> 1494
   <212> ADN
   <213> VIH-1 mutant g12
<400> 3
   atgagagtga aggagatcag gaggaattat cagcacttgt ggagatgggg cacgatgctc 60
   cttgggatgt tcatgatctg tagtgcagaa gaacaattgt gggtcacagt ctattatggg 120
   gtacctgtgt ggaaagaagc aactaccact ctattttgtg catcagatgc taaagcatat 180
   gatacagagg tacataatgt ctgggccaca catgcctgtg tacccacaga ccccaaccca 240
   cgagaagtag taatgggaaa tgtgacagaa gaatttaaca tatggaacaa tagcatggta 300
   gaacagatgc atgaagatat aattagttta tgggatcaaa gtctaaagcc atgtgtaaaa 360
   ttaaccccac tctgtgttac tttaaattgc actaactaca atagtactag taatgatact 420
   accaaggaaa caggaataaa aaaatgctct ttcaatatca ccacctcagg cgtaaaagat 480
   aggtttacga aggaaaatgc acttttgtat acagctgatg tagtaccaat agataatagt 540
   agtatcaact ataggttgat aggttgtaac acctcagtca ttacccaggc ctgtccaaag 600
   gtatcctttg agccaattcc catacattat tgtgccccgg ctggttttgc gattctaaag 660
   tgtaataatg aaacattcga tggaaaagga ccatgtacaa atgtcagcac agtacaatgt 720
   acacatggaa ttagaccagt agtatcaact caactgctgt taaatggcag tctagcagaa 780
   acagaggtag taattagatc tgacaatttc tcgaacaatg ctaaaaccat aatagtacaa 840
   ctgacagaac ctgtagtaat caattgtata agacccaacc agaacacaag aagaagtata 900
   catataggac cagggagcgc attttataca acaggacaaa taataggaga tataagacaa 960
   gcacattgta acattcaggg aacacaatgg cagaaaactt taagtctaat agttgataaa 1020
   ttaaagaaac aatttaataa taaaacaata atctttagga actcctcagg aggggaccca 1080
   gaaattgtaa tgcacagttt taactgtgga ggggaatttt tctactgtaa tacaacacaa 1140
   ctgtttaata gtatttggat gcttaataat acttgggatg gtactaaaga ggaaaatatt 1200
   acactcccat gcagaataaa acaaattata aacatgtggc aggaagtagg aaaagcaatg 1260
   tatgcccctc ccatcaaagg acaaattaga tgttcatcaa acattacagg gctgctatta 1320
   acaagagatg gtggtggtaa cacgagcagc gataatgaga ccttcagacc tggaggagga 1380
   gatatgaggg ataattggag aagtgaatta tataaatata aagtagtaaa aattgaacca 1440
   ttaggagtag cacccaccaa ggcaaggaga agagtggtgc agagagaaaa aaga       1494
<210> 4
   <211> 2007
   <212> ADN
   <213> VIH-1 mutant g12
<400> 4
   atgagagtga aggagatcag gaggaattat cagcacttgt ggagatgggg cacgatgctc 60
   cttgggatgt tcatgatctg tagtgcagaa gaacaattgt gggtcacagt ctattatggg 120
   gtacctgtgt ggaaagaagc aactaccact ctattttgtg catcagatgc taaagcatat 180
   gatacagagg tacataatgt ctgggccaca catgcctgtg tacccacaga ccccaaccca 240
   cgagaagtag taatgggaaa tgtgacagaa gaatttaaca tatggaacaa tagcatggta 300
   gaacagatgc atgaagatat aattagttta tgggatcaaa gtctaaagcc atgtgtaaaa 360
   ttaaccccac tctgtgttac tttaaattgc actaactaca atagtactag taatgatact 420
   accaaggaaa caggaataaa aaaatgctct ttcaatatca ccacctcagg cgtaaaagat 480
   aggtttacga aggaaaatgc acttttgtat acagctgatg tagtaccaat agataatagt 540
   agtatcaact ataggttgat aggttgtaac acctcagtca ttacccaggc ctgtccaaag 600
   gtatcctttg agccaattcc catacattat tgtgccccgg ctggttttgc gattctaaag 660
   tgtaataatg aaacattcga tggaaaagga ccatgtacaa atgtcagcac agtacaatgt 720
   acacatggaa ttagaccagt agtatcaact caactgctgt taaatggcag tctagcagaa 780
   acagaggtag taattagatc tgacaatttc tcgaacaatg ctaaaaccat aatagtacaa 840
   ctgacagaac ctgtagtaat caattgtata agacccaacc agaacacaag aagaagtata 900
   catataggac cagggagcgc attttataca acaggacaaa taataggaga tataagacaa 960
   gcacattgta acattcaggg aacacaatgg cagaaaactt taagtctaat agttgataaa 1020
   ttaaagaaac aatttaataa taaaacaata atctttagga actcctcagg aggggaccca 1080
   gaaattgtaa tgcacagttt taactgtgga ggggaatttt tctactgtaa tacaacacaa 1140
   ctgtttaata gtatttggat gcttaataat acttgggatg gtactaaaga ggaaaatatt 1200
   acactcccat gcagaataaa acaaattata aacatgtggc aggaagtagg aaaagcaatg 1260
   tatgcccctc ccatcaaagg acaaattaga tgttcatcaa acattacagg gctgctatta 1320
   acaagagatg gtggtggtaa cacgagcagc gataatgaga ccttcagacc tggaggagga 1380
   gatatgaggg ataattggag aagtgaatta tataaatata aagtagtaaa aattgaacca 1440
   ttaggagtag cacccaccaa ggcaaggaga agagtggtgc agagagaaaa aagagcagtg 1500
   ggaatgctag gagccatgtt ccttgggttc ttgggagcag caggaagcac tatgggcgca 1560
   gcgtcactgg cgttgacggt acaggccaga caagtattgt ctggtatagt gcaacagcag 1620
   aacaatttgc tgagggctat tgaggcgcaa cagcacctgt tgcaactcac agtctggggc 1680
   atcaagcagc tccaggcaag agtcctggct gtggaaaggt acctagggga tcaacagctc 1740
   ctgggaattt ggggttgctc tggaaaactc atctgcacca ctgctgtgcc ttggaatgat 1800
   agttggagta ataaatctct gaaatatatt tgggataaca tgacctggat gcagtgggaa 1860
   aaagaaattg acacccacat aggcacaata taccaattaa ttgaagaatc gcagtaccaa 1920
   caagaaaaga atgaaaaaga attattggaa ctagataaat gggcaagttt gtggaattgg 1980
   tttgacataa caaactggct gtggtat                                      2007
<210> 5
   <211> 2535
   <212> ADN
   <213> VIH-1 mutant g112
<400> 5
   atgagagtga aggagatcag gaggaattat cagcacttgt ggagatgggg cacgatgctc 60
   cttgggatgt tcatgatctg tagtgcagaa gaacaattgt gggtcacagt ctattatggg 120
   gtacctgtgt ggaaagaagc aactaccact ctattttgtg catcagatgc taaagcatat 180
   gatacagagg tacataatgt ctgggccaca catgcctgtg tacccacaga ccccaaccca 240
   cgagaagtag taatgggaaa tgtgacagaa gaatttaaca tatggaacaa tagcatggta 300
   gaacagatgc atgaagatat aattagttta tgggatcaaa gtctaaagcc atgtgtaaaa 360
   ttaaccccac tctgtgttac tttaaattgc actaactaca atagtactag taatgatact 420
   accaaggaaa caggaataaa aaaatgctct ttcaatatca ccacctcagg cgtaaaagat 480
   aggtttacga aggaaaatgc acttttgtat acagctgatg tagtaccaat agataatagt 540
   agtatcaact ataggttgat aggttgtaac acctcagtca ttacccaggc ctgtccaaag 600
   gtatcctttg agccaattcc catacattat tgtgccccgg ctggttttgc gattctaaag 660
   tgtaataatg aaacattcga tggaaaagga ccatgtacaa atgtcagcac agtacaatgt 720
   acacatggaa ttagaccagt agtatcaact caactgctgt taaatggcag tctagcagaa 780
   acagaggtag taattagatc tgacaatttc tcgaacaatg ctaaaaccat aatagtacaa 840
   ctgacagaac ctgtagtaat caattgtata agacccaacc agaacacaag aagaagtata 900
   catataggac cagggagcgc attttataca acaggacaaa taataggaga tataagacaa 960
   gcacattgta acattcaggg aacacaatgg cagaaaactt taagtctaat agttgataaa 1020
   ttaaagaaac aatttaatca gaaaacaata atctttaggc agtcctcagg aggggaccca 1080
   gaaattgtaa tgcacagttt taactgtgga ggggaatttt tctactgtaa tacaacacaa 1140
   ctgtttaata gtatttggat gcttaataat acttgggatg gtactaaaga ggaaaatatt 1200
   acactcccat gcagaataaa acaaattata aacatgtggc aggaagtagg aaaagcaatg 1260
   tatgcccctc ccatcaaagg acaaattaga tgttcatcaa acattacagg gctgctatta 1320
   acaagagatg gtggtggtaa cacgagcagc gataatgaga ccttcagacc tggaggagga 1380
   gatatgaggg ataattggag aagtgaatta tataaatata aagtagtaaa aattgaacca 1440
   ttaggagtag cacccaccaa ggcaaggaga agagtggtgc agagagaaaa aagagcagtg 1500
   ggaatgctag gagccatgtt ccttgggttc ttgggagcag caggaagcac tatgggcgca 1560
   gcgtcactgg cgttgacggt acaggccaga caagtattgt ctggtatagt gcaacagcag 1620
   aacaatttgc tgagggctat tgaggcgcaa cagcacctgt tgcaactcac agtctggggc 1680
   atcaagcagc tccaggcaag agtcctggct gtggaaaggt acctagggga tcaacagctc 1740
   ctgggaattt ggggttgctc tggaaaactc atctgcacca ctgctgtgcc ttggaatgat 1800
   agttggagta ataaatctct gaaatatatt tgggataaca tgacctggat gcagtgggaa 1860
   aaagaaattg acacccacat aggcacaata taccaattaa ttgaagaatc gcagtaccaa 1920
   caagaaaaga atgaaaaaga attattggaa ctagataaat gggcaagttt gtggaattgg 1980
   tttgacataa caaactggct gtggtatata aaaatattca taatgatagt aggaggcttg 2040
   ataggtttaa gaatagtttt tactgtactt tctatagtga atagagttag gcagggatac 2100
   tcaccattat cgttggagac ccgcctccca acccagaggg gacccgacag gcccgaagga 2160
   atcgaagaag aaggtggaga gagagacaga gacacatccg gtcaattagt gaatggattc 2220
   ttagcaatta tctgggtcga cctgcggagc ctgtgcctct tcagctacca ccgcttgaga 2280
   gacttactct tgattgtaac gaggattgtg gaacttctgg gacgcagggg gtgggaagcc 2340
   ctcaagtatt tgtggaatct cctccagtat tggagtcagg aactaaagaa gagtgctgtt 2400
   agcttgttca atgccatagc catagcagta gctgagggga cagataggat aatagaagta 2460
   atacaaaggg cttatagggc cattctccac atacctagaa gaataagaca gggcttggaa 2520
   aggcttttgc tataa                                                  2535
<210> 6
   <211> 1494
   <212> ADN
   <213> VIH-1 mutant g112
<400> 6
   atgagagtga aggagatcag gaggaattat cagcacttgt ggagatgggg cacgatgctc 60
   cttgggatgt tcatgatctg tagtgcagaa gaacaattgt gggtcacagt ctattatggg 120
   gtacctgtgt ggaaagaagc aactaccact ctattttgtg catcagatgc taaagcatat 180
   gatacagagg tacataatgt ctgggccaca catgcctgtg tacccacaga ccccaaccca 240
   cgagaagtag taatgggaaa tgtgacagaa gaatttaaca tatggaacaa tagcatggta 300
   gaacagatgc atgaagatat aattagttta tgggatcaaa gtctaaagcc atgtgtaaaa 360
   ttaaccccac tctgtgttac tttaaattgc actaactaca atagtactag taatgatact 420
   accaaggaaa caggaataaa aaaatgctct ttcaatatca ccacctcagg cgtaaaagat 480
   aggtttacga aggaaaatgc acttttgtat acagctgatg tagtaccaat agataatagt 540
   agtatcaact ataggttgat aggttgtaac acctcagtca ttacccaggc ctgtccaaag 600
   gtatcctttg agccaattcc catacattat tgtgccccgg ctggttttgc gattctaaag 660
   tgtaataatg aaacattcga tggaaaagga ccatgtacaa atgtcagcac agtacaatgt 720
   acacatggaa ttagaccagt agtatcaact caactgctgt taaatggcag tctagcagaa 780
   acagaggtag taattagatc tgacaatttc tcgaacaatg ctaaaaccat aatagtacaa 840
   ctgacagaac ctgtagtaat caattgtata agacccaacc agaacacaag aagaagtata 900
   catataggac cagggagcgc attttataca acaggacaaa taataggaga tataagacaa 960
   gcacattgta acattcaggg aacacaatgg cagaaaactt taagtctaat agttgataaa 1020
   ttaaagaaac aatttaatca gaaaacaata atctttaggc agtcctcagg aggggaccca 1080
   gaaattgtaa tgcacagttt taactgtgga ggggaatttt tctactgtaa tacaacacaa 1140
   ctgtttaata gtatttggat gcttaataat acttgggatg gtactaaaga ggaaaatatt 1200
   acactcccat gcagaataaa acaaattata aacatgtggc aggaagtagg aaaagcaatg 1260
   tatgcccctc ccatcaaagg acaaattaga tgttcatcaa acattacagg gctgctatta 1320
   acaagagatg gtggtggtaa cacgagcagc gataatgaga ccttcagacc tggaggagga 1380
   gatatgaggg ataattggag aagtgaatta tataaatata aagtagtaaa aattgaacca 1440
   ttaggagtag cacccaccaa ggcaaggaga agagtggtgc agagagaaaa aaga       1494
<210> 7
   <211> 2007
   <212> ADN
   <213> VIH-1 mutant g112
<400> 7
   atgagagtga aggagatcag gaggaattat cagcacttgt ggagatgggg cacgatgctc 60
   cttgggatgt tcatgatctg tagtgcagaa gaacaattgt gggtcacagt ctattatggg 120
   gtacctgtgt ggaaagaagc aactaccact ctattttgtg catcagatgc taaagcatat 180
   gatacagagg tacataatgt ctgggccaca catgcctgtg tacccacaga ccccaaccca 240
   cgagaagtag taatgggaaa tgtgacagaa gaatttaaca tatggaacaa tagcatggta 300
   gaacagatgc atgaagatat aattagttta tgggatcaaa gtctaaagcc atgtgtaaaa 360
   ttaaccccac tctgtgttac tttaaattgc actaactaca atagtactag taatgatact 420
   accaaggaaa caggaataaa aaaatgctct ttcaatatca ccacctcagg cgtaaaagat 480
   aggtttacga aggaaaatgc acttttgtat acagctgatg tagtaccaat agataatagt 540
   agtatcaact ataggttgat aggttgtaac acctcagtca ttacccaggc ctgtccaaag 600
   gtatcctttg agccaattcc catacattat tgtgccccgg ctggttttgc gattctaaag 660
   tgtaataatg aaacattcga tggaaaagga ccatgtacaa atgtcagcac agtacaatgt 720
   acacatggaa ttagaccagt agtatcaact caactgctgt taaatggcag tctagcagaa 780
   acagaggtag taattagatc tgacaatttc tcgaacaatg ctaaaaccat aatagtacaa 840
   ctgacagaac ctgtagtaat caattgtata agacccaacc agaacacaag aagaagtata 900
   catataggac cagggagcgc attttataca acaggacaaa taataggaga tataagacaa 960
   gcacattgta acattcaggg aacacaatgg cagaaaactt taagtctaat agttgataaa 1020
   ttaaagaaac aatttaatca gaaaacaata atctttaggc agtcctcagg aggggaccca 1080
   gaaattgtaa tgcacagttt taactgtgga ggggaatttt tctactgtaa tacaacacaa 1140
   ctgtttaata gtatttggat gcttaataat acttgggatg gtactaaaga ggaaaatatt 1200
   acactcccat gcagaataaa acaaattata aacatgtggc aggaagtagg aaaagcaatg 1260
   tatgcccctc ccatcaaagg acaaattaga tgttcatcaa acattacagg gctgctatta 1320
   acaagagatg gtggtggtaa cacgagcagc gataatgaga ccttcagacc tggaggagga 1380
   gatatgaggg ataattggag aagtgaatta tataaatata aagtagtaaa aattgaacca 1440
   ttaggagtag cacccaccaa ggcaaggaga agagtggtgc agagagaaaa aagagcagtg 1500
   ggaatgctag gagccatgtt ccttgggttc ttgggagcag caggaagcac tatgggcgca 1560
   gcgtcactgg cgttgacggt acaggccaga caagtattgt ctggtatagt gcaacagcag 1620
   aacaatttgc tgagggctat tgaggcgcaa cagcacctgt tgcaactcac agtctggggc 1680
   atcaagcagc tccaggcaag agtcctggct gtggaaaggt acctagggga tcaacagctc 1740
   ctgggaattt ggggttgctc tggaaaactc atctgcacca ctgctgtgcc ttggaatgat 1800
   agttggagta ataaatctct gaaatatatt tgggataaca tgacctggat gcagtgggaa 1860
   aaagaaattg acacccacat aggcacaata taccaattaa ttgaagaatc gcagtaccaa 1920
   caagaaaaga atgaaaaaga attattggaa ctagataaat gggcaagttt gtggaattgg 1980
   tttgacataa caaactggct gtggtat                                     2007
<210> 8
   <211> 2535
   <212> ADN
   <213> VIH-1 mutant g14
<400> 8
   atgagagtga aggagatcag gaggaattat cagcacttgt ggagatgggg cacgatgctc 60
   cttgggatgt tcatgatctg tagtgcagaa gaacaattgt gggtcacagt ctattatggg 120
   gtacctgtgt ggaaagaagc aactaccact ctattttgtg catcagatgc taaagcatat 180
   gatacagagg tacataatgt ctgggccaca catgcctgtg tacccacaga ccccaaccca 240
   cgagaagtag taatgggaaa tgtgacagaa gaatttaaca tatggaacaa tagcatggta 300
   gaacagatgc atgaagatat aattagttta tgggatcaaa gtctaaagcc atgtgtaaaa 360
   ttaaccccac tctgtgttac tttaaattgc actaactaca atagtactag taatgatact 420
   accaaggaaa caggaataaa aaaatgctct ttcaatatca ccacctcagg cgtaaaagat 480
   aggtttacga aggaaaatgc acttttgtat acagctgatg tagtaccaat agataatagt 540
   agtatcaact ataggttgat aggttgtaac acctcagtca ttacccaggc ctgtccaaag 600
   gtatcctttg agccaattcc catacattat tgtgccccgg ctggttttgc gattctaaag 660
   tgtaataatg aaacattcga tggaaaagga ccatgtacaa atgtcagcac agtacaatgt 720
   acacatggaa ttagaccagt agtatcaact caactgctgt taaatggcag tctagcagaa 780
   acagaggtag taattagatc tgaccagttc tcgaacaatg ctaaaaccat aatagtacaa 840
   ctgacagaac ctgtagtaat caattgtata agacccaacc agaacacaag aagaagtata 900
   catataggac cagggagcgc attttataca acaggacaaa taataggaga tataagacaa 960
   gcacattgta acattaatgg aacacaatgg aataaaactt taagtctaat agttgataaa 1020
   ttaaagaaac aatttaataa taaaacaata atctttagga actcctcagg aggggaccca 1080
   gaaattgtaa tgcacagttt taactgtgga ggggaatttt tctactgtaa tacaacacaa 1140
   ctgtttaata gtatttggat gcttaataat acttgggatg gtactaaaga ggaaaatatt 1200
   acactcccat gcagaataaa acaaattata aacatgtggc aggaagtagg aaaagcaatg 1260
   tatgcccctc ccatcaaagg acaaattaga tgttcatcaa acattacagg gctgctatta 1320
   acaagagatg gtggtggtaa cacgagcagc gataatgaga ccttcagacc tggaggagga 1380
   gatatgaggg ataattggag aagtgaatta tataaatata aagtagtaaa aattgaacca 1440
   ttaggagtag cacccaccaa ggcaaggaga agagtggtgc agagagaaaa aagagcagtg 1500
   ggaatgctag gagccatgtt ccttgggttc ttgggagcag caggaagcac tatgggcgca 1560
   gcgtcactgg cgttgacggt acaggccaga caagtattgt ctggtatagt gcaacagcag 1620
   aacaatttgc tgagggctat tgaggcgcaa cagcacctgt tgcaactcac agtctggggc 1680
   atcaagcagc tccaggcaag agtcctggct gtggaaaggt acctagggga tcaacagctc 1740
   ctgggaattt ggggttgctc tggaaaactc atctgcacca ctgctgtgcc ttggaatgat 1800
   agttggagta ataaatctct gaaatatatt tgggataaca tgacctggat gcagtgggaa 1860
   aaagaaattg acacccacat aggcacaata taccaattaa ttgaagaatc gcagtaccaa 1920
   caagaaaaga atgaaaaaga attattggaa ctagataaat gggcaagttt gtggaattgg 1980
   tttgacataa caaactggct gtggtatata aaaatattca taatgatagt aggaggcttg 2040
   ataggtttaa gaatagtttt tactgtactt tctatagtga atagagttag gcagggatac 2100
   tcaccattat cgttggagac ccgcctccca acccagaggg gacccgacag gcccgaagga 2160
   atcgaagaag aaggtggaga gagagacaga gacacatccg gtcaattagt gaatggattc 2220
   ttagcaatta tctgggtcga cctgcggagc ctgtgcctct tcagctacca ccgcttgaga 2280
   gacttactct tgattgtaac gaggattgtg gaacttctgg gacgcagggg gtgggaagcc 2340
   ctcaagtatt tgtggaatct cctccagtat tggagtcagg aactaaagaa gagtgctgtt 2400
   agcttgttca atgccatagc catagcagta gctgagggga cagataggat aatagaagta 2460
   atacaaaggg cttatagggc cattctccac atacctagaa gaataagaca gggcttggaa 2520
   aggcttttgc tataa 2535
<210> 9
   <211> 1494
   <212> ADN
   <213> VIH-1 mutant g14
<400> 9
   atgagagtga aggagatcag gaggaattat cagcacttgt ggagatgggg cacgatgctc 60
   cttgggatgt tcatgatctg tagtgcagaa gaacaattgt gggtcacagt ctattatggg 120
   gtacctgtgt ggaaagaagc aactaccact ctattttgtg catcagatgc taaagcatat 180
   gatacagagg tacataatgt ctgggccaca catgcctgtg tacccacaga ccccaaccca 240
   cgagaagtag taatgggaaa tgtgacagaa gaatttaaca tatggaacaa tagcatggta 300
   gaacagatgc atgaagatat aattagttta tgggatcaaa gtctaaagcc atgtgtaaaa 360
   ttaaccccac tctgtgttac tttaaattgc actaactaca atagtactag taatgatact 420
   accaaggaaa caggaataaa aaaatgctct ttcaatatca ccacctcagg cgtaaaagat 480
   aggtttacga aggaaaatgc acttttgtat acagctgatg tagtaccaat agataatagt 540
   agtatcaact ataggttgat aggttgtaac acctcagtca ttacccaggc ctgtccaaag 600
   gtatcctttg agccaattcc catacattat tgtgccccgg ctggttttgc gattctaaag 660
   tgtaataatg aaacattcga tggaaaagga ccatgtacaa atgtcagcac agtacaatgt 720
   acacatggaa ttagaccagt agtatcaact caactgctgt taaatggcag tctagcagaa 780
   acagaggtag taattagatc tgaccagttc tcgaacaatg ctaaaaccat aatagtacaa 840
   ctgacagaac ctgtagtaat caattgtata agacccaacc agaacacaag aagaagtata 900
   catataggac cagggagcgc attttataca acaggacaaa taataggaga tataagacaa 960
   gcacattgta acattaatgg aacacaatgg aataaaactt taagtctaat agttgataaa 1020
   ttaaagaaac aatttaataa taaaacaata atctttagga actcctcagg aggggaccca 1080
   gaaattgtaa tgcacagttt taactgtgga ggggaatttt tctactgtaa tacaacacaa 1140
   ctgtttaata gtatttggat gcttaataat acttgggatg gtactaaaga ggaaaatatt 1200
   acactcccat gcagaataaa acaaattata aacatgtggc aggaagtagg aaaagcaatg 1260
   tatgcccctc ccatcaaagg acaaattaga tgttcatcaa acattacagg gctgctatta 1320
   acaagagatg gtggtggtaa cacgagcagc gataatgaga ccttcagacc tggaggagga 1380
   gatatgaggg ataattggag aagtgaatta tataaatata aagtagtaaa aattgaacca 1440
   ttaggagtag cacccaccaa ggcaaggaga agagtggtgc agagagaaaa aaga       1494
<210> 10
   <211> 2007
   <212> ADN
   <213> VIH-1 mutant g14
<400> 10
   atgagagtga aggagatcag gaggaattat cagcacttgt ggagatgggg cacgatgctc 60
   cttgggatgt tcatgatctg tagtgcagaa gaacaattgt gggtcacagt ctattatggg 120
   gtacctgtgt ggaaagaagc aactaccact ctattttgtg catcagatgc taaagcatat 180
   gatacagagg tacataatgt ctgggccaca catgcctgtg tacccacaga ccccaaccca 240
   cgagaagtag taatgggaaa tgtgacagaa gaatttaaca tatggaacaa tagcatggta 300
   gaacagatgc atgaagatat aattagttta tgggatcaaa gtctaaagcc atgtgtaaaa 360
   ttaaccccac tctgtgttac tttaaattgc actaactaca atagtactag taatgatact 420
   accaaggaaa caggaataaa aaaatgctct ttcaatatca ccacctcagg cgtaaaagat 480
   aggtttacga aggaaaatgc acttttgtat acagctgatg tagtaccaat agataatagt 540
   agtatcaact ataggttgat aggttgtaac acctcagtca ttacccaggc ctgtccaaag 600
   gtatcctttg agccaattcc catacattat tgtgccccgg ctggttttgc gattctaaag 660
   tgtaataatg aaacattcga tggaaaagga ccatgtacaa atgtcagcac agtacaatgt 720
   acacatggaa ttagaccagt agtatcaact caactgctgt taaatggcag tctagcagaa 780
   acagaggtag taattagatc tgaccagttc tcgaacaatg ctaaaaccat aatagtacaa 840
   ctgacagaac ctgtagtaat caattgtata agacccaacc agaacacaag aagaagtata 900
   catataggac cagggagcgc attttataca acaggacaaa taataggaga tataagacaa 960
   gcacattgta acattaatgg aacacaatgg aataaaactt taagtctaat agttgataaa 1020
   ttaaagaaac aatttaataa taaaacaata atctttagga actcctcagg aggggaccca 1080
   gaaattgtaa tgcacagttt taactgtgga ggggaatttt tctactgtaa tacaacacaa 1140
   ctgtttaata gtatttggat gcttaataat acttgggatg gtactaaaga ggaaaatatt 1200
   acactcccat gcagaataaa acaaattata aacatgtggc aggaagtagg aaaagcaatg 1260
   tatgcccctc ccatcaaagg acaaattaga tgttcatcaa acattacagg gctgctatta 1320
   acaagagatg gtggtggtaa cacgagcagc gataatgaga ccttcagacc tggaggagga 1380
   gatatgaggg ataattggag aagtgaatta tataaatata aagtagtaaa aattgaacca 1440
   ttaggagtag cacccaccaa ggcaaggaga agagtggtgc agagagaaaa aagagcagtg 1500
   ggaatgctag gagccatgtt ccttgggttc ttgggagcag caggaagcac tatgggcgca 1560
   gcgtcactgg cgttgacggt acaggccaga caagtattgt ctggtatagt gcaacagcag 1620
   aacaatttgc tgagggctat tgaggcgcaa cagcacctgt tgcaactcac agtctggggc 1680
   atcaagcagc tccaggcaag agtcctggct gtggaaaggt acctagggga tcaacagctc 1740
   ctgggaattt ggggttgctc tggaaaactc atctgcacca ctgctgtgcc ttggaatgat 1800
   agttggagta ataaatctct gaaatatatt tgggataaca tgacctggat gcagtgggaa 1860
   aaagaaattg acacccacat aggcacaata taccaattaa ttgaagaatc gcagtaccaa 1920
   caagaaaaga atgaaaaaga attattggaa ctagataaat gggcaagttt gtggaattgg 1980
   tttgacataa caaactggct gtggtat                                     2007
<210> 11
   <211> 844
   <212> PRT
   <213> VIH-1 133
<400> 11
   Met Arg Val Lys Glu Ile Arg Arg Asn Tyr Gln His Leu Trp Arg Trp
    1               5                  10                  15
   Gly Thr Met Leu Leu Gly Met Phe Met Ile Cys Ser Ala Glu Glu Gln
               20                  25                  30
   Leu Trp Val Thr Val Tyr Tyr Gly Val Pro Val Trp Lys Glu Ala Thr
           35                  40                  45
   Thr Thr Leu Phe Cys Ala Ser Asp Ala Lys Ala Tyr Asp Thr Glu Val
       50                  55                  60
   His Asn Val Trp Ala Thr His Ala Cys Val Pro Thr Asp Pro Asn Pro
   65                  70                  75                  80
   Arg Glu Val Val Met Gly Asn Val Thr Glu Glu Phe Asn Ile Trp Asn
                   85                  90                  95
   Asn Ser Met Val Glu Gln Met His Glu Asp Ile Ile Ser Leu Trp Asp
               100                 105                 110
   Gln Ser Leu Lys Pro Cys Val Lys Leu Thr Pro Leu Cys Val Thr Leu
           115                 120                 125
   Asn Cys Thr Asn Tyr Asn Ser Thr Ser Asn Asp Thr Thr Lys Glu Thr
       130                 135                 140
   Gly Ile Lys Lys Cys Ser Phe Asn Ile Thr Thr Ser Gly Val Lys Asp
   145 150 155 160
   Arg Phe Thr Lys Glu Asn Ala Leu Leu Tyr Thr Ala Asp Val Val Pro
   165 170 175
   Ile Asp Asn Ser Ser Ile Asn Tyr Arg Leu Ile Gly Cys Asn Thr Ser
   180 185 190
   Val Ile Thr Gln Ala Cys Pro Lys Val Ser Phe Glu Pro Ile Pro Ile
   195 200 205
   His Tyr Cys Ala Pro Ala Gly Phe Ala Ile Leu Lys Cys Asn Asn Glu
   210 215 220
   Thr Phe Asp Gly Lys Gly Pro Cys Thr Asn Val Ser Thr Val Gln Cys
   225 230 235 240
   Thr His Gly Ile Arg Pro Val Val Ser Thr Gln Leu Leu Leu Asn Gly
   245 250 255
   Ser Leu Ala Glu Thr Glu Val Val Ile Arg Ser Asp Asn Phe Ser Asn
   260 265 270
   Asn Ala Lys Thr Ile Ile Val Gln Leu Thr Glu Pro Val Val Ile Asn
   275 280 285
   Cys Ile Arg Pro Asn Asn Asn Thr Arg Arg Ser Ile His Ile Gly Pro
   290 295 300
   Gly Ser Ala Phe Tyr Thr Thr Gly Gln Ile Ile Gly Asp Ile Arg Gln
   305 310 315 320
   Ala His Cys Asn Ile Asn Gly Thr Gln Trp Asn Lys Thr Leu Ser Leu
   325 330 335
   Ile Val Asp Lys Leu Lys Lys Gln Phe Asn Asn Lys Thr Ile Ile Phe
   340 345 350
   Arg Asn Ser Ser Gly Gly Asp Pro Glu Ile Val Met His Ser Phe Asn
   355 360 365
   Cys Gly Gly Glu Phe Phe Tyr Cys Asn Thr Thr Gln Leu Phe Asn Ser
   370 375 380
   Ile Trp Met Leu Asn Asn Thr Trp Asp Gly Thr Lys Glu Glu Asn Ile
   385 390 395 400
   Thr Leu Pro Cys Arg Ile Lys Gln Ile Ile Asn Met Trp Gln Glu Val
                   405                 410                 415
   Gly Lys Ala Met Tyr Ala Pro Pro Ile Lys Gly Gln Ile Arg Cys Ser
               420                 425                 430
   Ser Asn Ile Thr Gly Leu Leu Leu Thr Arg Asp Gly Gly Gly Asn Thr
           435                 440                 445
   Ser Ser Asp Asn Glu Thr Phe Arg Pro Gly Gly Gly Asp Met Arg Asp
       450                 455                 460
   Asn Trp Arg Ser Glu Leu Tyr Lys Tyr Lys Val Val Lys Ile Glu Pro
   465                 470                 475                 480
   Leu Gly Val Ala Pro Thr Lys Ala Arg Arg Arg Val Val Gln Arg Glu
                   485                 490                 495
   Lys Arg Ala Val Gly Met Leu Gly Ala Met Phe Leu Gly Phe Leu Gly
               500                 505                 510
   Ala Ala Gly Ser Thr Met Gly Ala Ala Ser Leu Ala Leu Thr Val Gln
           515                 520                 525
   Ala Arg Gln Val Leu Ser Gly Ile Val Gln Gln Gln Asn Asn Leu Leu
       530                 535                 540
   Arg Ala Ile Glu Ala Gln Gln His Leu Leu Gln Leu Thr Val Trp Gly
   545                 550                 555                 560
   Ile Lys Gln Leu Gln Ala Arg Val Leu Ala Val Glu Arg Tyr Leu Gly
                   565                 570                 575
   Asp Gln Gln Leu Leu Gly Ile Trp Gly Cys Ser Gly Lys Leu Ile Cys
               580                 585                 590
   Thr Thr Ala Val Pro Trp Asn Asp Ser Trp Ser Asn Lys Ser Leu Lys
           595                 600                 605
   Tyr Ile Trp Asp Asn Met Thr Trp Met Gln Trp Glu Lys Glu Ile Asp
       610                 615                 620
   Thr His Ile Gly Thr Ile Tyr Gln Leu Ile Glu Glu Ser Gln Tyr Gln
   625                 630                 635                 640
   Gln Glu Lys Asn Glu Lys Glu Leu Leu Glu Leu Asp Lys Trp Ala Ser
                   645                 650                 655
   Leu Trp Asn Trp Phe Asp Ile Thr Asn Trp Leu Trp Tyr Ile Lys Ile
               660                 665                 670
   Phe Ile Met Ile Val Gly Gly Leu Ile Gly Leu Arg Ile Val Phe Thr
           675                 680                 685
   Val Leu Ser Ile Val Asn Arg Val Arg Gln Gly Tyr Ser Pro Leu Ser
       690                 695                 700
   Leu Glu Thr Arg Leu Pro Thr Gln Arg Gly Pro Asp Arg Pro Glu Gly
   705                 710                 715                 720
   Ile Glu Glu Glu Gly Gly Glu Arg Asp Arg Asp Thr Ser Gly Gln Leu
                   725                 730                 735
   Val Asn Gly Phe Leu Ala Ile Ile Trp Val Asp Leu Arg Ser Leu Cys
               740                 745                 750
   Leu Phe Ser Tyr His Arg Leu Arg Asp Leu Leu Leu Ile Val Thr Arg
           755                 760                 765
   Ile Val Glu Leu Leu Gly Arg Arg Gly Trp Glu Ala Leu Lys Tyr Leu
       770                 775                 780
   Trp Asn Leu Leu Gln Tyr Trp Ser Gln Glu Leu Lys Lys Ser Ala Val
   785                 790                 795                 800
   Ser Leu Phe Asn Ala Ile Ala Ile Ala Val Ala Glu Gly Thr Asp Arg
                   805                 810                 815
   Ile Ile Glu Val Ile Gln Arg Ala Tyr Arg Ala Ile Leu His Ile Pro
               820                 825                 830
   Arg Arg Ile Arg Gln Gly Leu Glu Arg Leu Leu Leu
           835                 840
<210> 12
   <211> 844
   <212> PRT
   <213> VIH-1 mutant g12
<400> 12
   Met Arg Val Lys Glu Ile Arg Arg Asn Tyr Gln His Leu Trp Arg Trp
   1 5 10 15
   Gly Thr Met Leu Leu Gly Met Phe Met Ile Cys Ser Ala Glu Glu Gln
   20 25 30
   Leu Trp Val Thr Val Tyr Tyr Gly Val Pro Val Trp Lys Glu Ala Thr
   35 40 45
   Thr Thr Leu Phe Cys Ala Ser Asp Ala Lys Ala Tyr Asp Thr Glu Val
   50 55 60
   His Asn Val Trp Ala Thr His Ala Cys Val Pro Thr Asp Pro Asn Pro
   65 70 75 80
   Arg Glu Val Val Met Gly Asn Val Thr Glu Glu Phe Asn Ile Trp Asn
   85 90 95
   Asn Ser Met Val Glu Gln Met His Glu Asp Ile Ile Ser Leu Trp Asp
   100 105 110
   Gln Ser Leu Lys Pro Cys Val Lys Leu Thr Pro Leu Cys Val Thr Leu
   115 120 125
   Asn Cys Thr Asn Tyr Asn Ser Thr Ser Asn Asp Thr Thr Lys Glu Thr
   130 135 140
   Gly Ile Lys Lys Cys Ser Phe Asn Ile Thr Thr Ser Gly Val Lys Asp
   145 150 155 160
   Arg Phe Thr Lys Glu Asn Ala Leu Leu Tyr Thr Ala Asp Val Val Pro
   165 170 175
   Ile Asp Asn Ser Ser Ile Asn Tyr Arg Leu Ile Gly Cys Asn Thr Ser
   180 185 190
   Val Ile Thr Gln Ala Cys Pro Lys Val Ser Phe Glu Pro Ile Pro Ile
   195 200 205
   His Tyr Cys Ala Pro Ala Gly Phe Ala Ile Leu Lys Cys Asn Asn Glu
   210 215 220
   Thr Phe Asp Gly Lys Gly Pro Cys Thr Asn Val Ser Thr Val Gln Cys
   225 230 235 240
   Thr His Gly Ile Arg Pro Val Val Ser Thr Gln Leu Leu Leu Asn Gly
   245 250 255
   Ser Leu Ala Glu Thr Glu Val Val Ile Arg Ser Asp Asn Phe Ser Asn
   260 265 270
   Asn Ala Lys Thr Ile Ile Val Gln Leu Thr Glu Pro Val Val Ile Asn
   275 280 285
   Cys Ile Arg Pro Asn Gln Asn Thr Arg Arg Ser Ile His Ile Gly Pro
   290 295 300
   Gly Ser Ala Phe Tyr Thr Thr Gly Gln Ile Ile Gly Asp Ile Arg Gln
   305 310 315 320
   Ala His Cys Asn Ile Gln Gly Thr Gln Trp Gln Lys Thr Leu Ser Leu
   325 330 335
   Ile Val Asp Lys Leu Lys Lys Gln Phe Asn Asn Lys Thr Ile Ile Phe
   340 345 350
   Arg Asn Ser Ser Gly Gly Asp Pro Glu Ile Val Met His Ser Phe Asn
   355 360 365
   Cys Gly Gly Glu Phe Phe Tyr Cys Asn Thr Thr Gln Leu Phe Asn Ser
   370 375 380
   Ile Trp Met Leu Asn Asn Thr Trp Asp Gly Thr Lys Glu Glu Asn Ile
   385 390 395 400
   Thr Leu Pro Cys Arg Ile Lys Gln Ile Ile Asn Met Trp Gln Glu Val
   405 410 415
   Gly Lys Ala Met Tyr Ala Pro Pro Ile Lys Gly Gln Ile Arg Cys Ser
   420 425 430
   Ser Asn Ile Thr Gly Leu Leu Leu Thr Arg Asp Gly Gly Gly Asn Thr
   435 440 445
   Ser Ser Asp Asn Glu Thr Phe Arg Pro Gly Gly Gly Asp Met Arg Asp
   450 455 460
   Asn Trp Arg Ser Glu Leu Tyr Lys Tyr Lys Val Val Lys Ile Glu Pro
   465 470 475 480
   Leu Gly Val Ala Pro Thr Lys Ala Arg Arg Arg Val Val Gln Arg Glu
   485 490 495
   Lys Arg Ala Val Gly Met Leu Gly Ala Met Phe Leu Gly Phe Leu Gly
   500 505 510
   Ala Ala Gly Ser Thr Met Gly Ala Ala Ser Leu Ala Leu Thr Val Gln
   515 520 525
   Ala Arg Gln Val Leu Ser Gly Ile Val Gln Gln Gln Asn Asn Leu Leu
   530 535 540
   Arg Ala Ile Glu Ala Gln Gln His Leu Leu Gln Leu Thr Val Trp Gly
   545 550 555 560
   Ile Lys Gln Leu Gln Ala Arg Val Leu Ala Val Glu Arg Tyr Leu Gly
   565 570 575
   Asp Gln Gln Leu Leu Gly Ile Trp Gly Cys Ser Gly Lys Leu Ile Cys
   580 585 590
   Thr Thr Ala Val Pro Trp Asn Asp Ser Trp Ser Asn Lys Ser Leu Lys
   595 600 605
   Tyr Ile Trp Asp Asn Met Thr Trp Met Gln Trp Glu Lys Glu Ile Asp
   610 615 620
   Thr His Ile Gly Thr Ile Tyr Gln Leu Ile Glu Glu Ser Gln Tyr Gln
   625 630 635 640
   Gln Glu Lys Asn Glu Lys Glu Leu Leu Glu Leu Asp Lys Trp Ala Ser
   645 650 655
   Leu Trp Asn Trp Phe Asp Ile Thr Asn Trp Leu Trp Tyr Ile Lys Ile
   660 665 670
   Phe Ile Met Ile Val Gly Gly Leu Ile Gly Leu Arg Ile Val Phe Thr
   675 680 685
   Val Leu Ser Ile Val Asn Arg Val Arg Gln Gly Tyr Ser Pro Leu Ser
   690 695 700
   Leu Glu Thr Arg Leu Pro Thr Gln Arg Gly Pro Asp Arg Pro Glu Gly
   705 710 715 720
   Ile Glu Glu Glu Gly Gly Glu Arg Asp Arg Asp Thr Ser Gly Gln Leu
   725 730 735
   Val Asn Gly Phe Leu Ala Ile Ile Trp Val Asp Leu Arg Ser Leu Cys
   740 745 750
   Leu Phe Ser Tyr His Arg Leu Arg Asp Leu Leu Leu Ile Val Thr Arg
   755 760 765
   Ile Val Glu Leu Leu Gly Arg Arg Gly Trp Glu Ala Leu Lys Tyr Leu
   770 775 780
   Trp Asn Leu Leu Gln Tyr Trp Ser Gln Glu Leu Lys Lys Ser Ala Val
   785 790 795 800
   Ser Leu Phe Asn Ala Ile Ala Ile Ala Val Ala Glu Gly Thr Asp Arg
   805 810 815
   Ile Ile Glu Val Ile Gln Arg Ala Tyr Arg Ala Ile Leu His Ile Pro
   820 825 830
   Arg Arg Ile Arg Gln Gly Leu Glu Arg Leu Leu Leu
   835 840
<210> 13
   <211> 498
   <212> PRT
   <213> VIH-1 mutant g12
<400> 13
   Met Arg Val Lys Glu Ile Arg Arg Asn Tyr Gln His Leu Trp Arg Trp
    1               5                  10                  15
   Gly Thr Met Leu Leu Gly Met Phe Met Ile Cys Ser Ala Glu Glu Gln
               20                  25                  30
   Leu Trp Val Thr Val Tyr Tyr Gly Val Pro Val Trp Lys Glu Ala Thr
           35                  40                  45
   Thr Thr Leu Phe Cys Ala Ser Asp Ala Lys Ala Tyr Asp Thr Glu Val
       50                  55                  60
   His Asn Val Trp Ala Thr His Ala Cys Val Pro Thr Asp Pro Asn Pro
   65                  70                  75                  80
   Arg Glu Val Val Met Gly Asn Val Thr Glu Glu Phe Asn Ile Trp Asn
                   85                  90                  95
   Asn Ser Met Val Glu Gln Met His Glu Asp Ile Ile Ser Leu Trp Asp
               100                 105                 110
   Gln Ser Leu Lys Pro Cys Val Lys Leu Thr Pro Leu Cys Val Thr Leu
           115                 120                 125
   Asn Cys Thr Asn Tyr Asn Ser Thr Ser Asn Asp Thr Thr Lys Glu Thr
       130                 135                 140
   Gly Ile Lys Lys Cys Ser Phe Asn Ile Thr Thr Ser Gly Val Lys Asp
   145                 150                 155                 160
   Arg Phe Thr Lys Glu Asn Ala Leu Leu Tyr Thr Ala Asp Val Val Pro
   165 170 175
   Ile Asp Asn Ser Ser Ile Asn Tyr Arg Leu Ile Gly Cys Asn Thr Ser
   180 185 190
   Val Ile Thr Gln Ala Cys Pro Lys Val Ser Phe Glu Pro Ile Pro Ile
   195 200 205
   His Tyr Cys Ala Pro Ala Gly Phe Ala Ile Leu Lys Cys Asn Asn Glu
   210 215 220
   Thr Phe Asp Gly Lys Gly Pro Cys Thr Asn Val Ser Thr Val Gln Cys
   225 230 235 240
   Thr His Gly Ile Arg Pro Val Val Ser Thr Gln Leu Leu Leu Asn Gly
   245 250 255
   Ser Leu Ala Glu Thr Glu Val Val Ile Arg Ser Asp Asn Phe Ser Asn
   260 265 270
   Asn Ala Lys Thr Ile Ile Val Gln Leu Thr Glu Pro Val Val Ile Asn
   275 280 285
   Cys Ile Arg Pro Asn Gln Asn Thr Arg Arg Ser Ile His Ile Gly Pro
   290 295 300
   Gly Ser Ala Phe Tyr Thr Thr Gly Gln Ile Ile Gly Asp Ile Arg Gln
   305 310 315 320
   Ala His Cys Asn Ile Gln Gly Thr Gln Trp Gln Lys Thr Leu Ser Leu
   325 330 335
   Ile Val Asp Lys Leu Lys Lys Gln Phe Asn Asn Lys Thr Ile Ile Phe
   340 345 350
   Arg Asn Ser Ser Gly Gly Asp Pro Glu Ile Val Met His Ser Phe Asn
   355 360 365
   Cys Gly Gly Glu Phe Phe Tyr Cys Asn Thr Thr Gln Leu Phe Asn Ser
   370 375 380
   Ile Trp Met Leu Asn Asn Thr Trp Asp Gly Thr Lys Glu Glu Asn Ile
   385 390 395 400
   Thr Leu Pro Cys Arg Ile Lys Gln Ile Ile Asn Met Trp Gln Glu Val
   405 410 415
   Gly Lys Ala Met Tyr Ala Pro Pro Ile Lys Gly Gln Ile Arg Cys Ser
   420 425 430
   Ser Asn Ile Thr Gly Leu Leu Leu Thr Arg Asp Gly Gly Gly Asn Thr
   435 440 445
   Ser Ser Asp Asn Glu Thr Phe Arg Pro Gly Gly Gly Asp Met Arg Asp
   450 455 460
   Asn Trp Arg Ser Glu Leu Tyr Lys Tyr Lys Val Val Lys Ile Glu Pro
   465 470 475 480
   Leu Gly Val Ala Pro Thr Lys Ala Arg Arg Arg Val Val Gln Arg Glu
   485 490 495
   Lys Arg
<210> 14
   <211> 669
   <212> PRT
   <213> VIH-1 mutant g12
<400> 14
   Met Arg Val Lys Glu Ile Arg Arg Asn Tyr Gln His Leu Trp Arg Trp
   1 5 10 15
   Gly Thr Met Leu Leu Gly Met Phe Met Ile Cys Ser Ala Glu Glu Gln
   20 25 30
   Leu Trp Val Thr Val Tyr Tyr Gly Val Pro Val Trp Lys Glu Ala Thr
   35 40 45
   Thr Thr Leu Phe Cys Ala Ser Asp Ala Lys Ala Tyr Asp Thr Glu Val
   50 55 60
   His Asn Val Trp Ala Thr His Ala Cys Val Pro Thr Asp Pro Asn Pro
   65 70 75 80
   Arg Glu Val Val Met Gly Asn Val Thr Glu Glu Phe Asn Ile Trp Asn
   85 90 95
   Asn Ser Met Val Glu Gln Met His Glu Asp Ile Ile Ser Leu Trp Asp
   100 105 110
   Gln Ser Leu Lys Pro Cys Val Lys Leu Thr Pro Leu Cys Val Thr Leu
   115 120 125
   Asn Cys Thr Asn Tyr Asn Ser Thr Ser Asn Asp Thr Thr Lys Glu Thr
   130 135 140
   Gly Ile Lys Lys Cys Ser Phe Asn Ile Thr Thr Ser Gly Val Lys Asp
   145 150 155 160
   Arg Phe Thr Lys Glu Asn Ala Leu Leu Tyr Thr Ala Asp Val Val Pro
   165 170 175
   Ile Asp Asn Ser Ser Ile Asn Tyr Arg Leu Ile Gly Cys Asn Thr Ser
   180 185 190
   Val Ile Thr Gln Ala Cys Pro Lys Val Ser Phe Glu Pro Ile Pro Ile
   195 200 205
   His Tyr Cys Ala Pro Ala Gly Phe Ala Ile Leu Lys Cys Asn Asn Glu
   210 215 220
   Thr Phe Asp Gly Lys Gly Pro Cys Thr Asn Val Ser Thr Val Gln Cys
   225 230 235 240
   Thr His Gly Ile Arg Pro Val Val Ser Thr Gln Leu Leu Leu Asn Gly
   245 250 255
   Ser Leu Ala Glu Thr Glu Val Val Ile Arg Ser Asp Asn Phe Ser Asn
   260 265 270
   Asn Ala Lys Thr Ile Ile Val Gln Leu Thr Glu Pro Val Val Ile Asn
   275 280 285
   Cys Ile Arg Pro Asn Gln Asn Thr Arg Arg Ser Ile His Ile Gly Pro
   290 295 300
   Gly Ser Ala Phe Tyr Thr Thr Gly Gln Ile Ile Gly Asp Ile Arg Gln
   305 310 315 320
   Ala His Cys Asn Ile Gln Gly Thr Gln Trp Gln Lys Thr Leu Ser Leu
   325 330 335
   Ile Val Asp Lys Leu Lys Lys Gln Phe Asn Asn Lys Thr Ile Ile Phe
   340 345 350
   Arg Asn Ser Ser Gly Gly Asp Pro Glu Ile Val Met His Ser Phe Asn
   355 360 365
   Cys Gly Gly Glu Phe Phe Tyr Cys Asn Thr Thr Gln Leu Phe Asn Ser
   370 375 380
   Ile Trp Met Leu Asn Asn Thr Trp Asp Gly Thr Lys Glu Glu Asn Ile
   385                 390                 395                 400
   Thr Leu Pro Cys Arg Ile Lys Gln Ile Ile Asn Met Trp Gln Glu Val
                   405                 410                 415
   Gly Lys Ala Met Tyr Ala Pro Pro Ile Lys Gly Gln Ile Arg Cys Ser
               420                 425                 430
   Ser Asn Ile Thr Gly Leu Leu Leu Thr Arg Asp Gly Gly Gly Asn Thr
           435                 440                 445
   Ser Ser Asp Asn Glu Thr Phe Arg Pro Gly Gly Gly Asp Met Arg Asp
       450                 455                 460
   Asn Trp Arg Ser Glu Leu Tyr Lys Tyr Lys Val Val Lys Ile Glu Pro
   465                 470                 475                 480
   Leu Gly Val Ala Pro Thr Lys Ala Arg Arg Arg Val Val Gln Arg Glu
                   485                 490                 495
   Lys Arg Ala Val Gly Met Leu Gly Ala Met Phe Leu Gly Phe Leu Gly
               500                 505                 510
   Ala Ala Gly Ser Thr Met Gly Ala Ala Ser Leu Ala Leu Thr Val Gln
           515                 520                 525
   Ala Arg Gln Val Leu Ser Gly Ile Val Gln Gln Gln Asn Asn Leu Leu
       530                 535                 540
   Arg Ala Ile Glu Ala Gln Gln His Leu Leu Gln Leu Thr Val Trp Gly
   545                 550                 555                 560
   Ile Lys Gln Leu Gln Ala Arg Val Leu Ala Val Glu Arg Tyr Leu Gly
                   565                 570                 575
   Asp Gln Gln Leu Leu Gly Ile Trp Gly Cys Ser Gly Lys Leu Ile Cys
               580                 585                 590
   Thr Thr Ala Val Pro Trp Asn Asp Ser Trp Ser Asn Lys Ser Leu Lys
           595                 600                 605
   Tyr Ile Trp Asp Asn Met Thr Trp Met Gln Trp Glu Lys Glu Ile Asp
       610                 615                 620
   Thr His Ile Gly Thr Ile Tyr Gln Leu Ile Glu Glu Ser Gln Tyr Gln
   625                 630                 635                 640
   Gln Glu Lys Asn Glu Lys Glu Leu Leu Glu Leu Asp Lys Trp Ala Ser
   645 650 655
   Leu Trp Asn Trp Phe Asp Ile Thr Asn Trp Leu Trp Tyr
   660 665
<210> 15
   <211> 844
   <212> PRT
   <213> VIH-1 mutant g112
<400> 15
   Met Arg Val Lys Glu Ile Arg Arg Asn Tyr Gln His Leu Trp Arg Trp
   1 5 10 15
   Gly Thr Met Leu Leu Gly Met Phe Met Ile Cys Ser Ala Glu Glu Gln
   20 25 30
   Leu Trp Val Thr Val Tyr Tyr Gly Val Pro Val Trp Lys Glu Ala Thr
   35 40 45
   Thr Thr Leu Phe Cys Ala Ser Asp Ala Lys Ala Tyr Asp Thr Glu Val
   50 55 60
   His Asn Val Trp Ala Thr His Ala Cys Val Pro Thr Asp Pro Asn Pro
   65 70 75 80
   Arg Glu Val Val Met Gly Asn Val Thr Glu Glu Phe Asn Ile Trp Asn
   85 90 95
   Asn Ser Met Val Glu Gln Met His Glu Asp Ile Ile Ser Leu Trp Asp
   100 105 110
   Gln Ser Leu Lys Pro Cys Val Lys Leu Thr Pro Leu Cys Val Thr Leu
   115 120 125
   Asn Cys Thr Asn Tyr Asn Ser Thr Ser Asn Asp Thr Thr Lys Glu Thr
   130 135 140
   Gly Ile Lys Lys Cys Ser Phe Asn Ile Thr Thr Ser Gly Val Lys Asp
   145 150 155 160
   Arg Phe Thr Lys Glu Asn Ala Leu Leu Tyr Thr Ala Asp Val Val Pro
   165 170 175
   Ile Asp Asn Ser Ser Ile Asn Tyr Arg Leu Ile Gly Cys Asn Thr Ser
   180 185 190
   Val Ile Thr Gln Ala Cys Pro Lys Val Ser Phe Glu Pro Ile Pro Ile
   195 200 205
   His Tyr Cys Ala Pro Ala Gly Phe Ala Ile Leu Lys Cys Asn Asn Glu
   210 215 220
   Thr Phe Asp Gly Lys Gly Pro Cys Thr Asn Val Ser Thr Val Gln Cys
   225 230 235 240
   Thr His Gly Ile Arg Pro Val Val Ser Thr Gln Leu Leu Leu Asn Gly
   245 250 255
   Ser Leu Ala Glu Thr Glu Val Val Ile Arg Ser Asp Asn Phe Ser Asn
   260 265 270
   Asn Ala Lys Thr Ile Ile Val Gln Leu Thr Glu Pro Val Val Ile Asn
   275 280 285
   Cys Ile Arg Pro Asn Gln Asn Thr Arg Arg Ser Ile His Ile Gly Pro
   290 295 300
   Gly Ser Ala Phe Tyr Thr Thr Gly Gln Ile Ile Gly Asp Ile Arg Gln
   305 310 315 320
   Ala His Cys Asn Ile Gln Gly Thr Gln Trp Gln Lys Thr Leu Ser Leu
   325 330 335
   Ile Val Asp Lys Leu Lys Lys Gln Phe Asn Gln Lys Thr Ile Ile Phe
   340 345 350
   Arg Gln Ser Ser Gly Gly Asp Pro Glu Ile Val Met His Ser Phe Asn
   355 360 365
   Cys Gly Gly Glu Phe Phe Tyr Cys Asn Thr Thr Gln Leu Phe Asn Ser
   370 375 380
   Ile Trp Met Leu Asn Asn Thr Trp Asp Gly Thr Lys Glu Glu Asn Ile
   385 390 395 400
   Thr Leu Pro Cys Arg Ile Lys Gln Ile Ile Asn Met Trp Gln Glu Val
   405 410 415
   Gly Lys Ala Met Tyr Ala Pro Pro Ile Lys Gly Gln Ile Arg Cys Ser
   420 425 430
   Ser Asn Ile Thr Gly Leu Leu Leu Thr Arg Asp Gly Gly Gly Asn Thr
   435 440 445
   Ser Ser Asp Asn Glu Thr Phe Arg Pro Gly Gly Gly Asp Met Arg Asp
   450 455 460
   Asn Trp Arg Ser Glu Leu Tyr Lys Tyr Lys Val Val Lys Ile Glu Pro
   465 470 475 480
   Leu Gly Val Ala Pro Thr Lys Ala Arg Arg Arg Val Val Gln Arg Glu
   485 490 495
   Lys Arg Ala Val Gly Met Leu Gly Ala Met Phe Leu Gly Phe Leu Gly
   500 505 510
   Ala Ala Gly Ser Thr Met Gly Ala Ala Ser Leu Ala Leu Thr Val Gln
   515 520 525
   Ala Arg Gln Val Leu Ser Gly Ile Val Gln Gln Gln Asn Asn Leu Leu
   530 535 540
   Arg Ala Ile Glu Ala Gln Gln His Leu Leu Gln Leu Thr Val Trp Gly
   545 550 555 560
   Ile Lys Gln Leu Gln Ala Arg Val Leu Ala Val Glu Arg Tyr Leu Gly
   565 570 575
   Asp Gln Gln Leu Leu Gly Ile Trp Gly Cys Ser Gly Lys Leu Ile Cys
   580 585 590
   Thr Thr Ala Val Pro Trp Asn Asp Ser Trp Ser Asn Lys Ser Leu Lys
   595 600 605
   Tyr Ile Trp Asp Asn Met Thr Trp Met Gln Trp Glu Lys Glu Ile Asp
   610 615 620
   Thr His Ile Gly Thr Ile Tyr Gln Leu Ile Glu Glu Ser Gln Tyr Gln
   625 630 635 640
   Gln Glu Lys Asn Glu Lys Glu Leu Leu Glu Leu Asp Lys Trp Ala Ser
   645 650 655
   Leu Trp Asn Trp Phe Asp Ile Thr Asn Trp Leu Trp Tyr Ile Lys Ile
   660 665 670
   Phe Ile Met Ile Val Gly Gly Leu Ile Gly Leu Arg Ile Val Phe Thr
   675 680 685
   Val Leu Ser Ile Val Asn Arg Val Arg Gln Gly Tyr Ser Pro Leu Ser
   690 695 700
   Leu Glu Thr Arg Leu Pro Thr Gln Arg Gly Pro Asp Arg Pro Glu Gly
   705 710 715 720
   Ile Glu Glu Glu Gly Gly Glu Arg Asp Arg Asp Thr Ser Gly Gln Leu
   725 730 735
   Val Asn Gly Phe Leu Ala Ile Ile Trp Val Asp Leu Arg Ser Leu Cys
   740 745 750
   Leu Phe Ser Tyr His Arg Leu Arg Asp Leu Leu Leu Ile Val Thr Arg
   755 760 765
   Ile Val Glu Leu Leu Gly Arg Arg Gly Trp Glu Ala Leu Lys Tyr Leu
   770 775 780
   Trp Asn Leu Leu Gln Tyr Trp Ser Gln Glu Leu Lys Lys Ser Ala Val
   785 790 795 800
   Ser Leu Phe Asn Ala Ile Ala Ile Ala Val Ala Glu Gly Thr Asp Arg
   805 810 815
   Ile Ile Glu Val Ile Gln Arg Ala Tyr Arg Ala Ile Leu His Ile Pro
   820 825 830
   Arg Arg Ile Arg Gln Gly Leu Glu Arg Leu Leu Leu
   835 840
<210> 16
   <211> 498
   <212> PRT
   <213> VIH-1 mutant g112
<400> 16
   Met Arg Val Lys Glu Ile Arg Arg Asn Tyr Gln His Leu Trp Arg Trp
   1 5 10 15
   Gly Thr Met Leu Leu Gly Met Phe Met Ile Cys Ser Ala Glu Glu Gln
   20 25 30
   Leu Trp Val Thr Val Tyr Tyr Gly Val Pro Val Trp Lys Glu Ala Thr
   35 40 45
   Thr Thr Leu Phe Cys Ala Ser Asp Ala Lys Ala Tyr Asp Thr Glu Val
   50 55 60
   His Asn Val Trp Ala Thr His Ala Cys Val Pro Thr Asp Pro Asn Pro
   65 70 75 80
   Arg Glu Val Val Met Gly Asn Val Thr Glu Glu Phe Asn Ile Trp Asn
   85 90 95
   Asn Ser Met Val Glu Gln Met His Glu Asp Ile Ile Ser Leu Trp Asp
   100 105 110
   Gln Ser Leu Lys Pro Cys Val Lys Leu Thr Pro Leu Cys Val Thr Leu
   115 120 125
   Asn Cys Thr Asn Tyr Asn Ser Thr Ser Asn Asp Thr Thr Lys Glu Thr
   130 135 140
   Gly Ile Lys Lys Cys Ser Phe Asn Ile Thr Thr Ser Gly Val Lys Asp
   145 150 155 160
   Arg Phe Thr Lys Glu Asn Ala Leu Leu Tyr Thr Ala Asp Val Val Pro
   165 170 175
   Ile Asp Asn Ser Ser Ile Asn Tyr Arg Leu Ile Gly Cys Asn Thr Ser
   180 185 190
   Val Ile Thr Gln Ala Cys Pro Lys Val Ser Phe Glu Pro Ile Pro Ile
   195 200 205
   His Tyr Cys Ala Pro Ala Gly Phe Ala Ile Leu Lys Cys Asn Asn Glu
   210 215 220
   Thr Phe Asp Gly Lys Gly Pro Cys Thr Asn Val Ser Thr Val Gln Cys
   225 230 235 240
   Thr His Gly Ile Arg Pro Val Val Ser Thr Gln Leu Leu Leu Asn Gly
   245 250 255
   Ser Leu Ala Glu Thr Glu Val Val Ile Arg Ser Asp Asn Phe Ser Asn
   260 265 270
   Asn Ala Lys Thr Ile Ile Val Gln Leu Thr Glu Pro Val Val Ile Asn
   275 280 285
   Cys Ile Arg Pro Asn Gln Asn Thr Arg Arg Ser Ile His Ile Gly Pro
   290 295 300
   Gly Ser Ala Phe Tyr Thr Thr Gly Gln Ile Ile Gly Asp Ile Arg Gln
   305 310 315 320
   Ala His Cys Asn Ile Gln Gly Thr Gln Trp Gln Lys Thr Leu Ser Leu
   325 330 335
   Ile Val Asp Lys Leu Lys Lys Gln Phe Asn Gln Lys Thr Ile Ile Phe
   340 345 350
   Arg Gln Ser Ser Gly Gly Asp Pro Glu Ile Val Met His Ser Phe Asn
   355 360 365
   Cys Gly Gly Glu Phe Phe Tyr Cys Asn Thr Thr Gln Leu Phe Asn Ser
   370 375 380
   Ile Trp Met Leu Asn Asn Thr Trp Asp Gly Thr Lys Glu Glu Asn Ile
   385 390 395 400
   Thr Leu Pro Cys Arg Ile Lys Gln Ile Ile Asn Met Trp Gln Glu Val
   405 410 415
   Gly Lys Ala Met Tyr Ala Pro Pro Ile Lys Gly Gln Ile Arg Cys Ser
   420 425 430
   Ser Asn Ile Thr Gly Leu Leu Leu Thr Arg Asp Gly Gly Gly Asn Thr
   435 440 445
   Ser Ser Asp Asn Glu Thr Phe Arg Pro Gly Gly Gly Asp Met Arg Asp
   450 455 460
   Asn Trp Arg Ser Glu Leu Tyr Lys Tyr Lys Val Val Lys Ile Glu Pro
   465 470 475 480
   Leu Gly Val Ala Pro Thr Lys Ala Arg Arg Arg Val Val Gln Arg Glu
   485 490 495
   Lys Arg
<210> 17
   <211> 669
   <212> PRT
   <213> VIH-1 mutant g112
<400> 17
   Met Arg Val Lys Glu Ile Arg Arg Asn Tyr Gln His Leu Trp Arg Trp
   1 5 10 15
   Gly Thr Met Leu Leu Gly Met Phe Met Ile Cys Ser Ala Glu Glu Gln
   20 25 30
   Leu Trp Val Thr Val Tyr Tyr Gly Val Pro Val Trp Lys Glu Ala Thr
   35 40 45
   Thr Thr Leu Phe Cys Ala Ser Asp Ala Lys Ala Tyr Asp Thr Glu Val
   50 55 60
   His Asn Val Trp Ala Thr His Ala Cys Val Pro Thr Asp Pro Asn Pro
   65 70 75 80
   Arg Glu Val Val Met Gly Asn Val Thr Glu Glu Phe Asn Ile Trp Asn
   85 90 95
   Asn Ser Met Val Glu Gln Met His Glu Asp Ile Ile Ser Leu Trp Asp
   100 105 110
   Gln Ser Leu Lys Pro Cys Val Lys Leu Thr Pro Leu Cys Val Thr Leu
   115 120 125
   Asn Cys Thr Asn Tyr Asn Ser Thr Ser Asn Asp Thr Thr Lys Glu Thr
   130 135 140
   Gly Ile Lys Lys Cys Ser Phe Asn Ile Thr Thr Ser Gly Val Lys Asp
   145 150 155 160
   Arg Phe Thr Lys Glu Asn Ala Leu Leu Tyr Thr Ala Asp Val Val Pro
   165 170 175
   Ile Asp Asn Ser Ser Ile Asn Tyr Arg Leu Ile Gly Cys Asn Thr Ser
   180 185 190
   Val Ile Thr Gln Ala Cys Pro Lys Val Ser Phe Glu Pro Ile Pro Ile
   195 200 205
   His Tyr Cys Ala Pro Ala Gly Phe Ala Ile Leu Lys Cys Asn Asn Glu
   210 215 220
   Thr Phe Asp Gly Lys Gly Pro Cys Thr Asn Val Ser Thr Val Gln Cys
   225 230 235 240
   Thr His Gly Ile Arg Pro Val Val Ser Thr Gln Leu Leu Leu Asn Gly
   245 250 255
   Ser Leu Ala Glu Thr Glu Val Val Ile Arg Ser Asp Asn Phe Ser Asn
   260 265 270
   Asn Ala Lys Thr Ile Ile Val Gln Leu Thr Glu Pro Val Val Ile Asn
   275 280 285
   Cys Ile Arg Pro Asn Gln Asn Thr Arg Arg Ser Ile His Ile Gly Pro
   290 295 300
   Gly Ser Ala Phe Tyr Thr Thr Gly Gln Ile Ile Gly Asp Ile Arg Gln
   305 310 315 320
   Ala His Cys Asn Ile Gln Gly Thr Gln Trp Gln Lys Thr Leu Ser Leu
   325 330 335
   Ile Val Asp Lys Leu Lys Lys Gln Phe Asn Gln Lys Thr Ile Ile Phe
   340 345 350
   Arg Gln Ser Ser Gly Gly Asp Pro Glu Ile Val Met His Ser Phe Asn
   355 360 365
   Cys Gly Gly Glu Phe Phe Tyr Cys Asn Thr Thr Gln Leu Phe Asn Ser
   370 375 380
   Ile Trp Met Leu Asn Asn Thr Trp Asp Gly Thr Lys Glu Glu Asn Ile
   385 390 395 400
   Thr Leu Pro Cys Arg Ile Lys Gln Ile Ile Asn Met Trp Gln Glu Val
   405 410 415
   Gly Lys Ala Met Tyr Ala Pro Pro Ile Lys Gly Gln Ile Arg Cys Ser
   420 425 430
   Ser Asn Ile Thr Gly Leu Leu Leu Thr Arg Asp Gly Gly Gly Asn Thr
   435 440 445
   Ser Ser Asp Asn Glu Thr Phe Arg Pro Gly Gly Gly Asp Met Arg Asp
   450 455 460
   Asn Trp Arg Ser Glu Leu Tyr Lys Tyr Lys Val Val Lys Ile Glu Pro
   465 470 475 480
   Leu Gly Val Ala Pro Thr Lys Ala Arg Arg Arg Val Val Gln Arg Glu
   485 490 495
   Lys Arg Ala Val Gly Met Leu Gly Ala Met Phe Leu Gly Phe Leu Gly
   500 505 510
   Ala Ala Gly Ser Thr Met Gly Ala Ala Ser Leu Ala Leu Thr Val Gln
   515 520 525
   Ala Arg Gln Val Leu Ser Gly Ile Val Gln Gln Gln Asn Asn Leu Leu
   530 535 540
   Arg Ala Ile Glu Ala Gln Gln His Leu Leu Gln Leu Thr Val Trp Gly
   545                 550                 555                 560
   Ile Lys Gln Leu Gln Ala Arg Val Leu Ala Val Glu Arg Tyr Leu Gly
                   565                 570                 575
   Asp Gln Gln Leu Leu Gly Ile Trp Gly Cys Ser Gly Lys Leu Ile Cys
               580                 585                 590
   Thr Thr Ala Val Pro Trp Asn Asp Ser Trp Ser Asn Lys Ser Leu Lys
           595                 600                 605
   Tyr Ile Trp Asp Asn Met Thr Trp Met Gln Trp Glu Lys Glu Ile Asp
       610                 615                 620
   Thr His Ile Gly Thr Ile Tyr Gln Leu Ile Glu Glu Ser Gln Tyr Gln
   625                 630                 635                 640
   Gln Glu Lys Asn Glu Lys Glu Leu Leu Glu Leu Asp Lys Trp Ala Ser
                   645                 650                 655
   Leu Trp Asn Trp Phe Asp Ile Thr Asn Trp Leu Trp Tyr
               660                 665
<210> 18
   <211> 844
   <212> PRT
   <213> VIH-1 mutant g14
<400> 18
   Met Arg Val Lys Glu Ile Arg Arg Asn Tyr Gln His Leu Trp Arg Trp
    1               5                  10                  15
   Gly Thr Met Leu Leu Gly Met Phe Met Ile Cys Ser Ala Glu Glu Gln
               20                  25                  30
   Leu Trp Val Thr Val Tyr Tyr Gly Val Pro Val Trp Lys Glu Ala Thr
           35                  40                  45
   Thr Thr Leu Phe Cys Ala Ser Asp Ala Lys Ala Tyr Asp Thr Glu Val
       50                  55                  60
   His Asn Val Trp Ala Thr His Ala Cys Val Pro Thr Asp Pro Asn Pro
   65                  70                  75                  80
   Arg Glu Val Val Met Gly Asn Val Thr Glu Glu Phe Asn Ile Trp Asn
   85 90 95
   Asn Ser Met Val Glu Gln Met His Glu Asp Ile Ile Ser Leu Trp Asp
   100 105 110
   Gln Ser Leu Lys Pro Cys Val Lys Leu Thr Pro Leu Cys Val Thr Leu
   115 120 125
   Asn Cys Thr Asn Tyr Asn Ser Thr Ser Asn Asp Thr Thr Lys Glu Thr
   130 135 140
   Gly Ile Lys Lys Cys Ser Phe Asn Ile Thr Thr Ser Gly Val Lys Asp
   145 150 155 160
   Arg Phe Thr Lys Glu Asn Ala Leu Leu Tyr Thr Ala Asp Val Val Pro
   165 170 175
   Ile Asp Asn Ser Ser Ile Asn Tyr Arg Leu Ile Gly Cys Asn Thr Ser
   180 185 190
   Val Ile Thr Gln Ala Cys Pro Lys Val Ser Phe Glu Pro Ile Pro Ile
   195 200 205
   His Tyr Cys Ala Pro Ala Gly Phe Ala Ile Leu Lys Cys Asn Asn Glu
   210 215 220
   Thr Phe Asp Gly Lys Gly Pro Cys Thr Asn Val Ser Thr Val Gln Cys
   225 230 235 240
   Thr His Gly Ile Arg Pro Val Val Ser Thr Gln Leu Leu Leu Asn Gly
   245 250 255
   Ser Leu Ala Glu Thr Glu Val Val Ile Arg Ser Asp Gln Phe Ser Asn
   260 265 270
   Asn Ala Lys Thr Ile Ile Val Gln Leu Thr Glu Pro Val Val Ile Asn
   275 280 285
   Cys Ile Arg Pro Asn Gln Asn Thr Arg Arg Ser Ile His Ile Gly Pro
   290 295 300
   Gly Ser Ala Phe Tyr Thr Thr Gly Gln Ile Ile Gly Asp Ile Arg Gln
   305 310 315 320
   Ala His Cys Asn Ile Asn Gly Thr Gln Trp Asn Lys Thr Leu Ser Leu
   325 330 335
   Ile Val Asp Lys Leu Lys Lys Gln Phe Asn Asn Lys Thr Ile Ile Phe
   340 345 350
   Arg Asn Ser Ser Gly Gly Asp Pro Glu Ile Val Met His Ser Phe Asn
   355 360 365
   Cys Gly Gly Glu Phe Phe Tyr Cys Asn Thr Thr Gln Leu Phe Asn Ser
   370 375 380
   Ile Trp Met Leu Asn Asn Thr Trp Asp Gly Thr Lys Glu Glu Asn Ile
   385 390 395 400
   Thr Leu Pro Cys Arg Ile Lys Gln Ile Ile Asn Met Trp Gln Glu Val
   405 410 415
   Gly Lys Ala Met Tyr Ala Pro Pro Ile Lys Gly Gln Ile Arg Cys Ser
   420 425 430
   Ser Asn Ile Thr Gly Leu Leu Leu Thr Arg Asp Gly Gly Gly Asn Thr
   435 440 445
   Ser Ser Asp Asn Glu Thr Phe Arg Pro Gly Gly Gly Asp Met Arg Asp
   450 455 460
   Asn Trp Arg Ser Glu Leu Tyr Lys Tyr Lys Val Val Lys Ile Glu Pro
   465 470 475 480
   Leu Gly Val Ala Pro Thr Lys Ala Arg Arg Arg Val Val Gln Arg Glu
   485 490 495
   Lys Arg Ala Val Gly Met Leu Gly Ala Met Phe Leu Gly Phe Leu Gly
   500 505 510
   Ala Ala Gly Ser Thr Met Gly Ala Ala Ser Leu Ala Leu Thr Val Gln
   515 520 525
   Ala Arg Gln Val Leu Ser Gly Ile Val Gln Gln Gln Asn Asn Leu Leu
   530 535 540
   Arg Ala Ile Glu Ala Gln Gln His Leu Leu Gln Leu Thr Val Trp Gly
   545 550 555 560
   Ile Lys Gln Leu Gln Ala Arg Val Leu Ala Val Glu Arg Tyr Leu Gly
   565 570 575
   Asp Gln Gln Leu Leu Gly Ile Trp Gly Cys Ser Gly Lys Leu Ile Cys
   580 585 590
   Thr Thr Ala Val Pro Trp Asn Asp Ser Trp Ser Asn Lys Ser Leu Lys
   595 600 605
   Tyr Ile Trp Asp Asn Met Thr Trp Met Gln Trp Glu Lys Glu Ile Asp
   610 615 620
   Thr His Ile Gly Thr Ile Tyr Gln Leu Ile Glu Glu Ser Gln Tyr Gln
   625 630 635 640
   Gln Glu Lys Asn Glu Lys Glu Leu Leu Glu Leu Asp Lys Trp Ala Ser
   645 650 655
   Leu Trp Asn Trp Phe Asp Ile Thr Asn Trp Leu Trp Tyr Ile Lys Ile
   660 665 670
   Phe Ile Met Ile Val Gly Gly Leu Ile Gly Leu Arg Ile Val Phe Thr
   675 680 685
   Val Leu Ser Ile Val Asn Arg Val Arg Gln Gly Tyr Ser Pro Leu Ser
   690 695 700
   Leu Glu Thr Arg Leu Pro Thr Gln Arg Gly Pro Asp Arg Pro Glu Gly
   705 710 715 720
   Ile Glu Glu Glu Gly Gly Glu Arg Asp Arg Asp Thr Ser Gly Gln Leu
   725 730 735
   Val Asn Gly Phe Leu Ala Ile Ile Trp Val Asp Leu Arg Ser Leu Cys
   740 745 750
   Leu Phe Ser Tyr His Arg Leu Arg Asp Leu Leu Leu Ile Val Thr Arg
   755 760 765
   Ile Val Glu Leu Leu Gly Arg Arg Gly Trp Glu Ala Leu Lys Tyr Leu
   770 775 780
   Trp Asn Leu Leu Gln Tyr Trp Ser Gln Glu Leu Lys Lys Ser Ala Val
   785 790 795 800
   Ser Leu Phe Asn Ala Ile Ala Ile Ala Val Ala Glu Gly Thr Asp Arg
   805 810 815
   Ile Ile Glu Val Ile Gln Arg Ala Tyr Arg Ala Ile Leu His Ile Pro
   820 825 830
   Arg Arg Ile Arg Gln Gly Leu Glu Arg Leu Leu Leu
   835 840
<210> 19
   <211> 498
   <212> PRT
   <213> VIH-1 mutant g14
<400> 19
   Met Arg Val Lys Glu Ile Arg Arg Asn Tyr Gln His Leu Trp Arg Trp
   1 5 10 15
   Gly Thr Met Leu Leu Gly Met Phe Met Ile Cys Ser Ala Glu Glu Gln
   20 25 30
   Leu Trp Val Thr Val Tyr Tyr Gly Val Pro Val Trp Lys Glu Ala Thr
   35 40 45
   Thr Thr Leu Phe Cys Ala Ser Asp Ala Lys Ala Tyr Asp Thr Glu Val
   50 55 60
   His Asn Val Trp Ala Thr His Ala Cys Val Pro Thr Asp Pro Asn Pro
   65 70 75 80
   Arg Glu Val Val Met Gly Asn Val Thr Glu Glu Phe Asn Ile Trp Asn
   85 90 95
   Asn Ser Met Val Glu Gln Met His Glu Asp Ile Ile Ser Leu Trp Asp
   100 105 110
   Gln Ser Leu Lys Pro Cys Val Lys Leu Thr Pro Leu Cys Val Thr Leu
   115 120 125
   Asn Cys Thr Asn Tyr Asn Ser Thr Ser Asn Asp Thr Thr Lys Glu Thr
   130 135 140
   Gly Ile Lys Lys Cys Ser Phe Asn Ile Thr Thr Ser Gly Val Lys Asp
   145 150 155 160
   Arg Phe Thr Lys Glu Asn Ala Leu Leu Tyr Thr Ala Asp Val Val Pro
   165 170 175
   Ile Asp Asn Ser Ser Ile Asn Tyr Arg Leu Ile Gly Cys Asn Thr Ser
   180 185 190
   Val Ile Thr Gln Ala Cys Pro Lys Val Ser Phe Glu Pro Ile Pro Ile
   195 200 205
   His Tyr Cys Ala Pro Ala Gly Phe Ala Ile Leu Lys Cys Asn Asn Glu
   210 215 220
   Thr Phe Asp Gly Lys Gly Pro Cys Thr Asn Val Ser Thr Val Gln Cys
   225 230 235 240
   Thr His Gly Ile Arg Pro Val Val Ser Thr Gln Leu Leu Leu Asn Gly
   245 250 255
   Ser Leu Ala Glu Thr Glu Val Val Ile Arg Ser Asp Gln Phe Ser Asn
   260 265 270
   Asn Ala Lys Thr Ile Ile Val Gln Leu Thr Glu Pro Val Val Ile Asn
   275 280 285
   Cys Ile Arg Pro Asn Gln Asn Thr Arg Arg Ser Ile His Ile Gly Pro
   290 295 300
   Gly Ser Ala Phe Tyr Thr Thr Gly Gln Ile Ile Gly Asp Ile Arg Gln
   305 310 315 320
   Ala His Cys Asn Ile Asn Gly Thr Gln Trp Asn Lys Thr Leu Ser Leu
   325 330 335
   Ile Val Asp Lys Leu Lys Lys Gln Phe Asn Asn Lys Thr Ile Ile Phe
   340 345 350
   Arg Asn Ser Ser Gly Gly Asp Pro Glu Ile Val Met His Ser Phe Asn
   355 360 365
   Cys Gly Gly Glu Phe Phe Tyr Cys Asn Thr Thr Gln Leu Phe Asn Ser
   370 375 380
   Ile Trp Met Leu Asn Asn Thr Trp Asp Gly Thr Lys Glu Glu Asn Ile
   385 390 395 400
   Thr Leu Pro Cys Arg Ile Lys Gln Ile Ile Asn Met Trp Gln Glu Val
   405 410 415
   Gly Lys Ala Met Tyr Ala Pro Pro Ile Lys Gly Gln Ile Arg Cys Ser
   420 425 430
   Ser Asn Ile Thr Gly Leu Leu Leu Thr Arg Asp Gly Gly Gly Asn Thr
   435 440 445
   Ser Ser Asp Asn Glu Thr Phe Arg Pro Gly Gly Gly Asp Met Arg Asp
   450 455 460
   Asn Trp Arg Ser Glu Leu Tyr Lys Tyr Lys Val Val Lys Ile Glu Pro
   465 470 475 480
   Leu Gly Val Ala Pro Thr Lys Ala Arg Arg Arg Val Val Gln Arg Glu
   485 490 495
   Lys Arg
<210> 20
   <211> 669
   <212> PRT
   <213> VIH-1 mutant g14
<400> 20
   Met Arg Val Lys Glu Ile Arg Arg Asn Tyr Gln His Leu Trp Arg Trp
   1 5 10 15
   Gly Thr Met Leu Leu Gly Met Phe Met Ile Cys Ser Ala Glu Glu Gln
   20 25 30
   Leu Trp Val Thr Val Tyr Tyr Gly Val Pro Val Trp Lys Glu Ala Thr
   35 40 45
   Thr Thr Leu Phe Cys Ala Ser Asp Ala Lys Ala Tyr Asp Thr Glu Val
   50 55 60
   His Asn Val Trp Ala Thr His Ala Cys Val Pro Thr Asp Pro Asn Pro
   65 70 75 80
   Arg Glu Val Val Met Gly Asn Val Thr Glu Glu Phe Asn Ile Trp Asn
   85 90 95
   Asn Ser Met Val Glu Gln Met His Glu Asp Ile Ile Ser Leu Trp Asp
   100 105 110
   Gln Ser Leu Lys Pro Cys Val Lys Leu Thr Pro Leu Cys Val Thr Leu
   115 120 125
   Asn Cys Thr Asn Tyr Asn Ser Thr Ser Asn Asp Thr Thr Lys Glu Thr
   130 135 140
   Gly Ile Lys Lys Cys Ser Phe Asn Ile Thr Thr Ser Gly Val Lys Asp
   145 150 155 160
   Arg Phe Thr Lys Glu Asn Ala Leu Leu Tyr Thr Ala Asp Val Val Pro
   165 170 175
   Ile Asp Asn Ser Ser Ile Asn Tyr Arg Leu Ile Gly Cys Asn Thr Ser
   180 185 190
   Val Ile Thr Gln Ala Cys Pro Lys Val Ser Phe Glu Pro Ile Pro Ile
   195 200 205
   His Tyr Cys Ala Pro Ala Gly Phe Ala Ile Leu Lys Cys Asn Asn Glu
   210 215 220
   Thr Phe Asp Gly Lys Gly Pro Cys Thr Asn Val Ser Thr Val Gln Cys
   225 230 235 240
   Thr His Gly Ile Arg Pro Val Val Ser Thr Gln Leu Leu Leu Asn Gly
   245 250 255
   Ser Leu Ala Glu Thr Glu Val Val Ile Arg Ser Asp Gln Phe Ser Asn
   260 265 270
   Asn Ala Lys Thr Ile Ile Val Gln Leu Thr Glu Pro Val Val Ile Asn
   275 280 285
   Cys Ile Arg Pro Asn Gln Asn Thr Arg Arg Ser Ile His Ile Gly Pro
   290 295 300
   Gly Ser Ala Phe Tyr Thr Thr Gly Gln Ile Ile Gly Asp Ile Arg Gln
   305 310 315 320
   Ala His Cys Asn Ile Asn Gly Thr Gln Trp Asn Lys Thr Leu Ser Leu
   325 330 335
   Ile Val Asp Lys Leu Lys Lys Gln Phe Asn Asn Lys Thr Ile Ile Phe
   340 345 350
   Arg Asn Ser Ser Gly Gly Asp Pro Glu Ile Val Met His Ser Phe Asn
   355 360 365
   Cys Gly Gly Glu Phe Phe Tyr Cys Asn Thr Thr Gln Leu Phe Asn Ser
   370 375 380
   Ile Trp Met Leu Asn Asn Thr Trp Asp Gly Thr Lys Glu Glu Asn Ile
   385 390 395 400
   Thr Leu Pro Cys Arg Ile Lys Gln Ile Ile Asn Met Trp Gln Glu Val
   405 410 415
   Gly Lys Ala Met Tyr Ala Pro Pro Ile Lys Gly Gln Ile Arg Cys Ser
   420 425 430
   Ser Asn Ile Thr Gly Leu Leu Leu Thr Arg Asp Gly Gly Gly Asn Thr
   435 440 445
   Ser Ser Asp Asn Glu Thr Phe Arg Pro Gly Gly Gly Asp Met Arg Asp
   450 455 460
   Asn Trp Arg Ser Glu Leu Tyr Lys Tyr Lys Val Val Lys Ile Glu Pro
   465 470 475 480
   Leu Gly Val Ala Pro Thr Lys Ala Arg Arg Arg Val Val Gln Arg Glu
   485 490 495
   Lys Arg Ala Val Gly Met Leu Gly Ala Met Phe Leu Gly Phe Leu Gly
   500 505 510
   Ala Ala Gly Ser Thr Met Gly Ala Ala Ser Leu Ala Leu Thr Val Gln
   515 520 525
   Ala Arg Gln Val Leu Ser Gly Ile Val Gln Gln Gln Asn Asn Leu Leu
   530 535 540
   Arg Ala Ile Glu Ala Gln Gln His Leu Leu Gln Leu Thr Val Trp Gly
   545 550 555 560
   Ile Lys Gln Leu Gln Ala Arg Val Leu Ala Val Glu Arg Tyr Leu Gly
   565 570 575
   Asp Gln Gln Leu Leu Gly Ile Trp Gly Cys Ser Gly Lys Leu Ile Cys
   580 585 590
   Thr Thr Ala Val Pro Trp Asn Asp Ser Trp Ser Asn Lys Ser Leu Lys
   595 600 605
   Tyr Ile Trp Asp Asn Met Thr Trp Met Gln Trp Glu Lys Glu Ile Asp
   610 615 620
   Thr His Ile Gly Thr Ile Tyr Gln Leu Ile Glu Glu Ser Gln Tyr Gln
   625 630 635 640
   Gln Glu Lys Asn Glu Lys Glu Leu Leu Glu Leu Asp Lys Trp Ala Ser
   645 650 655
   Leu Trp Asn Trp Phe Asp Ile Thr Asn Trp Leu Trp Tyr
   660 665
<210> 21
   <211> 2535
   <212> ADN
   <213> VIH-1 mutant g22
<400> 21
   atgagagtga aggagatcag gaggaattat cagcacttgt ggagatgggg cacgatgctc 60
   cttgggatgt tcatgatctg tagtgcagaa gaacaattgt gggtcacagt ctattatggg 120
   gtacctgtgt ggaaagaagc aactaccact ctattttgtg catcagatgc taaagcatat 180
   gatacagagg tacataatgt ctgggccaca catgcctgtg tacccacaga ccccaaccca 240
   cgagaagtag taatgggaaa tgtgacagaa gaatttaaca tatggaacaa tagcatggta 300
   gaacagatgc atgaagatat aattagttta tgggatcaaa gtctaaagcc atgtgtaaaa 360
   ttaaccccac tctgtgttac tttaaattgc actaactaca atagtactag taatgatact 420
   accaaggaaa caggaataaa aaaatgctct ttcaatatca ccacctcagg cgtaaaagat 480
   aggtttacga aggaaaatgc acttttgtat acagctgatg tagtaccaat agataatagt 540
   agtatcaact ataggttgat aggttgtaac acctcagtca ttacccaggc ctgtccaaag 600
   gtatcctttg agccaattcc catacattat tgtgccccgg ctggttttgc gattctaaag 660
   tgtaataatg aaacattcga tggaaaagga ccatgtacaa atgtcagcac agtacaatgt 720
   acacatggaa ttagaccagt agtatcaact caactgctgt taaatggcag tctagcagaa 780
   acagaggtag taattagatc tgacaatttc tcgaacaatg ctaaaaccat aatagtacaa 840
   ctgacagaac ctgtagtaat caattgtata agacccaaca acaacacaag aagaagtata 900
   catataggac cagggagcgc attttataca acaggacaaa taataggaga tataagacaa 960
   gcacattgta acattcaggg aacacaatgg cagaaaactt taagtctaat agttgataaa 1020
   ttaaagaaac aatttaatca gaaaacaata atctttaggc agtcctcagg aggggaccca 1080
   gaaattgtaa tgcacagttt taactgtgga ggggaatttt tctactgtaa tacaacacaa 1140
   ctgtttaata gtatttggat gcttaataat acttgggatg gtactaaaga ggaaaatatt 1200
   acactcccat gcagaataaa acaaattata aacatgtggc aggaagtagg aaaagcaatg 1260
   tatgcccctc ccatcaaagg acaaattaga tgttcatcaa acattacagg gctgctatta 1320
   acaagagatg gtggtggtaa cacgagcagc gataatgaga ccttcagacc tggaggagga 1380
   gatatgaggg ataattggag aagtgaatta tataaatata aagtagtaaa aattgaacca 1440
   ttaggagtag cacccaccaa ggcaaggaga agagtggtgc agagagaaaa aagagcagtg 1500
   ggaatgctag gagccatgtt ccttgggttc ttgggagcag caggaagcac tatgggcgca 1560
   gcgtcactgg cgttgacggt acaggccaga caagtattgt ctggtatagt gcaacagcag 1620
   aacaatttgc tgagggctat tgaggcgcaa cagcacctgt tgcaactcac agtctggggc 1680
   atcaagcagc tccaggcaag agtcctggct gtggaaaggt acctagggga tcaacagctc 1740
   ctgggaattt ggggttgctc tggaaaactc atctgcacca ctgctgtgcc ttggaatgat 1800
   agttggagta ataaatctct gaaatatatt tgggataaca tgacctggat gcagtgggaa 1860
   aaagaaattg acacccacat aggcacaata taccaattaa ttgaagaatc gcagtaccaa 1920
   caagaaaaga atgaaaaaga attattggaa ctagataaat gggcaagttt gtggaattgg 1980
   tttgacataa caaactggct gtggtatata aaaatattca taatgatagt aggaggcttg 2040
   ataggtttaa gaatagtttt tactgtactt tctatagtga atagagttag gcagggatac 2100
   tcaccattat cgttggagac ccgcctccca acccagaggg gacccgacag gcccgaagga 2160
   atcgaagaag aaggtggaga gagagacaga gacacatccg gtcaattagt gaatggattc 2220
   ttagcaatta tctgggtcga cctgcggagc ctgtgcctct tcagctacca ccgcttgaga 2280
   gacttactct tgattgtaac gaggattgtg gaacttctgg gacgcagggg gtgggaagcc 2340
   ctcaagtatt tgtggaatct cctccagtat tggagtcagg aactaaagaa gagtgctgtt 2400
   agcttgttca atgccatagc catagcagta gctgagggga cagataggat aatagaagta 2460
   atacaaaggg cttatagggc cattctccac atacctagaa gaataagaca gggcttggaa 2520
   aggcttttgc tataa 2535
<210> 22
   <211> 2007
   <212> ADN
   <213> VIH-1 mutant g22
<400> 22
   atgagagtga aggagatcag gaggaattat cagcacttgt ggagatgggg cacgatgctc 60
   cttgggatgt tcatgatctg tagtgcagaa gaacaattgt gggtcacagt ctattatggg 120
   gtacctgtgt ggaaagaagc aactaccact ctattttgtg catcagatgc taaagcatat 180
   gatacagagg tacataatgt ctgggccaca catgcctgtg tacccacaga ccccaaccca 240
   cgagaagtag taatgggaaa tgtgacagaa gaatttaaca tatggaacaa tagcatggta 300
   gaacagatgc atgaagatat aattagttta tgggatcaaa gtctaaagcc atgtgtaaaa 360
   ttaaccccac tctgtgttac tttaaattgc actaactaca atagtactag taatgatact 420
   accaaggaaa caggaataaa aaaatgctct ttcaatatca ccacctcagg cgtaaaagat 480
   aggtttacga aggaaaatgc acttttgtat acagctgatg tagtaccaat agataatagt 540
   agtatcaact ataggttgat aggttgtaac acctcagtca ttacccaggc ctgtccaaag 600
   gtatcctttg agccaattcc catacattat tgtgccccgg ctggttttgc gattctaaag 660
   tgtaataatg aaacattcga tggaaaagga ccatgtacaa atgtcagcac agtacaatgt 720
   acacatggaa ttagaccagt agtatcaact caactgctgt taaatggcag tctagcagaa 780
   acagaggtag taattagatc tgacaatttc tcgaacaatg ctaaaaccat aatagtacaa 840
   ctgacagaac ctgtagtaat caattgtata agacccaaca acaacacaag aagaagtata 900
   catataggac cagggagcgc attttataca acaggacaaa taataggaga tataagacaa 960
   gcacattgta acattcaggg aacacaatgg cagaaaactt taagtctaat agttgataaa 1020
   ttaaagaaac aatttaatca gaaaacaata atctttaggc agtcctcagg aggggaccca 1080
   gaaattgtaa tgcacagttt taactgtgga ggggaatttt tctactgtaa tacaacacaa 1140
   ctgtttaata gtatttggat gcttaataat acttgggatg gtactaaaga ggaaaatatt 1200
   acactcccat gcagaataaa acaaattata aacatgtggc aggaagtagg aaaagcaatg 1260
   tatgcccctc ccatcaaagg acaaattaga tgttcatcaa acattacagg gctgctatta 1320
   acaagagatg gtggtggtaa cacgagcagc gataatgaga ccttcagacc tggaggagga 1380
   gatatgaggg ataattggag aagtgaatta tataaatata aagtagtaaa aattgaacca 1440
   ttaggagtag cacccaccaa ggcaaggaga agagtggtgc agagagaaaa aagagcagtg 1500
   ggaatgctag gagccatgtt ccttgggttc ttgggagcag caggaagcac tatgggcgca 1560
   gcgtcactgg cgttgacggt acaggccaga caagtattgt ctggtatagt gcaacagcag 1620
   aacaatttgc tgagggctat tgaggcgcaa cagcacctgt tgcaactcac agtctggggc 1680
   atcaagcagc tccaggcaag agtcctggct gtggaaaggt acctagggga tcaacagctc 1740
   ctgggaattt ggggttgctc tggaaaactc atctgcacca ctgctgtgcc ttggaatgat 1800
   agttggagta ataaatctct gaaatatatt tgggataaca tgacctggat gcagtgggaa 1860
   aaagaaattg acacccacat aggcacaata taccaattaa ttgaagaatc gcagtaccaa 1920
   caagaaaaga atgaaaaaga attattggaa ctagataaat gggcaagttt gtggaattgg 1980
   tttgacataa caaactggct gtggtat                                     2007
<210> 23
   <211> 1494
   <212> ADN
   <213> VIH-1 mutant g22
<400> 23
   atgagagtga aggagatcag gaggaattat cagcacttgt ggagatgggg cacgatgctc 60
   cttgggatgt tcatgatctg tagtgcagaa gaacaattgt gggtcacagt ctattatggg 120
   gtacctgtgt ggaaagaagc aactaccact ctattttgtg catcagatgc taaagcatat 180
   gatacagagg tacataatgt ctgggccaca catgcctgtg tacccacaga ccccaaccca 240
   cgagaagtag taatgggaaa tgtgacagaa gaatttaaca tatggaacaa tagcatggta 300
   gaacagatgc atgaagatat aattagttta tgggatcaaa gtctaaagcc atgtgtaaaa 360
   ttaaccccac tctgtgttac tttaaattgc actaactaca atagtactag taatgatact 420
   accaaggaaa caggaataaa aaaatgctct ttcaatatca ccacctcagg cgtaaaagat 480
   aggtttacga aggaaaatgc acttttgtat acagctgatg tagtaccaat agataatagt 540
   agtatcaact ataggttgat aggttgtaac acctcagtca ttacccaggc ctgtccaaag 600
   gtatcctttg agccaattcc catacattat tgtgccccgg ctggttttgc gattctaaag 660
   tgtaataatg aaacattcga tggaaaagga ccatgtacaa atgtcagcac agtacaatgt 720
   acacatggaa ttagaccagt agtatcaact caactgctgt taaatggcag tctagcagaa 780
   acagaggtag taattagatc tgacaatttc tcgaacaatg ctaaaaccat aatagtacaa 840
   ctgacagaac ctgtagtaat caattgtata agacccaaca acaacacaag aagaagtata 900
   catataggac cagggagcgc attttataca acaggacaaa taataggaga tataagacaa 960
   gcacattgta acattcaggg aacacaatgg cagaaaactt taagtctaat agttgataaa 1020
   ttaaagaaac aatttaatca gaaaacaata atctttaggc agtcctcagg aggggaccca 1080
   gaaattgtaa tgcacagttt taactgtgga ggggaatttt tctactgtaa tacaacacaa 1140
   ctgtttaata gtatttggat gcttaataat acttgggatg gtactaaaga ggaaaatatt 1200
   acactcccat gcagaataaa acaaattata aacatgtggc aggaagtagg aaaagcaatg 1260
   tatgcccctc ccatcaaagg acaaattaga tgttcatcaa acattacagg gctgctatta 1320
   acaagagatg gtggtggtaa cacgagcagc gataatgaga ccttcagacc tggaggagga 1380
   gatatgaggg ataattggag aagtgaatta tataaatata aagtagtaaa aattgaacca 1440
   ttaggagtag cacccaccaa ggcaaggaga agagtggtgc agagagaaaa aaga       1494
<210> 24
   <211> 844
   <212> PRT
   <213> VIH-1 mutant g22
<400> 24
   Met Arg Val Lys Glu Ile Arg Arg Asn Tyr Gln His Leu Trp Arg Trp
   1 5 10 15
   Gly Thr Met Leu Leu Gly Met Phe Met Ile Cys Ser Ala Glu Glu Gln
   20 25 30
   Leu Trp Val Thr Val Tyr Tyr Gly Val Pro Val Trp Lys Glu Ala Thr
   35 40 45
   Thr Thr Leu Phe Cys Ala Ser Asp Ala Lys Ala Tyr Asp Thr Glu Val
   50 55 60
   His Asn Val Trp Ala Thr His Ala Cys Val Pro Thr Asp Pro Asn Pro
   65 70 75 80
   Arg Glu Val Val Met Gly Asn Val Thr Glu Glu Phe Asn Ile Trp Asn
   85 90 95
   Asn Ser Met Val Glu Gln Met His Glu Asp Ile Ile Ser Leu Trp Asp
   100 105 110
   Gln Ser Leu Lys Pro Cys Val Lys Leu Thr Pro Leu Cys Val Thr Leu
   115 120 125
   Asn Cys Thr Asn Tyr Asn Ser Thr Ser Asn Asp Thr Thr Lys Glu Thr
   130 135 140
   Gly Ile Lys Lys Cys Ser Phe Asn Ile Thr Thr Ser Gly Val Lys Asp
   145 150 155 160
   Arg Phe Thr Lys Glu Asn Ala Leu Leu Tyr Thr Ala Asp Val Val Pro
   165 170 175
   Ile Asp Asn Ser Ser Ile Asn Tyr Arg Leu Ile Gly Cys Asn Thr Ser
   180 185 190
   Val Ile Thr Gln Ala Cys Pro Lys Val Ser Phe Glu Pro Ile Pro Ile
   195 200 205
   His Tyr Cys Ala Pro Ala Gly Phe Ala Ile Leu Lys Cys Asn Asn Glu
   210 215 220
   Thr Phe Asp Gly Lys Gly Pro Cys Thr Asn Val Ser Thr Val Gln Cys
   225 230 235 240
   Thr His Gly Ile Arg Pro Val Val Ser Thr Gln Leu Leu Leu Asn Gly
   245 250 255
   Ser Leu Ala Glu Thr Glu Val Val Ile Arg Ser Asp Asn Phe Ser Asn
   260 265 270
   Asn Ala Lys Thr Ile Ile Val Gln Leu Thr Glu Pro Val Val Ile Asn
   275 280 285
   Cys Ile Arg Pro Asn Asn Asn Thr Arg Arg Ser Ile His Ile Gly Pro
   290 295 300
   Gly Ser Ala Phe Tyr Thr Thr Gly Gln Ile Ile Gly Asp Ile Arg Gln
   305 310 315 320
   Ala His Cys Asn Ile Gln Gly Thr Gln Trp Gln Lys Thr Leu Ser Leu
   325 330 335
   Ile Val Asp Lys Leu Lys Lys Gln Phe Asn Gln Lys Thr Ile Ile Phe
   340 345 350
   Arg Gln Ser Ser Gly Gly Asp Pro Glu Ile Val Met His Ser Phe Asn
   355 360 365
   Cys Gly Gly Glu Phe Phe Tyr Cys Asn Thr Thr Gln Leu Phe Asn Ser
   370 375 380
   Ile Trp Met Leu Asn Asn Thr Trp Asp Gly Thr Lys Glu Glu Asn Ile
   385 390 395 400
   Thr Leu Pro Cys Arg Ile Lys Gln Ile Ile Asn Met Trp Gln Glu Val
   405 410 415
   Gly Lys Ala Met Tyr Ala Pro Pro Ile Lys Gly Gln Ile Arg Cys Ser
   420 425 430
   Ser Asn Ile Thr Gly Leu Leu Leu Thr Arg Asp Gly Gly Gly Asn Thr
   435 440 445
   Ser Ser Asp Asn Glu Thr Phe Arg Pro Gly Gly Gly Asp Met Arg Asp
   450 455 460
   Asn Trp Arg Ser Glu Leu Tyr Lys Tyr Lys Val Val Lys Ile Glu Pro
   465 470 475 480
   Leu Gly Val Ala Pro Thr Lys Ala Arg Arg Arg Val Val Gln Arg Glu
   485 490 495
   Lys Arg Ala Val Gly Met Leu Gly Ala Met Phe Leu Gly Phe Leu Gly
   500 505 510
   Ala Ala Gly Ser Thr Met Gly Ala Ala Ser Leu Ala Leu Thr Val Gln
   515 520 525
   Ala Arg Gln Val Leu Ser Gly Ile Val Gln Gln Gln Asn Asn Leu Leu
   530 535 540
   Arg Ala Ile Glu Ala Gln Gln His Leu Leu Gln Leu Thr Val Trp Gly
   545 550 555 560
   Ile Lys Gln Leu Gln Ala Arg Val Leu Ala Val Glu Arg Tyr Leu Gly
   565 570 575
   Asp Gln Gln Leu Leu Gly Ile Trp Gly Cys Ser Gly Lys Leu Ile Cys
   580 585 590
   Thr Thr Ala Val Pro Trp Asn Asp Ser Trp Ser Asn Lys Ser Leu Lys
   595 600 605
   Tyr Ile Trp Asp Asn Met Thr Trp Met Gln Trp Glu Lys Glu Ile Asp
   610 615 620
   Thr His Ile Gly Thr Ile Tyr Gln Leu Ile Glu Glu Ser Gln Tyr Gln
   625 630 635 640
   Gln Glu Lys Asn Glu Lys Glu Leu Leu Glu Leu Asp Lys Trp Ala Ser
   645 650 655
   Leu Trp Asn Trp Phe Asp Ile Thr Asn Trp Leu Trp Tyr Ile Lys Ile
   660 665 670
   Phe Ile Met Ile Val Gly Gly Leu Ile Gly Leu Arg Ile Val Phe Thr
   675 680 685
   Val Leu Ser Ile Val Asn Arg Val Arg Gln Gly Tyr Ser Pro Leu Ser
   690 695 700
   Leu Glu Thr Arg Leu Pro Thr Gln Arg Gly Pro Asp Arg Pro Glu Gly
   705 710 715 720
   Ile Glu Glu Glu Gly Gly Glu Arg Asp Arg Asp Thr Ser Gly Gln Leu
   725 730 735
   Val Asn Gly Phe Leu Ala Ile Ile Trp Val Asp Leu Arg Ser Leu Cys
   740 745 750
   Leu Phe Ser Tyr His Arg Leu Arg Asp Leu Leu Leu Ile Val Thr Arg
   755 760 765
   Ile Val Glu Leu Leu Gly Arg Arg Gly Trp Glu Ala Leu Lys Tyr Leu
   770 775 780
   Trp Asn Leu Leu Gln Tyr Trp Ser Gln Glu Leu Lys Lys Ser Ala Val
   785 790 795 800
   Ser Leu Phe Asn Ala Ile Ala Ile Ala Val Ala Glu Gly Thr Asp Arg
   805 810 815
   Ile Ile Glu Val Ile Gln Arg Ala Tyr Arg Ala Ile Leu His Ile Pro
   820 825 830
   Arg Arg Ile Arg Gln Gly Leu Glu Arg Leu Leu Leu
   835 840
<210> 25
   <211> 669
   <212> PRT
   <213> VIH-1 mutant g22
<400> 25
   Met Arg Val Lys Glu Ile Arg Arg Asn Tyr Gln His Leu Trp Arg Trp
   1 5 10 15
   Gly Thr Met Leu Leu Gly Met Phe Met Ile Cys Ser Ala Glu Glu Gln
   20 25 30
   Leu Trp Val Thr Val Tyr Tyr Gly Val Pro Val Trp Lys Glu Ala Thr
   35 40 45
   Thr Thr Leu Phe Cys Ala Ser Asp Ala Lys Ala Tyr Asp Thr Glu Val
   50 55 60
   His Asn Val Trp Ala Thr His Ala Cys Val Pro Thr Asp Pro Asn Pro
   65 70 75 80
   Arg Glu Val Val Met Gly Asn Val Thr Glu Glu Phe Asn Ile Trp Asn
   85 90 95
   Asn Ser Met Val Glu Gln Met His Glu Asp Ile Ile Ser Leu Trp Asp
   100 105 110
   Gln Ser Leu Lys Pro Cys Val Lys Leu Thr Pro Leu Cys Val Thr Leu
   115 120 125
   Asn Cys Thr Asn Tyr Asn Ser Thr Ser Asn Asp Thr Thr Lys Glu Thr
   130 135 140
   Gly Ile Lys Lys Cys Ser Phe Asn Ile Thr Thr Ser Gly Val Lys Asp
   145 150 155 160
   Arg Phe Thr Lys Glu Asn Ala Leu Leu Tyr Thr Ala Asp Val Val Pro
   165 170 175
   Ile Asp Asn Ser Ser Ile Asn Tyr Arg Leu Ile Gly Cys Asn Thr Ser
   180 185 190
   Val Ile Thr Gln Ala Cys Pro Lys Val Ser Phe Glu Pro Ile Pro Ile
   195 200 205
   His Tyr Cys Ala Pro Ala Gly Phe Ala Ile Leu Lys Cys Asn Asn Glu
   210 215 220
   Thr Phe Asp Gly Lys Gly Pro Cys Thr Asn Val Ser Thr Val Gln Cys
   225 230 235 240
   Thr His Gly Ile Arg Pro Val Val Ser Thr Gln Leu Leu Leu Asn Gly
   245 250 255
   Ser Leu Ala Glu Thr Glu Val Val Ile Arg Ser Asp Asn Phe Ser Asn
   260 265 270
   Asn Ala Lys Thr Ile Ile Val Gln Leu Thr Glu Pro Val Val Ile Asn
   275 280 285
   Cys Ile Arg Pro Asn Asn Asn Thr Arg Arg Ser Ile His Ile Gly Pro
   290 295 300
   Gly Ser Ala Phe Tyr Thr Thr Gly Gln Ile Ile Gly Asp Ile Arg Gln
   305 310 315 320
   Ala His Cys Asn Ile Gln Gly Thr Gln Trp Gln Lys Thr Leu Ser Leu
   325 330 335
   Ile Val Asp Lys Leu Lys Lys Gln Phe Asn Gln Lys Thr Ile Ile Phe
   340 345 350
   Arg Gln Ser Ser Gly Gly Asp Pro Glu Ile Val Met His Ser Phe Asn
   355 360 365
   Cys Gly Gly Glu Phe Phe Tyr Cys Asn Thr Thr Gln Leu Phe Asn Ser
   370 375 380
   Ile Trp Met Leu Asn Asn Thr Trp Asp Gly Thr Lys Glu Glu Asn Ile
   385 390 395 400
   Thr Leu Pro Cys Arg Ile Lys Gln Ile Ile Asn Met Trp Gln Glu Val
   405 410 415
   Gly Lys Ala Met Tyr Ala Pro Pro Ile Lys Gly Gln Ile Arg Cys Ser
   420 425 430
   Ser Asn Ile Thr Gly Leu Leu Leu Thr Arg Asp Gly Gly Gly Asn Thr
   435 440 445
   Ser Ser Asp Asn Glu Thr Phe Arg Pro Gly Gly Gly Asp Met Arg Asp
   450 455 460
   Asn Trp Arg Ser Glu Leu Tyr Lys Tyr Lys Val Val Lys Ile Glu Pro
   465 470 475 480
   Leu Gly Val Ala Pro Thr Lys Ala Arg Arg Arg Val Val Gln Arg Glu
   485 490 495
   Lys Arg Ala Val Gly Met Leu Gly Ala Met Phe Leu Gly Phe Leu Gly
   500 505 510
   Ala Ala Gly Ser Thr Met Gly Ala Ala Ser Leu Ala Leu Thr Val Gln
   515 520 525
   Ala Arg Gln Val Leu Ser Gly Ile Val Gln Gln Gln Asn Asn Leu Leu
   530 535 540
   Arg Ala Ile Glu Ala Gln Gln His Leu Leu Gln Leu Thr Val Trp Gly
   545 550 555 560
   Ile Lys Gln Leu Gln Ala Arg Val Leu Ala Val Glu Arg Tyr Leu Gly
   565 570 575
   Asp Gln Gln Leu Leu Gly Ile Trp Gly Cys Ser Gly Lys Leu Ile Cys
   580 585 590
   Thr Thr Ala Val Pro Trp Asn Asp Ser Trp Ser Asn Lys Ser Leu Lys
   595 600 605
   Tyr Ile Trp Asp Asn Met Thr Trp Met Gln Trp Glu Lys Glu Ile Asp
   610 615 620
   Thr His Ile Gly Thr Ile Tyr Gln Leu Ile Glu Glu Ser Gln Tyr Gln
   625 630 635 640
   Gln Glu Lys Asn Glu Lys Glu Leu Leu Glu Leu Asp Lys Trp Ala Ser
   645 650 655
   Leu Trp Asn Trp Phe Asp Ile Thr Asn Trp Leu Trp Tyr
   660 665
<210> 26
   <211> 498
   <212> PRT
   <213> VIH-1 mutant g22
<400> 26
   Met Arg Val Lys Glu Ile Arg Arg Asn Tyr Gln His Leu Trp Arg Trp
   1               5                   10                  15
   Gly Thr Met Leu Leu Gly Met Phe Met Ile Cys Ser Ala Glu Glu Gln
               20                  25                  30
   Leu Trp Val Thr Val Tyr Tyr Gly Val Pro Val Trp Lys Glu Ala Thr
           35                  40                  45
   Thr Thr Leu Phe Cys Ala Ser Asp Ala Lys Ala Tyr Asp Thr Glu Val
       50                  55                  60
   His Asn Val Trp Ala Thr His Ala Cys Val Pro Thr Asp Pro Asn Pro
   65                  70                  75                  80
   Arg Glu Val Val Met Gly Asn Val Thr Glu Glu Phe Asn Ile Trp Asn
                   85                  90                  95
   Asn Ser Met Val Glu Gln Met His Glu Asp Ile Ile Ser Leu Trp Asp
               100                 105                 110
   Gln Ser Leu Lys Pro Cys Val Lys Leu Thr Pro Leu Cys Val Thr Leu
           115                 120                 125
   Asn Cys Thr Asn Tyr Asn Ser Thr Ser Asn Asp Thr Thr Lys Glu Thr
       130                 135                 140
   Gly Ile Lys Lys Cys Ser Phe Asn Ile Thr Thr Ser Gly Val Lys Asp
   145                 150                 155                 160
   Arg Phe Thr Lys Glu Asn Ala Leu Leu Tyr Thr Ala Asp Val Val Pro
                   165                 170                 175
   Ile Asp Asn Ser Ser Ile Asn Tyr Arg Leu Ile Gly Cys Asn Thr Ser
               180                 185                 190
   Val Ile Thr Gln Ala Cys Pro Lys Val Ser Phe Glu Pro Ile Pro Ile
           195                 200                 205
   His Tyr Cys Ala Pro Ala Gly Phe Ala Ile Leu Lys Cys Asn Asn Glu
       210                 215                 220
   Thr Phe Asp Gly Lys Gly Pro Cys Thr Asn Val Ser Thr Val Gln Cys
   225                 230                 235                 240
   Thr His Gly Ile Arg Pro Val Val Ser Thr Gln Leu Leu Leu Asn Gly
                   245                 250                 255
   Ser Leu Ala Glu Thr Glu Val Val Ile Arg Ser Asp Asn Phe Ser Asn
               260                 265                 270
   Asn Ala Lys Thr Ile Ile Val Gln Leu Thr Glu Pro Val Val Ile Asn
           275                 280                 285
   Cys Ile Arg Pro Asn Asn Asn Thr Arg Arg Ser Ile His Ile Gly Pro
   290 295 300
   Gly Ser Ala Phe Tyr Thr Thr Gly Gln Ile Ile Gly Asp Ile Arg Gln
   305 310 315 320
   Ala His Cys Asn Ile Gln Gly Thr Gln Trp Gln Lys Thr Leu Ser Leu
   325 330 335
   Ile Val Asp Lys Leu Lys Lys Gln Phe Asn Gln Lys Thr Ile Ile Phe
   340 345 350
   Arg Gln Ser Ser Gly Gly Asp Pro Glu Ile Val Met His Ser Phe Asn
   355 360 365
   Cys Gly Gly Glu Phe Phe Tyr Cys Asn Thr Thr Gln Leu Phe Asn Ser
   370 375 380
   Ile Trp Met Leu Asn Asn Thr Trp Asp Gly Thr Lys Glu Glu Asn Ile
   385 390 395 400
   Thr Leu Pro Cys Arg Ile Lys Gln Ile Ile Asn Met Trp Gln Glu Val
   405 410 415
   Gly Lys Ala Met Tyr Ala Pro Pro Ile Lys Gly Gln Ile Arg Cys Ser
   420 425 430
   Ser Asn Ile Thr Gly Leu Leu Leu Thr Arg Asp Gly Gly Gly Asn Thr
   435 440 445
   Ser Ser Asp Asn Glu Thr Phe Arg Pro Gly Gly Gly Asp Met Arg Asp
   450 455 460
   Asn Trp Arg Ser Glu Leu Tyr Lys Tyr Lys Val Val Lys Ile Glu Pro
   465 470 475 480
   Leu Gly Val Ala Pro Thr Lys Ala Arg Arg Arg Val Val Gln Arg Glu
   485 490 495
   Lys Arg